# EUROPEAN PATENT SPECIFICATION

(11) **EP 1 910 359 B1**
(45) Date of publication and mention of the grant of the patent: **18.11.2009**
(21) Application number: 06777692.2
(22) Date of filing: 11.07.2006
(51) Int. Cl.: C07D 471/04, A61P 3/00, A61K 31/519

(54) **PYRIDO [2 , 3-D]PYRIMIDINE-2 , 4-DIAMINE COMPOUNDS AS PTPlB INHIBITORS**
PYRIDO[2,3-D]PYRIMIDIN-2,4-DIAMIN-VERBINDUNGEN ALS PTPIB-HEMMER
COMPOSES PYRIDO [2, 3-D]PYRIMIDINE-2, 4-DIAMINE UTILISES COMME INHIBITEURS DE PTP1B

(30) Priority: 21.07.2005 US 701467 P
(43) Date of publication of application: 16.04.2008
(73) Proprietor: F. Hoffmann-La Roche AG, 4070 Basel (CH)
(72) Inventor: BERTHEL, Steven, Joseph, Mendham Township, NJ 07945 (US); CHEUNG, Adrian, Wai-Hing, Glen Rock, NJ 07452 (US); KIM, Kyungjin, Livingston, NJ 07039 (US); LI, Shiming, Edison, NJ 08820 (US); THAKKAR, Kshitij, Chhabilbhai, Clifton, NJ 07012 (US); YUN, Weiya, Warren, NJ 07059 (US)
(74) Representative: Salud, Carlos E.
(86) International application number: PCT/EP2006/064091
(87) International publication number: WO 2007/009911

(56) References cited:
- WO-A-01/55147
- WO-A-98/46605
- WO-A-02/064594
- WO-A-03/001887
- REINHARD TROSCHÜTZ ET AL.: "Verbesserte synthese von methyl- und phenylsubstituierten Pyrido(2,3-d)pyrimidin-2,4-diaminen" ARCHIV DER PHARMAZIE., vol. 327, 1994, pages 221-224, XP002401737 DEVCH VERLAGSGESELLSCHAFT MBH, WEINHEIM.

## Description

The present invention comprises pyridopyrimidinediamine derivatives of the formula (I): 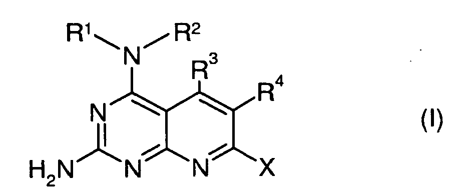
wherein X is a group X-1 of the formula: 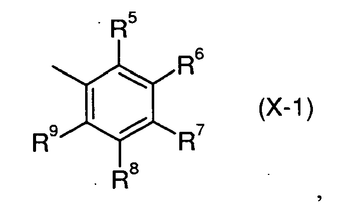
or X is a group X-2 of the formula: 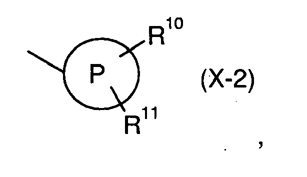
or X is a group X-3 of the formula: 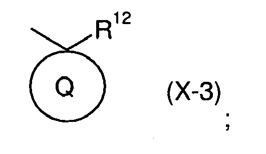
R¹ and R² are each independently selected from the group consisting of hydrogen, lower alkyl, methoxy lower alkyl and hydroxy lower alkyl, except that R¹ and R² may not both be hydrogen;
   R³ is hydrogen, lower alkyl or phenyl;
   R⁴ is hydrogen, lower alkyl, lower alkylsulfonyl, phenyl, carboxy, or together with R⁵ forms a 5-7 membered carbocyclic ring;
R⁵ when not in a ring with R⁴ is hydrogen, lower alkyl, substituted lower alkyl, lower alkoxy, substituted lower alkoxy, hydroxy, carboxy, halogen, lower alkylthio, lower alkylsulfinyl, lower alkylsulfonyl, aminosulfonyl, cyano, nitro, lower alkanoyl, aryl, aroyl, aryloxy, arylthio, lower alkylamino, lower alkanoylamino, sulfonylamino, cycloalkyl, cycloalkoxy, heterocyclyl, heterocyclyloxy, heterocyclylcarbonyl, heteroaryl, or together with R⁶ forms a 5 or 6 membered aromatic ring;
R⁶ when not in a ring with R⁵ is hydrogen, lower alkyl, substituted lower alkyl, lower alkoxy, substituted lower alkoxy, hydroxy, halogen, lower alkylthio, lower alkylsulfinyl, lower alkylsulfonyl, aminosulfonyl, cyano, nitro, lower alkanoyl, aryl, aroyl, aryloxy, lower alkylamino, lower alkanoylamino, sulfonylamino, cycloalkyl, heterocyclyl, heterocyclyloxy or heterocyclylcarbonyl;
R⁷ is hydrogen, lower alkyl, lower alkoxy, alkoxy lower alkyl, alkoxy lower alkoxy, hydroxy lower alkyl, hydroxy, hydroxyalkoxy, halogen, lower alkylthio, lower alkylsulfinyl, lower alkylsulfonyl, perfluoro lower alkyl, lower alkanoyl, aroyl or lower alkanoylami no;
R⁸ and R⁹ are each independently selected from the group consisting of hydrogen, lower alkyl, substituted lower alkyl, lower alkoxy, substituted lower alkoxy, hydroxy, halogen, lower alkylthio, lower alkylsulfinyl, lower alkylsulfonyl, aminosulfonyl, cyano, nitro, lower alkanoyl, aryl, aroyl, aryloxy, arylthio, lower alkylamino, lower alkanoylamino, sulfonylamino, cycloalkyl, cycloalkoxy, heteroaryl, heterocyclyl, heterocyclyloxy and heterocyclylcarbonyl;
P is a 5 or 6 membered heteroaromatic ring containing from 1 to 2 hetero atoms selected from the group consisting of oxygen, sulfur and nitrogen;
R¹⁰ and R¹¹ are each independently selected from the group consisting of hydrogen, lower alkyl, lower alkoxy, perfluoro lower alkyl, halogen, aryl lower alkyl, aryl and aryl lower alkoxy;
Q is a 3-6 membered cycloalkyl ring; and
   R¹² is hydrogen or aryl;
and the pharmaceutically acceptable salts or esters of the foregoing.

The compounds of the present invention are potent inhibitors of PTP1 B. Accordingly, the invention also encompasses pharmaceutical compositions and methods of treating or preventing PTP-1 B mediated diseases, including diabetes, obesity, and diabetes-related diseases.

Protein tyrosine phosphatases (PTPases) are key enzymes in processes that regulate cell growth and differentiation. The inhibition of these enzymes can play a role in the modulation of multiple signaling pathways in which tyrosine phosphorylation dephosphorylation plays a role. PTP1 B is a particular protein tyrosine phosphatase that is often used as a prototypical member of that class of enzymes. Kennedy et al., 1999, Science 283: 1544-1548 showed that protein tyrosine phosphatase PTP-1 B is a negative regulator of the insulin signaling pathway, suggesting that inhibitors of this enzyme may be beneficial in the treatment of diabetes.

PTPase inhibitors are recognized as potential therapeutic agents for the treatment of diabetes. See, e.g. Moeller et al., 3(5):527-40, Current Opinion in Drug Discovery and Development, 2000; or Zhang, Zhong-Yin, 5:416-23, Current Opinion in Chemical Biology, 2001. The utility of PTPase inhibitors as therapeutic agents has been a topic of discussion in several review articles, including, for example, Expert Opin Investig Drugs 12(2):223-33, Feb. 2003.

Inhibitors of PTP-1 B have utility in controlling or treating Type 1 and Type 2 diabetes, in improving glucose tolerance, and in improving insulin sensitivity in patients in need thereof.

WO 98/46605, WO 02/064594 and WO 01/55147 mention pyrido(2,3-d)pyrimidine derivatives useful for the treatment of diabetes. WO 03/001887 mentions compounds with antibacterial activity. The article by Troschütz et al. in Archiv der Pharmazie 1994, 327, 221-224 mentions compounds with antitumoral activity.

As used in this specification, the term "lower alkyl", alone or in combination (for example, as part of "lower alkanoyl," below), means a straight-chain or branched-chain alkyl group containing a maximum of six carbon atoms, such as methyl, ethyl, n-propyl, isopropyl, n-butyl, sec. butyl, isobutyl, tert,butyl, n-pentyl and n-hexyl.

"Substituted lower alkyl" means lower alkyl as defined substituted by one or more groups selected independently from cycloalkyl, nitro, aryloxy, aryl, heteroaryl, hydroxy, halogen, cyano, lower alkoxy, lower alkoxycarbonyl, lower alkanoyl, lower alkylthio, lower alkyl sulfinyl, lower alkyl sulfonyl, and substituted amino, e.g., dimethylamino. Preferred substituents are hydroxy, halogen, nitro, lower alkoxy phenoxy, phenyl and lower alkylthio. Examples of substituted lower alkyl groups include 2-hydroxyethyl, 2-methoxypropyl, 3-oxobutyl, cyanomethyl, trifluoromethyl, 2-nitropropyl, benzyl, including p-chloro-benzyl and p-methoxy-benryl, and 2-phenyl ethyl. The term "hydroxy lower alkyl" means a lower alkyl group which is mono- or di-substituted with hydroxy.

The term "cycloalkyl" means an unsubstituted or substituted 3- to 6- membered carbocyclic ring. Substituents useful in accordance with the present-invention are hydroxy, halogen, cyano, lower alkoxy, lower alkanoyl, lower alkyl, substituted lower alkyl, aroyl, lower alkylthio, lower alkyl sulfinyl, lower alkyl sulfonyl, aryl, heteroaryl and substituted amino. Preferred substitutents are hydroxy, halogen, lower alkoxy, lower alkyl, phenyl and benzyl.

The term "heterocyclyl" means an unsubstituted or substituted 5- to 6-membered carbocyclic ring in which one or two of the carbon atoms has been replaced by heteroatoms independently selected from O, S and N. "Heterocyclyl carbonyl" means a heterocyclyl group which is bonded to the rest of the molecule via a carbonyl group. Preferred heterocyclyl groups are pyrrolidinyl, piperidinyl, piperazinyl and morpholinyl. Substituents useful in accordance with the present invention are hydroxy, halogen, cyano, lower alkoxy, lower alkanoyl, lower alkyl, substituted lower alkyl, substituted lower alkoxy, aroyl, lower alkylthio, lower alkylsulfinyl, lower alkylsulfonyl, cycloalkyl, aryl, heteroaryl and substituted amino.

Preferred substitutents useful in accordance with the present invention are hydroxy, halogen, lower alkoxy, lower alkyl and benzyl.

The term "lower alkoxy" means a lower alkyl group (as defined above) bonded through an oxygen atom. Examples of unsubstituted lower alkoxy groups are methoxy, ethoxy, n-propoxy, isopropoxy, n-butoxy and tert-butoxy "Substituted lower alkoxy" means a lower alkoxy group substituted as described for lower alkyl. "Alkoxy lower alkoxy" means a lower alkoxy group substituted with a C₁₋₃ alkoxy. "Hydroxyalkoxy" means a lower alkoxy group which is mono- or disubstituted with hydroxy.

The term "lower alkylthio" means a lower alkyl group bonded through a divalent sulfur atom, for example, a methyl mercapto or an isopropyl mercapto group. The term "lower alkylsulfinyl" means a lower alkyl group as defined above bound to the rest of the molecule through the sulfur atom in the sulfinyl group. The term "lower alkylsulfonyl" means a lower alkyl group as defined above bound to the rest of the molecule through the sulfur atom in the sulfonyl group.

The term "aryl" means a monocylic aromatic group, such as phenyl, which is unsubstituted or substituted by one to three conventional substituent groups preferably selected from lower alkyl, lower alkoxy, hydroxy lower alkyl, hydroxy, hydroxyalkoxy, halogen, lower alkylthio, lower alkylsulfinyl, lower alkylsulfonyl, cyano, nitro, perfluoro lower alkyl, alkanoyl, phenyl, aroyl, aryl alkynyl, heteroaryl, lower alkynyl and lower alkanoylamino. Examples of aryl groups that may be used in accordance with this invention are unsubstituted phenyl, m- or o- nitrophenyl, p-tolyl, m- or p-methoxyphenyl, 3,4-dimethoxyphenyl, p-chlorophenyl, p-cyanophenyl, m-methylthiophenyl, 2-methyl-5-nitrophenyl, 2,6-dichlorophenyl and m-perfluorophenyl.

The term "aryloxy" means an aryl group, as hereinbefore defined which is bonded via an oxygen atom, "Arylthio" is aryl bonded via a sulfur atom.

The term "heteroaryl" means an unsubstituted or substituted 5- or 6-membered monocyclic heteroaromatic ring containing one to three heteroatoms which are independently N, S or O. Examples are pyridyl, thienyl, pyrimidinyl, oxazolyl, and furyl. Substituents as defined above for "aryl" are included in the definition of heteroaryl.

The term "perfluoro lower alkyl" means a lower alkyl group wherein all the hydrogens of the lower alkyl group are replaced by fluorine. Preferred perfluoro lower alkyl groups are trifluoromethyl and pentafluoroethyl.

The term "lower alkanoyl" means lower alkyl groups bonded to the rest of the molecule via a carbonyl group and embraces in the sense of the foregoing definition groups such as acetyl and propionyl. The term "perfluoro lower alkanoyl" means a perfluoro lower alkyl group which is bonded to the rest of the molecule via a carbonyl group. "Lower alkanoylamino" means a lower alkanoyl group bonded to the rest of the molecule via an amino group.

The term "aminosulfonyl" means an amino group bound to the rest of the molecule through the sulfur atom of a sulfonyl group wherein the amino may be optionally further mono- or di-substrtuted with methyl or ethyl.

The term "sulfonylamino" means a sulfonyl group bound to the rest of the molecule through the nitrogen atom of an amino group wherein the sulfonyl group may be optionally further substituted with methyl or ethyl.

The term "aroyl" means an aryl or heteroaryl group as defined bonded to the rest of the molecule via a carbonyl group. Examples of aroyl groups are benzoyl and 3-cyanobenzoyl.

The term "aryl lower alkoxy" means a lower alkoxy group in which one hydrogen atom is replaced by an aryl group. Benzyloxy is preferred.

The term "pharmaceutically acceptable salts" refers to conventional acid-addition salts or base-addition salts that retain the biological effectiveness and properties of the compounds of formulas I, I-A and I-B, and are formed from suitable non-toxic organic or inorganic acids, or organic or inorganic bases. Sample acid-addition salts include those derived from inorganic acids such as hydrochloric acid, hydrobromic acid, hydroiodic acid, sulfuric acid, sulfamic acid, phosphoric acid and nitric acid, and those derived from organic acids such as p-toluenesulfonic acid salicylic acid, methanesulfonic acid, oxalic acid, succinic acid, citric acid, malic acid, lactic acid and fumaric acid. Sample base-addition salts include those derived from ammonium, potassium, sodium and, quaternary ammonium hydroxides, such as for example, tetramethylammonium hydroxide. The chemical modification of a pharmaceutical compound (i.e., drug) into a salt is a technique well known to pharmaceutical chemists to obtain improved physical and chemical stability, hygroscopicity, flowability and solubility of compounds. See, e.g., H. Ansel et al., Pharmaceutical Dosage Forms and Drug Delivery Systems (6th Ed. 1995) at pp. 196 and 1456-1457.

Likewise, the term "pharmaceutically acceptable esters" refers to the well known practice in the pharmaceutical arts of preparing the non-toxic ester of a pharmaceutically active organic acid molecule, such as for example in the present invention where R⁴ or R⁵ are carboxy, which readily hydrolyze *in vivo* to thereby provide the active parent acid principle. It is accordingly understood that the claims presented hereinafter to compounds within Formula I include within their equivalent scope a corresponding pharmaceutically acceptable salt or ester.

In more detail, the present invention is concerned with compounds of the formula (I): 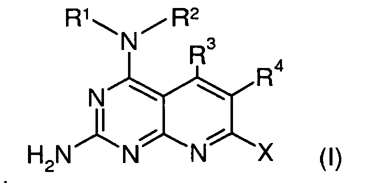
wherein X is a group X-1 of the formula: 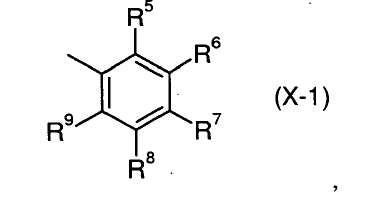
or X is a group X-2 of the formula: 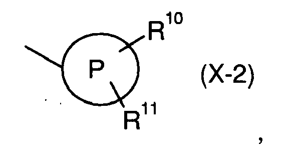
or X is a group X-3 of the formula: 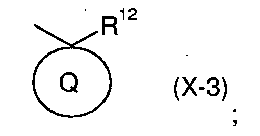
R¹ and R² are independently selected from the group consisting of hydrogen, lower alkyl, methoxy lower alkyl and hydroxy lower alkyl, except that R¹ and R² may not both be hydrogen;
R³ is hydrogen, lower alkyl or phenyl;
R⁴ is hydrogen, lower alkyl, lower alkylsulfonyl, phenyl, carboxy or together with R⁵ forms a 5-7 membered carbocyclic ring;
R⁵ when not fused in a ring with R⁴ is hydrogen, lower alkyl, substituted lower alkyl, lower alkoxy, substituted lower alkoxy, hydroxy, carboxy, halogen, lower alkylthio, lower alkylsulfinyl, lower alkylsulfonyl, aminosulfonyl, cyano, nitro, lower alkanoyl, aryl, aroyl, aryloxy, arylthio, perfluoro lower alkyl, lower alkylamino, lower alkanoylamino, sulfonylamino, cycloalkyl, cycloalkoxy, heterocyclyl, heterocyclyloxy, heterocyclylcarbonyl, heteroaryl, or together with R⁶ forms a second fused 5 or 6 membered aromatic ring;
R⁶ when not fused in a ring with R⁵ is hydrogen, lower alkyl, substituted lower alkyl, lower alkoxy, substituted lower alkoxy, hydroxy, halogen, lower alkylthio, lower alkylsulfinyl, lower alkylsulfonyl, aminosulfonyl, cyano, nitro, lower alkanoyl, aryl, aroyl, aryloxy, lower alkylamino, lower alkanoylamino, sulfonylamino, cycloalkyl, heterocyclyl, heterocyclyloxy or heterocyclylcarbonyl;
R⁷ is hydrogen, lower alkyl, lower alkoxy, alkoxy lower alkyl, alkoxy lower alkoxy, hydroxy lower alkyl, hydroxy, hydroxyalkoxy, halogen, lower alkylthio, lower alkylsulfinyl, lower alkylsulfonyl, perfluoro lower alkyl, lower alkanoyl, aroyl or lower alkanoylamino;
R⁸ and R⁹ are each independently selected from the group consisting of hydrogen, lower alkyl, substituted lower alkyl, lower alkoxy, substituted lower alkoxy, hydroxy, halogen, lower alkylthio, lower alkylsulfinyl, lower alkylsulfonyl, aminosulfonyl, cyano, nitro, lower alkanoyl, aryl, aroyl, aryloxy, lower alkylamino, lower alkanoylamino, sulfonylamino, cycloalkyl, heterocyclyl, heterocyclyloxy and heterocyclylcarbonyl;
P is a 5 or 6 membered heteroaromatic ring containing from 1 to 2 hetero atoms selected from the group consisting of oxygen, sulfur and nitrogen;
R¹⁰ and R¹¹ are each independently selected from the group consisting of hydrogen, lower alkyl, lower alkoxy, perfluoro lower alkyl, halogen, aryl lower alkyl, aryl and aryl lower alkoxy;
Q is a 3-6 membered cycloalkyl ring; and
R¹² is hydrogen or aryl;
or the pharmaceutically acceptable salts or esters thereof.

Preferred compounds are those of the formula (Ia): 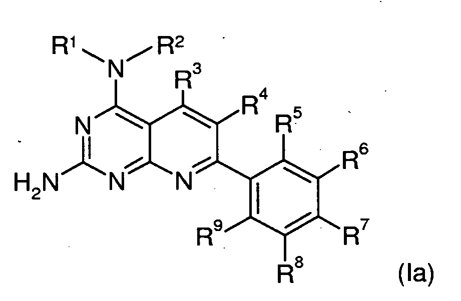 wherein R³ is hydrogen and R⁴ is hydrogen, lower alkyl, lower alkylsulfonyl, phenyl or carboxy, and R¹, R², R⁵, R⁶, R⁷, R⁸ and R⁹ are as defined above. Preferably, R⁶, R⁷ and R⁸ are each independently hydrogen, halogen, lower alkyl, lower alkoxy, hydroxy, hydroxy lower alkyl, lower alkylthio, lower alkyl sulfinyl, lower alkyl sulfonyl or perfluoro lower alkyl. More preferably, R⁷ is hydrogen or flourine. It is also preferred, that one of R⁶ and R⁸ is hydrogen or flourine. Preferably, one of R⁶ and R⁸ is hydrogen or fluorine and the other is halogen, lower alkyl, lower alkoxy, hydroxy, hydroxy lower alkyl, lower alkylthio, lower alkyl sulfinyl, lower alkyl sulfonyl or perfluoro lower alkyl. Most preferably, R⁶, R⁷ and R⁸ are hydrogen.

Other preferred compounds as defined above are those, wherein R⁵ and R⁹ are each independently selected from the group consisting of hydrogen, halogen, lower alkyl, lower alkoxy, alkoxy lower alkoxy, nitro, hydroxy, hydroxy lower alkoxy, hydroxy lower alkyl, lower alkylthio, lower alkylamino, lower alkyl sulfonyl, lower alkyl sulfinyl, perfluoro lower alkyl, cycloalkyl, cycloalkoxy, aryl, heteroaryl, aryloxy, arylthio and heterocyclyl. Preferably, R⁵ and R⁹ are each independently selected from the group consisting of chlorine, fluorine, trifluoromethyl, C1-4 alkyl, C1-3 alkylthio, C1-3 alkylsulfonyl, C1-3 alkoxy, phenoxy, phenoxy mono-substituted with fluorine, chlorine or oxygen, and C1-3 alkoxy substituted with hydroxy, methoxy or ethoxy.

It is preferred, that R¹. or R² is hydrogen. Preferably, the R¹ or R² which is substituted is substituted with C1-4 alkyl or hydroxy C1-3 alkyl.

Preferred compounds are those, wherein R⁶, R⁷ and R⁸ are each independently hydrogen, halogen, lower alkyl, lower alkoxy, hydroxy, hydroxy lower alkyl, lower alkylthio, lower alkyl sulfinyl, lower alkyl sulfonyl or perfluoro lower alkyl. Preferably, R⁷ is hydrogen or flourine. Preferably, one of R⁶ and R⁸ is hydrogen or flourine. More preferably, one of R⁶ and R⁸ is hydrogen or fluorine and the other is halogen, lower alkyl, lower alkoxy, hydroxy, hydroxy lower alkyl, lower alkylthio, lower alkyl sulfinyl, lower alkyl sulfonyl or perfluoro lower alkyl. Most preferably, R⁶, R⁷ and R⁸ are hydrogen.

Preferred compounds as defined above are those, wherein the R¹ or R² which is substituted is substituted with C1-4 alkyl or hydroxy C1-3 alkyl. Preferably, R⁵ and R⁹ are each independently selected from the group consisting of hydrogen, halogen, lower alkyl, lower alkoxy, alkoxy lower alkoxy, nitro, hydroxy, hydroxy lower alkoxy, hydroxy lower alkyl, lower alkylthio, lower alkylamino, lower alkyl sulfonyl, lower alkyl sulfinyl, perfluoro lower alkyl, cycloalkyl, cycloalkoxy, aryl, heteroaryl, aryloxy, arylthio and heterocyclyl. Preferably, the R¹ or R² which is substituted is substituted with C1-4 alkyl or hydroxy C1-3 alkyl.

Furthermore, it is preferred that R⁵ and R⁹ are each independently selected from the group consisting of chlorine, fluorine, trifluoromethyl, C1-4 alkyl, C1-3 alkylthio, C1-3 alkylsulfonyl, C1-3 alkoxy, phenoxy, phenoxy mono-substituted with fluorine, chlorine or oxygen, and C1-3 alkoxy substituted with hydroxy, methoxy or ethoxy. Preferably, the R¹ or R² which is substituted is substituted with C1-4 alkyl or hydroxy C1-3 alkyl.

Preferred compounds of formula (I) as defined above are those, wherein R⁴ and R⁵ form a 5-7 membered carbocyclic ring. Preferably, R¹ or R² is hydrogen. Preferably, R⁷ is hydrogen or flourine. Preferably, one of R⁶ and R⁸ is hydrogen. Preferably, one of R⁶ and R⁸ is hydrogen or fluorine and the other is halogen, lower alkyl, lower alkoxy, hydroxy, hydroxy lower alkyl, lower alkylthio, lower alkyl sulfinyl, lower alkyl sulfonyl or perfluoro lower alkyl. More preferably, R⁶, R⁷ and R⁸ are hydrogen. In such compounds, it is preferred that the R¹ or R² which is substituted is substituted with C1-4 alkyl or hydroxy C1-3 alkyl. Preferably, R⁵ and R⁹ are each independently hydrogen, halogen, lower alkyl, lower alkoxy, alkoxy lower alkoxy, nitro, hydroxy, hydroxy lower alkoxy, hydroxy lower alkyl, lower alkylthio, lower alkyl sulfinyl, lower alkyl sulfonyl, and perfluoro lower alkyl. Preferably, the R¹ or R² which is substituted is substituted with C1-4 alkyl or hydroxy C1-3 alkyl.

Another preferred embodiment of the present invention is related to compounds of the formula (Ib): 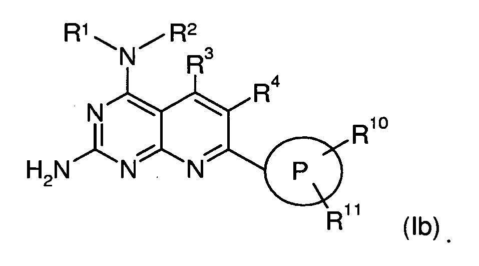 wherein R¹, R², R³, R⁴, P, R¹⁰ and R¹¹ are as defined above. Preferably, R¹ or R² is hydrogen. Preferably, R3 is hydrogen and R⁴ is hydrogen, lower alkyl, lower alkylsulfonyl, phenyl or carboxy. Preferably, R¹⁰ and R¹¹ are each independently lower alkyl, lower alkoxy, perfluoro lower alkyl or halogen. More preferably, the R¹ or R² which is substituted is substituted with C1-4 alkyl or hydroxy C1-3 alkyl. More preferably, the R¹ or R² which is substituted is substituted with C1-4 alkyl or hydroxy C1-3 alkyl.

Another preferred embodiment of the present invention is related to compounds of of the formula (Ic): 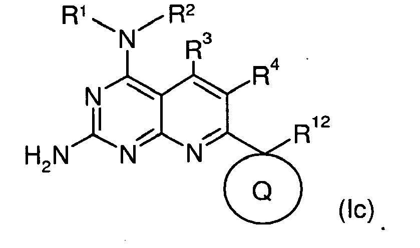 wherein R¹, R², R³, R⁴, Q and R¹² are as defined above. Preferably, R¹ or R² is hydrogen. Preferably, R³ is hydrogen and R⁴ is hydrogen, lower alkyl, lower alkylsulfonyl, phenyl or carboxy. Preferably, R¹² is unsubstituted or substituted phenyl. It is preferred that R¹² is mono-substituted phenyl. Preferably, the R¹ or R² which is substituted is substituted with C1-4 alkyl or hydroxy C1-3 alkyl.

Preferred compounds are those selected from the group consisting of
N4-Methyl-7-o-tolyl-pyrido[2,3-d]pyrimidine-2,4-diamine,
N4-Methyl-7-(2-trifluoromethyl-phenyl)-pyrido[2,3-d]pyrimidine-2,4-diamine,
7-(2,6-Dichloro-phenyl)-N4-methyl-pyrido[2,3-d]pyri midi ne-2,4-di ami ne,
7-(2-Chloro-phenyl)-N4-methyl-pyrido[2,3-d]pyrimidine-2,4-diamine,
7-(2,6-Difluoro-phenyl)-N4-methyl-pyrido[2,3-d]pyrimidine-2,4-diamine,
7-tert-Butyl-N4-methyl-pyrido[2,3-d]pyrimidine-2,4-diamine,
2-chloro-6-fluorophenyl-N4-methyl-pyrido[2,3-d]pyrimidine-2,4-diamine,
7-(2-Fluoro-6-trifluoromethyl-phenyl)-N4-methyl-pyrido[2,3-d]pyrimidine-2,4-diamine,
7-Cyclohexyl-N4-methyl-pyrido[2,3-d]pyrimidine-2,4-diamine,
7-(2-Methoxy-phenyl)-N4-methyl-pyrido[2,3-d]pyrimidine-2,4-diamine,
N4-Methyl-7-(2-nitro-phenyl)-pyrido[2,3-d]pyrimidine-2,4-diamine,
6,N4-Dimethyl-7-(2-trifluoromethyl-phenyl)-pyrido[2,3-d]pyrimidine-2,4-diamine,
N4-Methyl-7-thiophen-2-yl-pyrido[2,3-d]pyrimidine-2,4-diamine,
N4-Methyl-6,7-diphenyl-pyrido[2,3-d]pyrimidine-2,4-diamine,
7-(2,6-Dimethyl-phenyl)-N4-methyl-pyrido[2,3-d]pyrimidine-2,4-diamine,
7-(2,6-dimethyl-phenyl)-N4-ethyl-pyrido[2,3-d]pyrimidine-2,4-diamine,
7-(2-fluoro-6-trifluoromethyl-phenyl)-N4-methyl-pyrido[2,3-d]pyrimidine-2,4-diamine,
7-(2-Chloro-6-fluoro-phenyl)-N4-methyl-pyrido[2,3-d]pyrimidine-2,4-diamine,
N4-Methyl-7-(2,4,6-trimethyl-phenyl)-pyrido[2,3-d]pyrimidine-2,4-diamine,
7-(2-Bromo-phenyl)-N4-methyl-pyrido[2,3-d]pyrimidine-2,4-diamine,
7-(2-Benzyloxy-phenyl)-N4-methyl-pyrido[2,3-d]pyrimidine-2,4-diamine,
7-(2-Ethoxy-phenyl)-N4-methyl-pyrido[2,3-d]pyrimidine-2,4-diamine trifluoroacetic acid salt,
N4-Methyl-7-(2-p-tolyloxy-phenyl)-pyrido[2,3-d]pyrimidine-2,4-diamine trifluoroacetic acid salt,
N4-Methyl-6-propyl-7-(2-trifluoromethyl-phenyl)-pyrido[2,3-d]pyri midine-2,4-diamine,
N4-Methyl-7-(2,4-dimethyl-phenyl)-pyrido[2,3-d]pyrimidine-2,4-diamine, 7-(2,6-Dichloro-4-trifluoromethyl-phenyl)-N4-methyl-pyrido[2,3-d]pyrimidine-2,4-diamine trifluoroacetic acid salt,
N8-Methyl-5,6-dihydro-benzo[h]pyrimido[4,5-b]quinoline-8,10-diamine,
N4-Methyl-7-naphthalen-1-yl-pyrido[2,3-d]pyrimidine-2,4-diamine,
7-(2-lodo-phenyl)-N4-methyl-pyrido[2,3-d]pyrimidine-2,4-diamine trifluoroacetic acid salt,
7-(2-Fluoro-6-methoxy-phenyl)-N4-methyl-pyrido[2,3-d]pyrimidine-2,4-diamine trifluoroacetic acid salt,
7-(2-Ethoxy-6-fluoro-phenyl)-N4-methyl-pyrido[2,3-d]pyrimidine-2,4-diamine trifluoroacetic acid salt,
7-(2-Fluoro-6-isopropoxy-phenyl)-N4-methyl-pyrido[2,3-d]pyrimidine-2,4-diamine trifluoroacetic acid salt,
7-(2-Fluoro-6-propoxy-phenyl)-N4-methyl-pyrido[2,3-d]pyrimidine-2,4-diamine trifluoroacetic acid salt,
N4-Methyl-7-(2,3,5,6-tetramethyl-phenyl)-pyrido[2,3-d]pyrimidine-2,4-diamine trifluoroacetic acid salt,
N4-Methyl-7-phenyl-6-propyl-pyrido[2,3-d]pyrimidine-2,4-diamine trifluoroacetic acid salt,
6-Ethyl-N4-methyl-7-phenyl-pyrido[2,3-d]pyrimidine-2,4-diamine trifluoroacetic acid salt,
6-Methanesulfonyl-N4-methyl-7-(2-trifluoromethyl-phenyl)-pyrido[2,3-d]pyrimidine-2,4-diamine trifluoroacetic acid salt,
N4-Methyl-7-(2,3,6-trimethyl-phenyl)-pyrido[2,3-d]pyrimidine-2,4-diamine,
7-(2,6-Dichloro-3-fluoro-phenyl)-N4-methyl-pyrido[2,3-d]pyrimidine-2,4-diami ne,
7-(2,4-Bis-trifluoromethyl-phenyl)-N4-methyl-pyrido[2,3-d]pyrimidine-2,4-diami ne,
7-(2,6-Bis-trifluoromethyl-phenyl)-N4-methyl-pyrido[2,3-d]pyrimidine-2,4-diamine,
7-(2,5-Dimethyl-phenyl)-N4-methyl-pyrido[2,3-d]pyrimidine-2,4-diamine,
N4-Methyl-7-(2,3,6-trichloro-phenyl)-pyrido[2,3-d]pyrimidine-2,4-diami ne,
2-(2-Amino-4-methylamino-pyrido[2,3-d]pyrimidin-7-yl)-4-methyl-phenol,
N9-Methyl-6,7-dihydro-5H-10,12,13-triaza-benzo[3,4]cyclohepta[1,2-b]naphthalene-9,11-diamine trifluoroacetic acid salt,
6-lsopropyl-N4-methyl-7-phenyl-pyrido[2,3-d]pyrimidine-2,4-diamine trifluoroacetic acid salt,
2-(2-Amino-4-methylamino-pyrido[2,3-d]pyrimidin-7-yl)-phenol trifluoroacetic acid salt,
7-(2,5-Dichloro-phenyl)-N4-methyl-pyrido[2,3-d]pyrimidine-2,4-diamine trifluoroacetic acid salt,
7-(2,4-Dichloro-phenyl)-N4-methyl-pyrido[2,3-d]pyrimidine-2,4-diamine trifluoroacetic acid salt,
7-(2,3-Dichloro-phenyl)-N4-methyl-pyrido[2,3-d]pyri midi ne-2,4-diami ne trifluoroacetic acid salt,
N4-Methyl-6-phenethyl-7-phenyl-pyrido[2,3-d]pyrimidine-2,4-diamine trifluoroacetic acid salt,
7-(2-Cyclopentyloxy-6-fluoro-phenyl)-N4-methyl-pyrido[2,3-d]pyrimidine-2,4-diamine trifluoroacetic acid salt,
2-[2-(2-Amino-4-methylamino-pyrido[2,3-d]pyrimidin-7-yl)-3-fluoro-phenoxy]-ethanol trifluoroacetic acid,
3-[2-(2-Amino-4-methylamino-pyrido[2,3-d]pyrimidin-7-yl)-3-fluoro-phenoxy]-propan-1-ol trifluoroacetic acid,
7-(2-Chloro-6-ethoxy-phenyl)-N4-methyl-pyrido[2,3-d]pyri midi ne-2,4-diami ne trifluoroacetic acid salt,
2-Amino-4-methylamino-7-(2-trifluoromethyl-phenyl)-pyrido[2,3-d]pyrimidine-6-carboxylic acid trifluoroacetic acid salt,
N4-Methyl-7-(1-phenyl-cyclopropyl)-pyrido[2,3-d]pyrimidine-2,4-diamine,
N4-Methyl-7-(1-phenyl-cyclopentyl)-pyrido[2,3-d]pyrimidine-2,4-diamine,
N4-Methyl-7-(1-phenyl-cyclohexyl)-pyrido[2,3-d]pyrimidine-2,4-diamine,
potassium 2-(2-Amino-4-methylamino-pyrido[2,3-d]pyrimidin-7-yl)-benzoate,
7-(2,4-Diethyl-phenyl)-N4-methyl-pyrido[2,3-d]pyrimidi ne-2,4-diami ne,
N4-Ethyl-7-(2-trifluoromethyl-phenyl)-pyrido[2,3-d]pyri midi ne-2,4-diami ne trifluoroacetic acid salt,
N4-Ethyl-6-methyl-7-(2-trifluoromethyl-phenyl)-pyrido[2,3-d]pyrimidine-2,4-diamine,
N4-Ethyl-7-o-tolyl-pyrido[2,3-d]pyrimidine-2,4-diamine trifluoroacetic acid salt, 7-(2,6-Dichloro-phenyl)-N4-ethyl-pyrido[2,3-d]pyrimidine-2,4-diamine trifluoroacetic acid salt,
7-(2-Bromo-phenyl)-N4-ethyl-pyrido[2,3-d]pyrimidine-2,4-diamine trifluoroacetic acid salt,
N4-Ethyl-7-(2-fluoro-6-trifluoromethyl-phenyl)-pyrido[2,3-d]pyrimidine-2,4-diamine trifluoroacetic acid salt,
7-(2-Chloro-6-fluoro-phenyl)-N4-ethyl-pyrido[2,3-d]pyrimidine-2,4-diamine trifluoroacetic acid salt,
N4-Ethyl-7-(2,3,6-trimethyl-phenyl)-pyrido[2,3-d]pyrimidine-2,4-diamine trifluoroacetic acid salt,
2-[2-Amino-7-(2,6-dichloro-phenyl)-pyrido[2,3-d]pyrimidin-4-ylamino]-ethanol,
N4-Methyl-7-(2-pi peridi n-1-yl-6-trifluoro methyl-phenyl)-pyrido[2,3-d]pyri midi ne-2,4-diamine,
N4-Methyl-7-(2-morpholin-4-yl-6-trifluoromethyl-phenyl)-pyrido[2,3-d]pyrimidine-2,4-diamine trifluoroacetic acid salt,
7-(2,4-Dimethyl-phenyl)-N4-methyl-pyrido[2,3-d]pyrimidine-2,4-diamine,
N4-Methyl-7-[2-(4-methyl-piperazin-1-yl)-6-trifluoromethyl-phenyl]-pyrido[2,3-d]pyrimidine-2,4-diamine trifluoroacetic acid salt,
7-(2-Ethoxy-6-trifluoromethyl-phenyl)-N4-methyl-pyrido[2,3-d]pyri midi ne-2,4-diamine trifluoroacetic acid salt,
7-(2-Methoxy-6-trifluoromethyl-phenyl)-N4-methyl-pyrido[2,3-d]pyrimidine-2,4-diamine trifluoroacetic acid salt,
7-(2-Dimethylamino-6-trifluoromethyl-phenyl)-N4-methyl-pyrido[2,3-d]pyrimidine-2,4-diamine,
N4-Methyl-7-(2-methylamino-6-trifluoromethyl-phenyl)-pyrido[2,3-d]pyrimidine-2,4-diamine trifluoroacetic acid salt,
7-[2-(2-Dimethylamino-ethoxy)-6-trifluoromethyl-phenyl]-N4-methyl-pyrido[2,3-d]pyrimidine-2,4-diamine trifluoroacetic acid salt,
N4-Methyl-7-(2-phenoxy-6-trifluoromethyl-phenyl)-pyrido[2,3-d]pyrimidine-2,4-diamine trifluoroacetic acid salt,
N4-Methyl-7-(2-methylsulfanyl-6-trifluoromethyl-phenyl)-pyrido[2,3-d]pyrimidine-2,4-diamine trifluoroacetic acid salt,
2-[2-(2-Amino-4-methylamino-pyrido[2,3-d]pyrimidin-7-yl)-3-trifluoromethyl-phenoxy]-ethanol trifluoroacetic acid salt,
7-[2-(2-Methoxy-ethoxy)-6-trifluoromethyl-phenyl]-N4-methyl-pyrido[2,3-d]pyrimidine-2,4-diamine trifluoroacetic acid salt,
N4-Methyl-7-[2-(2-pyrrolidin-1-yl-ethoxy)-6-trifluoromethyl-phenyl]-pyrido[2,3-d]pyrimidine-2,4-diamine trifluoroacetic acid salt,
7-(2-Isopropoxy-6-trifluoromethyl-phenyl)-N4-methyl-pyrido[2,3-d]pyrimidine-2,4-diamine trifluoroacetic acid salt,
N4-Methyl-7-(2-propoxy-6-trifluoromethyl-phenyl)-pyrido[2,3-d]pyri midi ne-2,4-diamine trifluoroacetic acid salt,
7-[2-(2-Diethylamino-ethoxy)-6-trifluoromethyl-phenyl]-N4-methyl-pyrido[2,3-d]pyrimidine-2,4-diamine trifluoroacetic acid salt,
N4-Methyl-7-[2-(2-morpholi n-4-yl-ethoxy)-6-trifluoromethyl-phenyl]-pyrido[2,3-d]pyrimidine-2,4-diamine trifluoroacetic acid salt,
7-(2-fluoro-6-pyrrolidi n-1-yl-phenyl)-N4-methyl-pyrido[2,3-d]pyri midi ne-2,4-diami ne,
7-(2-Fluoro-6-piperidin-1-yl-phenyl)-N4-methyl-pyrido[2,3-d]pyrimidine-2,4-diamine,
2-(2-amino-4-methylamino-pyrido[2,3-d]pyrimidin-7-yl)-3-fluoro-phenol,
7-(2-Fluoro-6-morpholino-4-yl-phenyl)-N4-methyl-pyrido[2,3-d]pyrimidine-2,4-diamine,
7-(2-Fluoro-6-phenylsulfanyl-phenyl)-N4-methyl-pyrido[2,3-d]pyrimidine-2,4-diamine trifluoroacetic acid salt,
7-(2-Fluoro-6-phenoxy-phenyl)-N4-methyl-pyrido[2,3-d]pyrimidine-2,4-diamine trifluoroacetic acid,
7-(2-Fluoro-6-i midazol-1-yl-phenyl)-N4-methyl-pyrido[2,3-d]pyri midi ne-2,4-diami ne,
7-[2-(4-Benzyl-piperazin-1-yl)-6-fluoro-phenyl]-N4-methyl-pyrido[2,3-d]pyrimidine-2,4-diamine trifluoroacetic acid salt,
7-(2-Fluoro-6-methylamino-phenyl)-N4-methyl-pyrido[2,3-d]pyrimidine-2,4-diamine trifluoroacetic acid salt,
7-(2-Di methylami no-6-fluoro-phenyl)-N4-methyl-pyrido[2,3-d]pyri midi ne-2,4-diamine trifluoroacetic acid salt,
7-[2-Fluoro-6-(4-methyl-piperazin-1-yl)-phenyl]-N4-methyl-pyrido[2,3-d]pyrimidine-2,4-diamine trifluoroacetic acid salt,
1-[2-(2-Amino-4-methylamino-pyrido[2,3-d]pyrimidin-7-yl)-3-fluoro-phenyl]-piperidine-2-carboxylic acid ethyl ester trifluoroacetic acid salt,
7-(2-Fluoro-6-thiomorpholin-4-yl-phenyl)-N4-methyl-pyrido[2,3-d]pyrimidine-2,4-diamine trifluoroacetic acid salt,
7-[2-(3-Aminomethyl-piperidin-1-yl)-6-fluoro-phenyl]-N4-methyl-pyrido[2,3-d]pyrimidine-2,4-diamine trifluoroacetic acid salt,
7-[2-Fluoro-6-(2-methoxymethyl-pyrrolidin-1-yl)-phenyl]-N4-methyl-pyrido[2,3-d]pyrimidine-2,4-diamine trifluoroacetic acid salt,
7-[2-(4-Fluoro-phenoxy)-6-trifluoromethyl-phenyl]-N4-methyl-pyrido[2,3-d]pyrimidine-2,4-diamine trifluoroacetic acid salt,
7-(2-Cyclohexyloxy-6-trifluoromethyl-phenyl)-N4-methyl-pyrido[2,3-d]pyrimidine-2,4-diamine trifluoroacetic acid salt,
N4-Methyl-7-[2-(2-methyl-pyrrolidin-1-yl)-6-trifluoromethyl-phenyl]-pyrido[2,3-d]pyrimidine-2,4-diamine,
7-[2-(2,5-Di methyl-pyrrolidi n-1-yl)-6-trifluoromethyl-phenyl]-N4-methyl-pyrido[2,3-d]pyrimidine-2,4-diamine trifluoroacetic acid salt,
1-[2-(2-Amino-4-methylamino-pyrido[2,3-d]pyrimidin-7-yl)-3-trifluoromethyl-phenyl]-pyrrolidin-3-ol trifluoroacetic acid salt,
1-[2-(2-Amino-4-methylamino-pyrido[2,3-d]pyrimidin-7-yl)-3-trifluoromethyl-phenyl]-piperidin-4-ol trifluoroacetic acid salt,
2-[2-Amino-7-(2-phenoxy-6-trifluoromethyl-phenyl)-pyrido[2,3-d]pyrimidin-4-ylamino]-ethanol trifluoroacetic acid salt,
{1-[2-(2-Amino-4-methylamino-pyrido[2,3-d]pyrimidin-7-yl)-3-trifluoromethyl-phenyl]-pyrrolidin-2-yl}-methanol trifluoroacetic acid salt,
7-[2-(2-Methoxymethyl-pyrrolidin-1-yl)-6-trifluoromethyl-phenyl]-N4-methyl-pyrido[2,3-d]pyrimidine-2,4-diamine trifluoroacetic acid salt,
1-[2-(2-Amino-4-methylamino-pyrido[2,3-d]pyrimidin-7-yl)-3-trifluoromethyl-phenyl]-piperidin-3-ol trifluoroacetic acid salt,
7-[2-(4-Cyclohexyl-piperazin-1-yl)-6-trifluoromethyl-phenyl]-N4-methyl-pyrido[2,3-d]pyrimidine-2,4-diamine trifluoroacetic acid salt,
7-[2-(4-Ethyl-piperazin-1-yl)-6-trifluoromethyl-phenyl]-N4-methyl-pyrido[2,3-d]pyrimidine-2,4-diamine trifluoroacetic acid salt,
{4-[2-(2-Amino-4-methylamino-pyrido[2,3-d]pyrimidin-7-yl)-3-trifluoromethylphenyl]-piperazin-1-yl}-furan-2-yl-methanone trifluoroacetic acid salt,
7-(2-{4-[Bis-(4-fluoro-phenyl)-methyl]-piperazin-1-yl}-6-trifluoromethyl-phenyl)-N4-methyl-pyrido[2,3-d]pyrimidine-2,4-diamine trifluoroacetic acid salt,
N4-Methyl-7-[2-(4-phenyl-piperazin-1-yl)-6-trifluoromethyl-phenyl]-pyrido[2,3-d]pyrimidine-2,4-diamine trifluoroacetic acid salt,
7-[2-(4-Benzyl-piperazin-1-yl)-6-trifluoromethyl-phenyl]-N4-methyl-pyrido[2,3-d]pyrimidine-2,4-diamine trifluoroacetic acid salt,
1-[2-(2-Amino-4-methylamino-pyrido[2,3-d]pyrimidin-7-yl)-3-trifluoromethyl-phenyl]-4-phenyl-piperidin-4-ol trifluoroacetic acid salt,
7-[2-(4-Benzyl-piperidin-1-yl)-6-trifluoromethyl-phenyl]-N4-methyl-pyrido[2,3-d]pyrimidine-2,4-diamine trifluoroacetic acid salt,
7-[2-(4-Cyclopentyt-piperazin-1-yl)-6-trifluoromethyl-phenyl]-N4-methyl-pyrido[2,3-d]pyrimidine-2,4-diamine trifluoroacetic acid salt,
2-{4-[2-(2-Amino-4-methylamino-pyrido[2,3-d]pyrimidin-7-yl)-3-trifluoromethylphenyl]-piperazin-1-yl}-N-isopropyl-acetamide trifluoroacetic acid salt,
7-[2-(4-Isopropyl-piperazin-1-yl)-6-trifluoromethyl-phenyl]-N4-methyl-pyrido[2,3-d]pyrimidine-2,4-diamine trifluoroacetic acid salt,
7-[2-(2-Fluoro-phenoxy)-6-trifluoromethyl-phenyl]-N4-methyl-pyrido[2,3-d]pyrimidine-2,4-diamine trifluoroacetic acid salt,
7-[2-(3-Fluoro-phenoxy)-6-trifluoromethyl-phenyl]-N4-methyl-pyrido[2,3-d]pyrimidine-2,4-diamine trifluoroacetic acid salt,
7-[2-(3-Chloro-phenoxy)-6-trifluoromethyl-phenyl]-N4-methyl-pyrido[2,3-d]pyrimidine-2,4-diamine trifluoroacetic acid salt,
7-[2-(4-Chloro-phenoxy)-6-trifluoromethyl-phenyl]-N4-methyl-pyrido[2,3-d]pyrimidine-2,4-diamine trifluoroacetic acid salt,
N4-Methyl-7-(2-p-tolyloxy-6-trifluoromethyl-phenyl)-pyrido[2,3-d]pyrimidine-2,4-diamine trifluoroacetic acid salt,
7-[2-(Biphenyl-4-yloxy)-6-trifluoromethyl-phenyl]-N4-methyl-pyrido[2,3-d]pyrimidine-2,4-diamine trifluoroacetic acid salt,
2-[2-Amino-7-(2-pyrrolidin-1-yl-6-trifluoromethyl-phenyl)-pyrido[2,3-d]pyrimidin-4-ylamino]-ethanol trifluoroacetic acid salt,
4-[2-amino-7-(2-trifluoromethyl-phenyl)-pyrido[2,3-d]pyrimidin-4-ylamino]-propan-1-ol trifluoroacetic acid salt,
2-[2-Amino-7-(2-trifluoromethyl-phenyl)-pyrido[2,3-d]pyrimidin-4-ylamino]-ethanol,
N4-Propyl-7-(2-trifluoromethyl-phenyl)-pyrido[2,3-d]pyrimidine-2,4-diamine trifluoroacetic acid salt,
4-[2-Ami no-7-(2-trifluoromethyl-phenyl)-pyrido[2,3-d]pyri midi n-4-ylami no]-butan-1-ol trifluoroacetic acid salt,
2-[2-Amino-7-(2-fluoro-6-trifluoromethyl-phenyl)-pyrido[2,3-d]pyrimidin-4-ylamino]-ethanol,
2-[2-Amino-7-(2-bromo-phenyl)-pyrido[2,3-d]pyrimidin-4-ylamino]-ethanol,
5,N-4-Dimethyl-7-phenyl-pyrido[2,3-d]pyrimidine-2,4-diamine trifluoroacetate, and
N-4-Methyl-5,7-diphenyl-pyrido[2,3-d]pyrimidine-2,4-diamine trifluoroacetate,
and pharmaceutically acceptable salts and esters thereof.

Particularly preferred compounds are those selected from the group consisting of
N4-Methyl-7-(2-trifluoromethyl-phenyl)-pyrido[2,3-d]pyrimidine-2,4-diamine,
7-(2,6-Dichloro-phenyl)-N4-methyl-pyrido[2,3-d]pyrimidine-2,4-diamine,
2-chloro-6-fluorophenyl-N4-methyl-pyrido[2,3-d]pyrimidine-2,4-diamine,
7-(2-Fluoro-6-trifluoromethyl-phenyl)-N4-methyl-pyrido[2,3-d]pyri midi ne-2,4-diamine,
7-(2-Chloro-6-fluoro-phenyl)-N4-methyl-pyrido[2,3-d]pyrimidine-2,4-diamine,
7-(2,6-Dichloro-3-fluoro-phenyl)-N4-methyl-pyrido[2,3-d]pyri midi ne-2,4-diami ne,
7-(2,6-Bis-trifluoromethyl-phenyl)-N4-methyl-pyrido[2,3-d]pyrimidine-2,4-diamine,
7-(2-Ch loro-6-ethoxy-phenyl)-N4-methyl-pyrido[2,3-d]pyri midi ne-2,4-diami ne trifluoroacetic acid salt,
2-Amino-4-methylamino-7-(2-trifluoromethyl-phenyl)-pyrido[2,3-d]pyrimidine-6-carboxylic acid trifluoroacetic acid salt,
7-(2,4-Dimethyl-phenyl)-N4-methyl-pyrido[2,3-d]pyrimidine-2,4-diamine,
7-(2-Ethoxy-6-trifluoromethyl-phenyl)-N4-methyl-pyrido[2,3-d]pyrimidine-2,4-diamine trifluoroacetic acid salt,
N4-Methyl-7-(2-phenoxy-6-trifluoromethyl-phenyl)-pyrido[2,3-d]pyrimidine-2,4-diamine trifluoroacetic acid salt,
7-(2-Isopropoxy-6-trifluoromethyl-phenyl)-N4-methyl-pyrido[2,3-d]pyrimidine-2,4-diamine trifluoroacetic acid salt,
7-[2-(4-Fluoro-phenoxy)-6-trifluoromethyl-phenyl]-N4-methyl-pyrido[2,3-d]pyrimidine-2,4-diamine trifluoroacetic acid salt,
7-(2-Cyclohexyloxy-6-trifluoromethyl-phenyl)-N4-methyl-pyrido[2,3-d]pyrimidine-2,4-diamine trifluoroacetic acid salt,
7-[2-(2-Fluoro-phenoxy)-6-trifluoromethyl-phenyl]-N4-methyl-pyrido[2,3-d]pyrimidine-2,4-diamine trifluoroacetic acid salt,
7-[2-(3-Fluoro-phenoxy)-6-trifluoromethyl-phenyl]-N4-methyl-pyrido[2,3-d]pyrimidine-2,4-diamine trifluoroacetic acid salt,
7-[2-(4-Chloro-phenoxy)-6-trifluoromethyl-phenyl]-N4-methyl-pyrido[2,3-d]pyrimidine-2,4-diamine trifluoroacetic acid salt, and
N4-Methyl-7-(2-p-tolyloxy-6-trifluoromethyl-phenyl)-pyrido[2,3-d]pyrimidine-2,4-diamine trifluoroacetic acid salt,
and pharmaceutically acceptable salts and esters thereof.

It is preferred that the lower alkyl, methoxy lower alkyl, and hydroxy lower alkyl groups of R¹ and R² have up to 4 carbon atoms with C1-4 alkyl and hydroxy C1-3 alkyl being more preferred; and it is most preferable that one of R¹ or R² is hydrogen.

R³ and R⁴ are preferably hydrogen. Preferred substituents for R⁵ and R⁹ are hydrogen, halogen, lower alkyl, lower alkoxy, alkoxy lower alkoxy, nitro, hydroxy, hydroxy lower alkoxy, hydroxy lower alkyl, lower alkylthio, lower alkylamino, lower alkyl sulfonyl, lower alkyl sulfinyl, perfluoro lower alkyl, cycloalkyl, cycloalkoxy, aryl, heteroaryl, aryloxy, arylthio and heterocyclyl. Chlorine, fluorine, trifluoromethyl, C1-4 alkyl, C1-3 alkylthio, C1-3 alkylsulfonyl, C1-3 alkoxy, C1-3 alkoxy substituted with a group selected from hydroxy, methoxy and ethoxy, phenoxy and phenoxy mono-substituted with fluorine, chlorine or oxygen are still more preferred. Preferred substituents for R⁶ and R⁸ are hydrogen, halogen, lower alkyl, lower alkoxy, alkoxy lower alkoxy, nitro, hydroxy, hydroxy lower alkoxy, hydroxy lower alkyl, lower alkylthio, lower alkylamino, lower alkyl sulfonyl, and perfluoro lower alkyl. Hydrogen, chlorine, fluorine, trifluoromethyl, C1-4 alkyl, C1-3 alkylthio, C1-3 alkylsulfonyl, C1-3 alkoxy, C1-3 alkoxy substituted with a group selected from hydroxy, methoxy and ethoxy are further preferred. Hydrogen is more preferred. R⁷ is preferably hydrogen, lower alkyl and perfluoro lower alkyl. Hydrogen is most preferred.

Compounds of formula (I) are individually preferred, pharmaceutically acceptable salts thereof are individually preferred and pharmaceutically acceptable esters thereof are individually preferred, with the compounds of formula (I) being particularly preferred.

The compounds of formula (I) can have one or more asymmetric C atoms and can therefore exist as an enantiomeric mixture, mixture of stereoisomers or as optically pure compounds.

It will be appreciated that the compounds of general formula (I) in this invention may be derivatised at functional groups to provide derivatives which are capable of conversion back to the parent compound in vivo.

Another preferred embodiment of the present invention is concerned with a process for the preparation of compounds as defined above, comprising reacting a compound of formula (II) 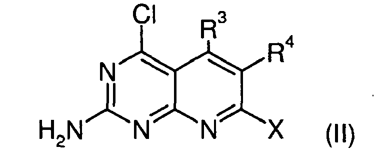 with a compound HN(R¹,R²), wherein R¹, R², R³, R⁴ and X are as defined above.

Another embodiment of the present invention is related to compounds as defined above, when manufactured by a process as defined above.

Reaction conditions for the process are known in the art or from the description, schemes and examples given below.

The invention is also concerned with pharmaceutical compositions comprising a compound of formula (I) as defined above and a pharmaceutically acceptable carrier and/or adjuvant.

Another embodiment of the present invention relates to compounds as defined above for use as therapeutic active substances, particularly for use as therapeutic active substances for the treatment and/or prophylaxis of diseases which are modulated by PTP-1B inhibitors, particularly diseases which are associated with high blood glucose concentration, particularly type 1 diabetes, type 2 diabetes, diabetes related diseases, impaired glucose tolerance, impaired insulin sensitivity or obesity.

The invention also embraces a method for the therapeutic and/or prophylactic treatment of diseases which are modulated by PTP-1 B inhibitors, particularly for the therapeutic and/or prophylactic treatment of diseases which are associated with high blood glucose concentration, particularly type 1 diabetes, type 2 diabetes, diabetes related diseases, impaired glucose tolerance, impaired insulin sensitivity or obesity, which method comprises administering a compound of formula (I) as defined above to a human being or animal.

The invention furthermore relates to the use of compounds of formula (I) as defined above for the therapeutic and/or prophylactic treatment of diseases which are modulated by PTP-1 B inhibitors, particularly diseases which are associated with high blood glucose concentration, especially type 1 diabetes, type 2 diabetes, diabetes related diseases, impaired glucose tolerance, impaired insulin sensitivity or obesity.

The invention also relates to the use of compounds of formula (I) as defined above for the preparation of medicaments for the therapeutic and/or prophylactic treatment of diseases which are modulated by PTP-1 B inhibitors, particularly diseases which are associated with high blood glucose concentration, especially type 1 diabetes, type 2 diabetes, diabetes related diseases, impaired glucose tolerance, impaired insulin sensitivity or obesity.

Of the diseases mentioned above, diabetes is the preferred medical indication, particularly type II diabetes.

Intravenous, intramuscular, oral or inhalation administrations are preferred forms of use. The dosages in which the compounds of the invention are administered in effective amount depend on the nature of the specific active ingredient, the age and requirements of the patient and the mode of administration. Dosages may be determined by any conventional means, e.g., by dose-limiting clinical trials. In general, dosages of about 0.1 to 20 mg/kg body weight per day are preferred, with dosages of 0,5-10 mg/kg per day being particularly preferred.

The invention further comprises pharmaceutical compositions that contain a pharmaceutically effective amount of a compound of the invention and a pharmaceutically acceptable carrier. Such compositions may be formulated by any conventional means. Tablets or granulates can contain a series of binders, fillers, carriers or diluents. Liquid compositions can be, for example, in the form of a sterile water-miscible solution. Capsules can contain a filler or thickener in addition to the active ingredient. Furthermore, flavor-improving additives as well as substances usually used as preserving, stabilizing, moisture-retaining and emulsifying agents as well as salts for varying the osmotic pressure, buffers and other additives can also be present. The previously mentioned carrier materials and diluents can comprise any conventional pharmaceutically acceptable organic or inorganic substances, e.g., water, gelatine, lactose, starch, magnesium stearate, talc, gum Arabic and polyalkylene glycols.

Oral unit dosage forms, such as tablets and capsules, preferably contain from 1 mg to 250 mg of a compound of this invention, The compounds of the invention may be prepared by conventional means.

In accordance with this invention, the compounds herein as well as their pharmaceutically acceptable salts are useful in the control or prevention of illnesses associated with high blood glucose concentration. A preferred indication associated with the present invention is that associated with diabetes.

The dosage can vary within wide limits and will, of course, have to be adjusted to the individual requirements in each particular case. In the case of oral administration, the dosage for adults may vary from about 1 mg to about 1000 mg per day of a compound of formula I, or of the corresponding amount of a pharmaceutically acceptable salt thereof. The daily dosage may be administered as single dose or in divided doses, and in addition, the upper limit can also be exceeded when this is found to be indicated.

The methods for preparing the compounds of this invention are described in the following schemes:

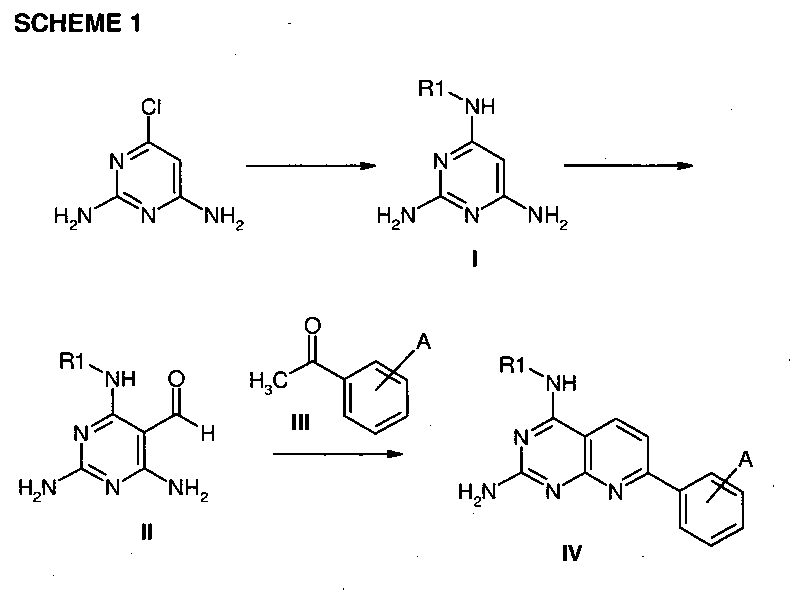

**SCHEME 1** describes a general method for the synthesis of pyrido[2,3-d]pyrimidine-2,4-diamine analogs **IV** bearing R1 group at N-4 and substituted (A group) phenyl at C-7. Alkylamine displacement of 6-chloro-2,4-diaminopyrimidine to give 2,4-diamino-6-alkylaminopyrimidine **I** was carried out using similar procedures described by Elion, G.B. et al., J. Am. Chem. Soc. 1953, 75, 4311. 2,4-diamino-6-alkylaminopyrimidine **I** was then formylated to give 2,4-diamino-6-alkylaminopyrimidine-5-carbaldehyde **II** according to the procedures described by Delia, T.J. et al., Heterocycles 1983, 20, 1805. Friedlander condensation of 2,4-diamino-6-alkylaminopyrimidine-5-carbaldehyde **II** and substituted acetophenone **III** was carried out in a similar fashion as described by Evens, G. et al., J. Org. Chem. 1975, 40, 1438 and Perandones, F. et al., J. Heterocyclic Chem. 1998, 35, 413 to give the desired product **IV.**

Substituted acetophenones **III** used in the Friedlander condensation reactions (**SCHEME 1**) are either commercially available or could be prepared using conventional synthetic methods: (a) from substituted benzoic acids, see e.g. Jorgenson, M.J. Org. React. 1970, 18, 1; (b) from substituted benzaldehydes, see e.g. Tanouchi, T. et al., J. Med. Chem. 1981, 24, 1149; (c) from substituted phenoltriflates (in turn prepared from substituted phenols), see e.g. Garrido, F. et al., Tet. Lett. 2001, 42, 265; (d) from substituted aryl iodides, see e.g. Cacchi, S. et al., Org. Letters. 2003, 5, 289.

The following procedures used in the synthesis of N4-Methyl-7-(2,4,6-trimethylphenyl)-pyrido[2,3-d]pyrimidine-2,4-diamine (IV, R1 = CH₃, A = 2,4,6-trimethyl) exemplify the typical reaction conditions described in **SCHEME 1**:
Compound **I**: To 6-chloro-2,4-diaminopyrimidine (5.0 g, 0.0347 mole) was added 25 mL of 25% aqueous MeNH₂ solution (0.182 mole, prepared from 40% aqueous MeNH₂ solution) in a sealed tube. The reaction was heated at 150°C for 4.5 hours. TLC (1/9/90 v/v/v conc.NH₄OH/MeOH/CH₂Cl₂) analysis indicated complete disappearance of starting material. The reaction was then cooled to room temperature and concentrated to give a crude oil. The crude was absorbed onto silica gel using methanol as solvent. The crude material on silica gel was purified using silica gel chromatography (conc.NH₄OH/MeOH/CH₂Cl₂) to give 3.98 g of an impure material. Recrystallization of the impure material from 45 mL of hot ethanol gave 1.57 g (11.3 mmole, 33% yield) of 2,4-diamino-6-methylaminopyrimidine I as an off-white solid. ¹H NMR (DMSO-d₆, 300 MHz) δ 5.9 (broad s, 1 H), 5.5 (broad s, 2H), 5.3 (broad s, 2H), 4.76 (s, 1 H), 2.60 (broad s, 3H).
Compound **II:** To a 250 mL three-necked round bottom flask equipped with a magnetic stirrer, argon inlet and thermometer was added *N,N*-dimethylformamide (20 mL, anhydrous). The flask was cooled in a dry ice/ethylene glycol bath and phosphorus oxychloride (1.97 mL, 21.14 mmol) was added slowly at a rate so as to keep the internal temperature below 0°C. 2,4-diamino-6-methylaminopyrimidine **I** (2.20 g, 15.8 mmole) was then carefully added as a slurry in *N,N-*dimethylformamide (20 mL, anhydrous) (Exothermic!). The reaction was transferred to a 40°C oil bath and stirred for 1.5 hours. The reaction was quenched with ice (∼70 g) and sodium hydroxide pellets (4 g) was added to make the solution slightly basic (pH ∼ 8). The mixture was then heated in a 90°C oil bath until methylamine gas was no longer evolved from the mixture. Sodium hydroxide pellets were added as needed to keep the pH of mixture ∼8. The reaction was then cooled to room temperature and concentrated to give a crude solid. The crude was absorbed onto silica gel using methanol as solvent. Silica gel chromatography (Isco 120 g, conc. NH₄OH/MeOH/CH₂Cl₂) gave 1.23 g (7.36 mmole, 47% yield) of 2,4-diamino-6-methylaminopyrimidine-5-carbaldehyde **II** as a light brown solid. ¹H NMR (DMSO-d₆, 300 MHz) δ 9.68 (s, 1 H), 9.1 (broad s, 1 H), 6.85 (broad s, 2H), 6.5 (broad s, 2H), 2.80 (broad s, 3H).
Compound **IV:** A mixture of 2,4-diamino-6-methylaminopyrimidine-5-carbaldehyde **II** (100 mg, 0.60 mmole), 2',4',6'-trimethylacetophenone (**III**, A = 2,4,6-trimethyl, 200 mg, 1.23 mmole), potassium hydroxide pellet (100 mg, 1.79 mmole) and ethanol (4 mL) in a sealed tube was heated in a 100°C oil bath for 18 h. The reaction was cooled to room temperature, concentrated *in vacuo* and purified by silica gel chromatography (Isco 120 g, NH₄OH/MeOH/CH₂Cl₂) to give 81 mg (46% yield) of N4-Methyl-7-(2,4,6-trimethyl-phenyl)-pyrido[2,3-d]pyrimidine-2,4-diamine (**IV**, R1 = CH₃, A = 2,4,6-trimethyl) as a light yellow solid; LR-MS for C₁₇H₁₉N₅ (M+H)⁺ at m/z = 294. ¹H NMR (DMSO-d₆, 300 MHz) δ 8.3 (d, 1 H), 8.09 (broad s, 1 H), 6.87-6.95 (m, 3H), 6.38 (broad s, 2H), 2.97 (broad s, 3H), 2.26 (s, 3H), 1.97 (s, 6H).

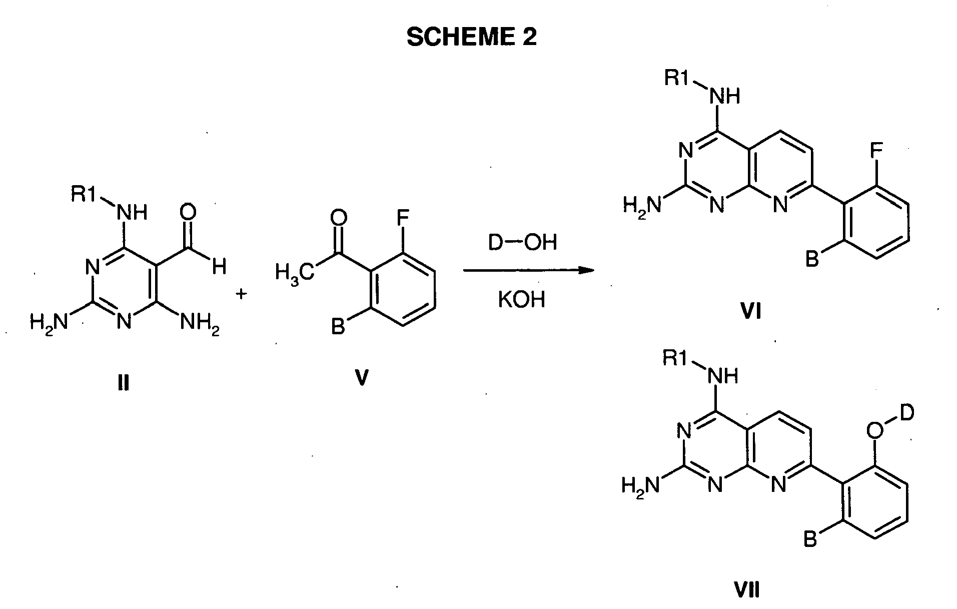

SCHEME 2 shows the special cases of Friedlander condensation reaction when highly electron-deficient acetophenones V containing 2'-fluoro group (B could be, but not limited to, F, Cl or CF₃) are used as substrates. In these special cases, analog **VII** in which the 2'-F was displaced by the alcoholic solvent could be isolated while the expected product **VI** might or might not be isolated. Examples of alcohol used in the fluoride displacement include, but not limited to, methanol, ethanol, 2-propanol, 1-propanol, cyclopentanol, ethylene glycol and 1,3-propanediol. Aromatic nucleophilic substitution reactions with fluoride ion acting as the leaving group have previously been reviewed by Vlasov, V.M. J. Fluorine Chem. 1993, 61, 193.

The following procedures used in the synthesis of 7-(2-Fluoro-6-ethoxy-phenyl)-N4-methyl-pyrido[2,3-d]pyrimidine-2,4-diamine (**VII**, R1 = CH₃, B = F, D = CH₂CH₃) exemplify the typical conditions used in the Friedlander condensation described in SCHEME **2**:
A mixture of 2,4-diamino-6-methylaminopyrimidine-5-carbaldehyde **II** (100 mg, 0.60 mmole), 2',6'-difluoroacetophenone (**V**, 200 mg, 1.23 mmole), potassium hydroxide pellet (100 mg, 1.79 mmole) and ethanol (4 mL) in a sealed tube was heated in a 100°C oil bath for 18 h. The reaction was cooled to room temperature, concentrated *in vacuo* and purified by silica gel chromatography (Isco 120 g, NH₄OH/MeOH/CH₂Cl₂) to give 81 mg (46% yield) of 7-(2-Fluoro-6-ethoxy-phenyl)-N4-methyl-pyrido[2,3-d]pyrimidine-2,4-diamine (**VI**I, R1 = CH₃, B = F, D = CH₂CH₃) as a light brown solid; LRMS for C₁₆H₁₆FN₅O (M+H)⁺ at m/z = 314. ¹H NMR (DMSO-d₆, 300 MHz) δ 8.37 (d, 1 H), 8.20 (broad s, 1 H), 7.40 (q, 1 H), 7.07 (d, 1 H), 6.97 (d, 1 H), 6.90 (t, 1 H), 6.47 (broad s, 2H), 4.05 (q, 2H), 2.97 (d, 3H), 1.18 (t, 3H).

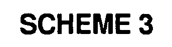

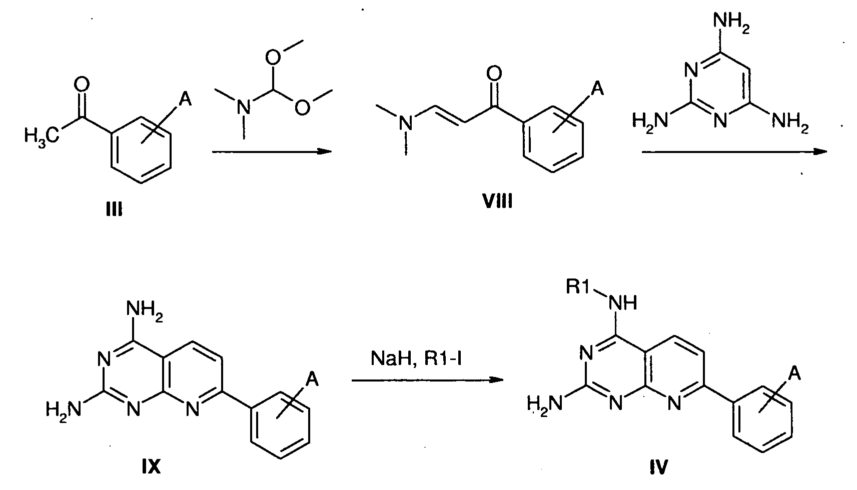

**SCHEME 3** describes an alternative general synthesis of pyrido[2,3-d]pyrimidine-2,4-diamine analogs **IV** bearing R1 group at N-4 and substituted (A group) phenyl at C-7. Condensation of substituted acetophenone **III** with dimethylformamide dimethyl acetal was carried out in a similar fashion as described in Tseng, S-S. et al., J. Heterocyclic Chem. 1987, 24, 837 and Moyroud, J. et al., Heterocycles 1996, 43, 221 to give dimethylamino-propenone **VIII.** Condensation of dimethylamino-propenone **VIII** with 2,4,6-triaminopyrimidine was carried out with slight modifications as described in Troschutz, R. et al., Arch. Pharm. 1994, 327, 221 to give pyrido[2,3-d]pyrimidine-2,4-diamine IX. Treatment of pyrido[2,3-d]pyrimidine-2,4-diamine **IX** with sodium hydride and alkyl iodide in dimethylformamide gave the desired product IV.

The following procedures used in the synthesis of N4-Methyl-7-o-tolyl-pyrido[2,3-d]pyrimidine-2,4-diamine (IV, R1 = CH₃, A = 2-CH₃) exemplify the typical conditions described in SCHEME 3.

Compound **VIII:** A mixture of 2'-methylacetophenone (**III,** A = 2-CH₃, 5 g, 37.3 mmol) and *N,N*-dimethylformamide dimethyl acetal (10 mL, 75.3 mmol) was heated at reflux for 48 h. The reaction mixture was cooled to room temperature and concentrated *in vacuo* to give a dark brown oil. Silica gel chromatography (Isco 120 g, ethyl acetate/hexanes) gave 4.66 g (66% yield) of 1-(o-tolyl)-3-dimethylamino-propenone (**VIII,** A = 2-CH₃) as a light brown oil. LRMS for C₁₂H₁₅NO (M+H)⁺ at m/z = 190

Compound **IX**: A mixture of 1-(o-tolyl)-3-dimethylamino-propenone (2.7 g, 14.3 mmol) and 2,4,6-triaminopyrimidine (**VIII,** A = CH₃, 1.61 g, 12.9 mmol) in glacial acetic acid (25 mL) was heated at reflux for 19 h. Concentration gave a crude which was taken up in hot methanol and absorbed onto silica gel. Silica gel chromatography (Isco 120 g, methylene chloride/methanol/ammonium hydroxide) gave a slightly impure material which was recrystallized from hot aqueous ethanol to give 7-o-Tolyl-pyrido[2,3-d]pyrimidine-2,4-diamine (**IX,** A = CH₃, 368 mg, 11 %) as a light brown solid; LRMS for C₁₄H₁₃N₅ (M+H)⁺ at m/z = 252.

Compound **IV**: To 7-o-Tolyl-pyrido[2,3-d]pyrimidine-2,4-diamine (**IX**, A = CH₃, 400 mg, 1.59 mmole) in *N,N*-dimethylformamide (5 mL) in an ice bath was carefully added sodium hydride (60% in mineral oil, 58 mg, 1.45 mmole). To the chilled mixture was added iodomethane (79 µL, 1.27 mmole) and the mixture was stirred at room temperature for 6 h. Concentration gave a crude which was taken up in hot methanol and absorbed onto silica gel. Silica gel chromatography (Isco 120 g, methylene chloride/methanol/ammonium hydroxide) afforded 20 mg (5% yield) of N4-Methyl-7-o-tolyl-pyrido[2,3-d]pyrimidine-2,4-diamine (**IV**, R1 = CH₃, A = 2-CH₃) as a light brown solid; EI-HRMS m/e calcd for C₁₅H₁₅N₅ (M)⁺ 265.1327, found 265.1322. ¹H NMR (DMSO-d₆, 300 MHz) δ 8.37 (d, 1 H), 8.11 (broad s, 1 H), 7.42 (d, 1 H), 7.3 (m, 3H), 7.16 (d, 1 H), 6.42 (broad s, 2H), 2.97 (d, 3H), 2.37 (s, 3H).

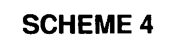

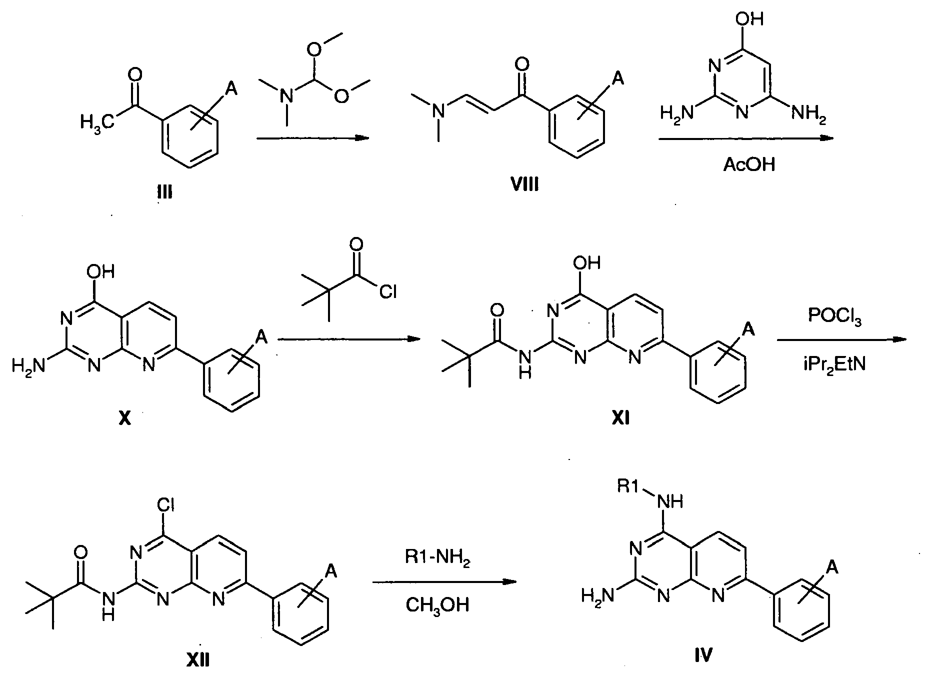

**SCHEME 4** describes an alternative general synthesis of pyrido[2,3-d]pyrimidine-2,4-diamine analogs **IV** bearing R1 group at N-4 and substituted (A group) phenyl at C-7. Condensation of substituted acetophenone **III** with dimethylformamide dimethyl acetal was carried out in a similar fashion as described in Tseng, S-S. et al., J. Heterocyclic Chem. 1987, 24, 837 and Moyroud, J. et al., Heterocycles 1996, 43, 221 to give dimethylamino-propenone **VIII.** Condensation of dimethylamino-propenone **VIII** with 2,4-diamino-6-hydroxypyrimidine was carried out with slight modifications as described in Troschutz, R. et al., Arch. Pharm. 1994, 327, 221 to give 2-amino-pyrido[2,3-d]pyrimidin-4-ol **X.** 2-amino-pyrido[2,3-d]pyrimidin-4-ol **X** was previously reported to be formed from the condensation of 4-diamino-6-hydroxypyrimidine with 3-ketoaldehydes by Robins, R.K. et al., J. Am. Chem. Soc. 1958, 80, 3449. Protection of 2-amino-pyrido[2,3-d]pyrimidin-4-ol **X** as the N-2 pivaloyl pyrido[2,3-d]pyrimidin-4-ol **XI** was carried out in a similar fashion as described by Taylor, E.C. et al. Heterocycles 1993, 36, 1883 and Taylor, E.C. et al. Syn. Commun. 1988, 18, 1187. Conversion of N-2-pivaloyl pyrido[2,3-d]pyrimidin-4-ol **XI** to its 4-chloro analog **XII** was achieved using a similar procedure as described by Ife, R.J. et al. J. Med. Chem. 1995, 38, 2763.

Treatment of 2-N-pivaloyl-4-chloro-pyrido[2,3-d]pyrimidine **XII** with alkylamine gave the desired pyrido[2,3-d]pyrimidine-2,4-diamine analog **IV.**

The following procedures used in the synthesis of 7-(2-fluoro-6-trifluoromethylphenyl)-N4-methyl-pyrido[2,3-d]pyrimidine-2,4-diamine **(IV,** R1 = CH₃, A = 2-F, 6-CF₃) exemplify the typical conditions described in **SCHEME 4.**

Compound **VIII:** A mixture of 2'-fluoro-6'-(trifluoromethyl)acetophenone (25.3 g, 0.123 mol) and *N*,*N*-dimethylformamide dimethyl acetal (200 mL, 1.51 mol) was heated at reflux for 16 h. The reaction mixture was cooled to room temperature and concentrated *in vacuo* to give 31.2 g (97% yield) of 1-(2-fluoro-6-(trifluoromethyl)phenyl)-3-dimethylamino-propenone **VIII** as a brown oil. This compound was used in the next step as a crude oil without further purification.

Compound X: A mixture of crude 1-(2-fluoro-6-(trifluoromethyl)phenyl)-3-dimethylamino-propenone **VIII** (31.2 g, 119 mmol) and 2,4-diamino-6-hydroxypyrimidine (13.6 g, 108 mmol) in glacial acetic acid (350 mL) was heated at reflux for 2 days. The slurry was cooled to 25°C, filtered, washed with glacial acetic acid and dried *in vacuo* to afford 2-amino-7-(2-fluoro-6-(trifluoromethyl)phenyl)-pyrido[2,3-d]pyrimidin-4-ol X (20.1 g, 57%) as a yellow solid; LR-MS for C₁₄H₈F₄N₄O (M+H)⁺ at m/z = 325.

Compound **XI:** A mixture of 2-amino-7-(2-fluoro-6-(trifluoromethyl)phenyl)-pyrido[2,3-d]pyrimidin-4-ol **X** (20.0 g, 61.7 mmol) and trimethylacetic anhydride (33.0 mL, 161 mmol) in pyridine (200 mL) was heated to reflux for 2 days. After cooling to room temperature, the reaction mixture was concentrated *in vacuo* and recrystallization of the crude solid from hot ethyl acetate gave N-[7-(2-fluoro-6-(trifluoromethyl)phenyl)-4-hydroxy-pyrido[2,3-d]pyrimidin-2-yl]-2,2-dimethyl-propionamide **XI** (13.0 g, 52% yield) as a yellow solid; LR-MS for C₁₉H₁₆F₄N₄O₂ (M+H)⁺ at m/z = 409.

Compound **IV:** To a mixture of phosphorous oxychloride (70 mL, 753 mmol) and N-[7-(2-fluoro-6-(trifluoromethyl)phenyl)-4-hydroxy-pyrido[2,3-d]pyri midi n-2-yl]-2,2-dimethyl-propionamide **XI** (7.10 g, 17.4 mmol) cooled in an ice bath was slowly added *N,N*-diisopropylethylamine (13.0 mL, 74.6 mmol). The reaction was then heated to 35°C for 18 h. After cooling to room temperature, the excess phosphorous oxychloride was distilled off *in vacuo* to afford N-[4-chloro-7-(2-fluoro-6-(trifluoromethyl)phenyl)-pyrido[2,3-d]pyri midin-2-yl]-2,2-dimethyl-propionamide **XII** as a brown oil. To the above crude **XII** was added chilled 2-propanol (300 mL) and the solution was saturated with methylamine gas while maintaining the internal temperature <20°C. The resulting mixture was stirred at room temperature for 18 h. The mixture was concentrated *in vacuo,* taken up in hot methanol and absorbed onto silica gel. Silica gel chromatography (Merck Silica gel 60, 230-400 mesh, methylene chloride/methanol/ammonium hydroxide) afforded 2.32 g (40% yield) of 7-(2-fluoro-6-trifluoromethyl-phenyl)-N4-methyl-pyrido[2,3-d]pyrimidine-2,4-diamine **IV** as a light yellow solid. LR-MS for C₁₅H₁₁F₄N₅ (M+H)⁺ at m/z = 338. ¹H NMR (DMSO-d₆, 300 MHz) δ 8.40 (d, 1H), 8.30 (broad s, 1 H), 7.7 (m, 3H), 7.11 (d, 1 H), 6.60 (broad s, 2H), 2.97 (d, 3H).

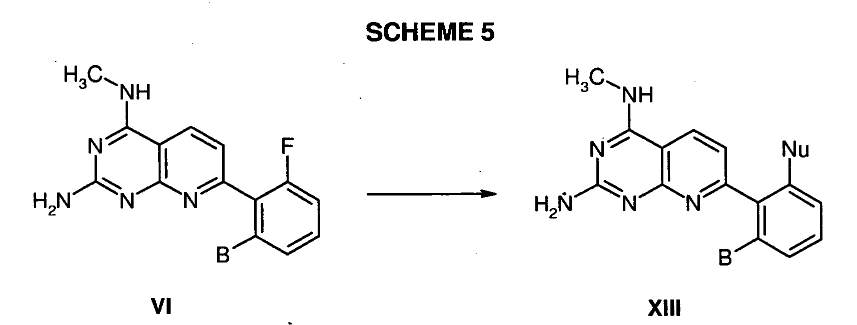

**SCHEME 5** describes a special scenario in which pyrido[2,3-d]pyrimidine-2,4-diamine analogs **VI** containing highly electron-deficient C-7 phenyl with o-,o'-disubstitution and o-fluoro group (B could be, but not limited to, F, Cl or CF₃) was treated with a number of nucleophiles under harsh conditions to give the corresponding pyrido[2,3-d]pyrimidine-2,4-diamine analogs **XIII** through the displacement of the o-fluoro group. Aromatic nucleophilic substitution reactions with fluoride ion acting as the leaving group have previously been reviewed by Vlasov, V.M. J. Fluorine Chem. 1993, 61, 193. Examples of nucleophiles used in the fluoride displacement reaction include, but not limited to, amines, alcohols, phenols, methanethiolate, benzenethiol and 1H-imidazole. Examples of amines used include, but not limited to, morpholine, dimethylamine, methylamine, thiomorpholine, pyrrolidine, 2-methylpyrrolidine, 2,5-dimethylpyrrolidine, 3-hydroxypyrrolidine, L-prolinol, (2-methoxymethyl)pyrrolidine, piperidine, piperidine-2-carboxylic acid ethyl ester, 4-hydroxypiperidine, 3-hydroxypiperidine, 3-methylamino-piperidine, 4-hydroxy-4-phenylpiperidine, 4-benzylpiperidine, N-methylpiperazine, 1-cyclohexylpiperazine, 1-ethylpiperazine, 1-benzylpiperazine, 1-phenylpiperazine, 1-(2-furoyl)piperazine, 1-cyclopentylpiperazine and 1-isopropylpiperazine. Examples of alcohols used include, but not limited to, methanol, ethanol, 2-propanol, 1-propanol, cyclopentanol, cyclohexanol, ethylene glycol, 1,3-propanediol, 2-dimethylaminoethanol, 2-diethylaminoethanol, 2-methoxyethanol, 1-(2-hydroxyethyl)pyrrolidine and 1-(2-hydroxyethyl)morpholine. Examples of phenols used include, but not limited to, phenol, p-cresol, 4-chlorophenol, 3-chlorophenol, 4-fluorophenol, 3-fluorophenol, 2-fluorophenol and 4-phenylphenol.

The following procedures used in the synthesis of N4-Methyl-7-(2-piperidin-1-yl-6-trifluoromethyl-phenyl)-pyrido[2,3-d]pyrimidine-2,4-diamine (**XIII,** B = CF₃, Nu = piperidine) exemplify the typical conditions described in **SCHEME 5**.

A mixture of 7-(2-fluoro-6-trifluoromethyl-phenyl)-N4-methyl-pyrido[2,3-d]pyrimidine-2,4-diamine (**VI,** B = CF₃, 30 mg, 0.089 mmole), piperidine (39 mg, 0.46 mmole) and potassium carbonate (60 mg, 0.43 mmole) in *N*,*N-*dimethylformamide (4 mL) or 1-methyl-2-pyrrolidinone (4 mL) in a sealed tube was heated in a 190°C oil bath overnight. After cooling to room temperature, the reaction was concentrated *in vacuo* and purified by silica gel chromatography (Merck Silica gel 60, 230-400 mesh, methylene chloride/methanol/ammonium hydroxide) to give 23 mg (41% yield) of N4-Methyl-7-(2-piperidin-1-yl-6-trifluoromethyl-phenyl)-pyrido[2,3-d]pyrimidine-2,4-diamine **(XIII,** B = CF₃, Nu = piperidine) as a light brown solid; LRMS for C₂₀H₂₁F₃N₆ (M+H)⁺ at m/z = 403. ¹H NMR (DMSO-d₆, 300 MHz) δ 8.28 (d, 1 H), 8.09 (broad s, 1 H), 7.56 (t, 1 H), 7.44 (m, 2H), 6.97 (d, 1 H), 6.40 (broad s, 2H), 2.97 (d, 3H), 2.6-2.9 (m, 4H), 1.0-1.4 (m, 6H).

### EXAMPLES

### Example 1

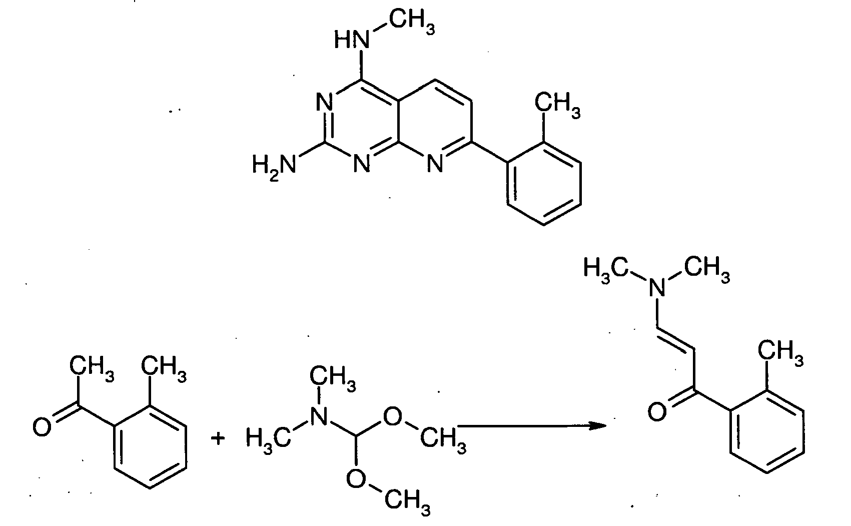

Step 1: A mixture of 2'-methylacetophenone (5 g, 37.3 mmol) and *N,N-*dimethylformamide dimethyl acetal (10 mL, 75.3 mmol) was heated to reflux for 48 h. The reaction mixture was cooled to room temperature and concentrated *in vacuo* to give a dark brown oil. Silica gel chromatography (Isco Silica gel 120 g, ethyl acetate/hexanes) gave 4.66 g (66% yield) of 1-(o-tolyl)-3-dimethylamino-propenone as a light brown oil. LRMS for C₁₂H₁₅NO (M+H)⁺ at m/z = 190.

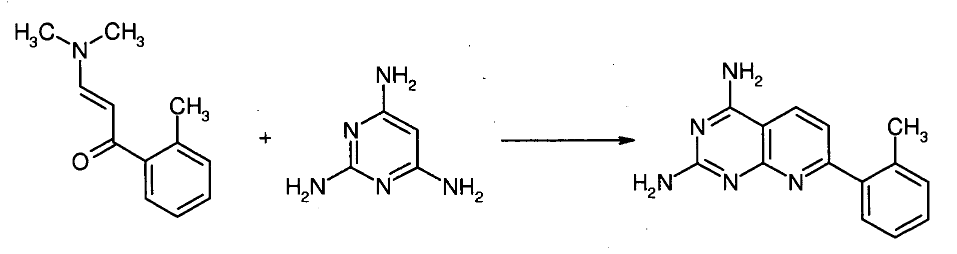

Step 2: A mixture of 1-(o-toyl)-3-dimethylamino-propenone (2.7 g, 14.3 mmol) and 2,4,6-triaminopyrimidine (1.61 g, 12.9 mmol) in glacial acetic acid (25 mL) was heated to reflux for 19 h. Concentration gave a crude which was taken up in hot methanol and absorbed onto silica gel. Silica gel chromatography (Isco silica gel 120 g, methylene chloride/methanol/ammonium hydroxide) gave a slightly impure material which was recrystallized from hot aqueous ethanol to give 7-o-Tolyl-pyrido[2,3-d]pyrimidine-2,4-diamine (368 mg, 11%) as a light brown solid; LRMS for C₁₄H₁₃N₅ (M+H)⁺ at m/z = 252.

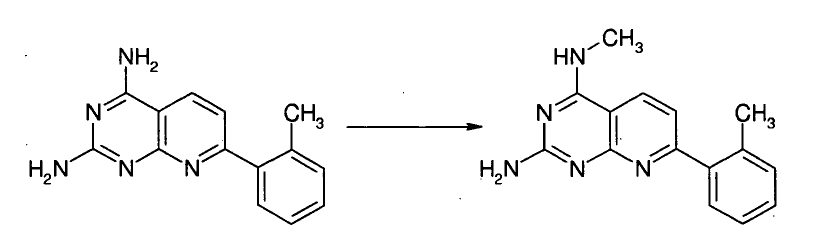

**Step 3:** To 7-o-Tolyl-pyrido[2,3-d]pyrimidine-2,4-diamine (400 mg, 1.59 mmole) in *N,N*-dimethylformamide (5 ml) in an ice bath was carefully added sodium hydride (60% in mineral oil, 58 mg, 1.45 mmole). To the chilled mixture was added iodomethane (79 µL, 1.27 mmole) and the mixture was stirred at room temperature for 6 h. Concentration gave a crude which was taken up in hot methanol and absorbed onto silica gel. Silica gel chromatography (Isco silica gel 120 g, methylene chloride/methanol/ammonium hydroxide) afforded 20 mg (5% yield) of N4-Methyl-7-o-tolyl-pyrido[2,3-d]pyrimidine-2,4-diamine as a light brown solid; EI-HRMS m/e calcd for C₁₅H₁₅N₅ (M)⁺ 265.1327, found 265.1322.

In an analogous manner, there were obtained:

### Example 2

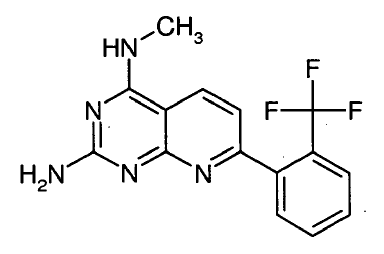

From 2'-trifluoromethylacetophenone: N4-Methyl-7-(2-trifluoromethyl-phenyl)-pyrido[2,3-d]pyrimidine-2,4-diamine as a light brown solid; LRMS for C₁₅H₁₂F₃N₅ (M+H)⁺ at m/z = 320.

### Example 3

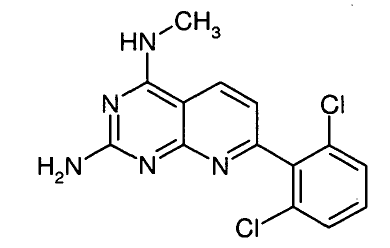

From 2',6'-dichloroacetophenone: 7-(2,6-Dichloro-phenyl)-N4-methyl-pyrido[2,3-d]pyrimidine-2,4-diamine as a brown solid; LRMS for C₁₄H₁₁Cl₂N₅ (M+H)⁺ at m/z = 320.

### Example 4

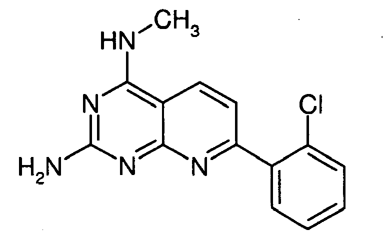

From 2'-chloroacetophenone: 7-(2-Chloro-phenyl)-N4-methyl-pyrido[2,3-d]pyrimidine-2,4-diamine as a light brown solid; LRMS for C₁₄H₁₂ClN₅ (M+H)⁺ at m/z = 286.

### Example 5

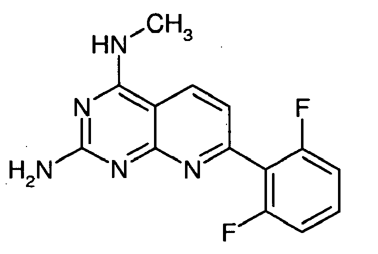

From 2',6'-difluoroacetophenone: 7-(2,6-Difluoro-phenyl)-N4-methyl-pyrido[2,3-d]pyrimidine-2,4-diamine as an off-white solid; LR-MS for C₁₄H₁₁F₂N₅ (M+H)⁺ at m/z = 288.

### Example 6

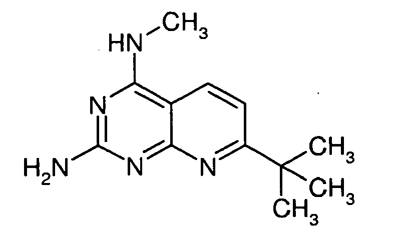

From pinacolone: 7-tert-Butyl-N4-methyl-pyrido[2,3-d]pyrimidine-2,4-diamine as a light brown solid; LRMS for C₁₂H₁₇N₅ (M+H)⁺ at m/z = 232.

### Example 7

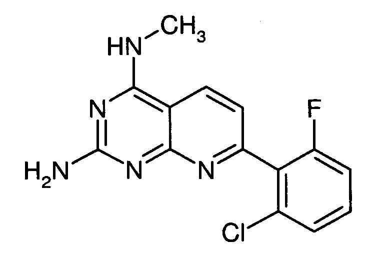

From 2'-chloro-6'-fluoroacetophenone: 2-chloro-6-fluorophenyl-N4-methyl-pyrido[2,3-d]pyrimidine-2,4-diamine as a light yellow solid; LR-MS for C₁₄H₁₁ClFN₅ (M+H)⁺ at m/z = 304.

### Example 8

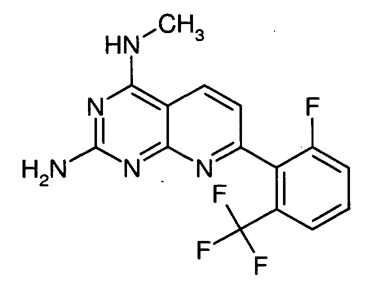

From 2'-fluoro-6'-trifluoromethylacetophenone: 7-(2-Fluoro-6-trifluoromethylphenyl)-N4-methyl-pyrido[2,3-d]pyrimidine-2,4-diamine as a light yellow solid; LR-MS for C₁₅H₁₁F₄N₅ (M+H)⁺ at m/z = 338.

### Example 9

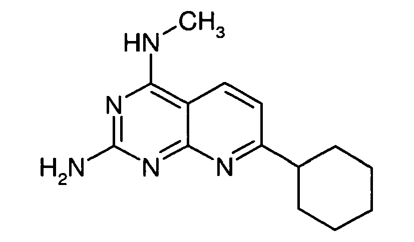

From 1-cyclohexyl-ethanone: 7-Cyclohexyl-N4-methyl-pyrido[2,3-d]pyrimidine-2,4-diamine as a light yellow solid; LR-MS for C₁₄H₁₉N₅ (M+H)⁺ at m/z = 258.

### Example 10

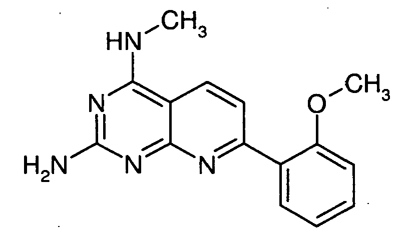

From 2'-Methoxyacetophenone: 7-(2-Methoxy-phenyl)-N4-methyl-pyrido[2,3-d]pyrimidine-2,4-diamine as a light brown solid; LRMS for C₁₅H₁₅N₅O (M+H)⁺ at m/z = 282.

### Example 11

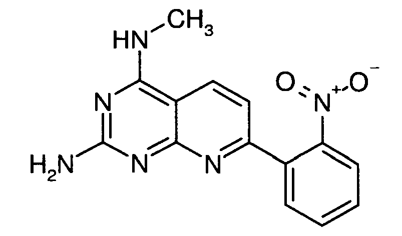

From 2'-Nitroacetophenone: N4-Methyl-7-(2-nitro-phenyl)-pyrido[2,3-d]pyrimidine-2,4-diamine as a light brown solid; LRMS for C₁₄H₁₂N₆O₂ (M+H)⁺ at m/z = 297.

### Example 12

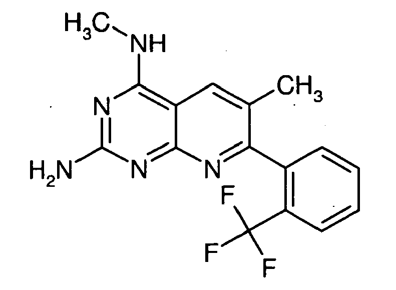

From 2'-(trifluoromethyl)propiophenone: 6,N4-Dimethyl-7-(2-trifluoromethylphenyl)-pyrido[2,3-d]pyrimidine-2,4-diamine as a light brown solid; LRMS for C₁₆H₁₄F₃N₅ (M+H)⁺ at m/z = 334.

### Example 13

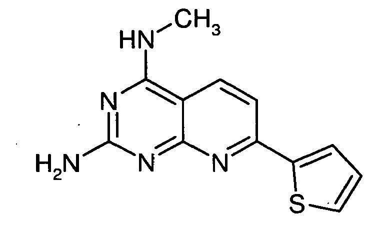

From 2-acetylthiophene: N4-Methyl-7-thiophen-2-yl-pyrido[2,3-d]pyrimidine-2,4-diamine as a light brown solid; LRMS for C₁₂H₁₁N₅S (M+H)⁺ at m/z = 258.

### Example 14

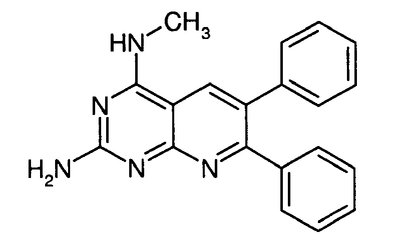

From deoxybenzoin: N4-Methyl-6,7-diphenyl-pyrido[2,3-d]pyrimidine-2,4-diamine as a light brown solid; LRMS for C₂₀H₁₇N₅ (M+H)⁺ at m/z = 328.

### Example 15

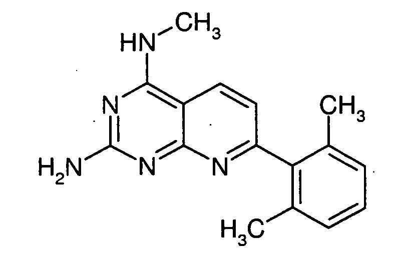

From 2',6'-dimethylacetophenone in step 1 and iodomethane in step 3: 7-(2,6-Dimethyl-phenyl)-N4-methyl-pyrido[2,3-d]pyrimidine-2,4-diamine as a yellow solid; EI-HRMS m/e calcd for C₁₆H₁₇N₅ (M⁺) 279.1484, found 279.1474.

### Example 16

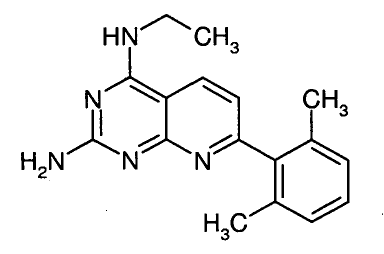

From 2',6'-dimethylacetophenone in step 1 and iodoethane in step 3: 7-(2,6-dimethyl-phenyl)-N4-ethyl-pyrido[2,3-d]pyrimidine-2,4-diamine as a yellow solid; EI-HRMS m/e calcd for C₁₇H₁₉N₅ (M-H)⁺ 292.1562, found 292.1563.

### Example 17

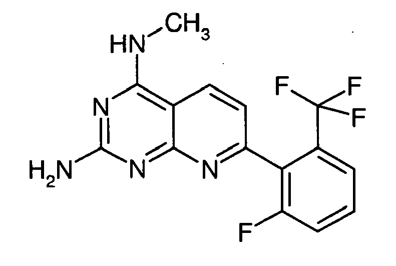

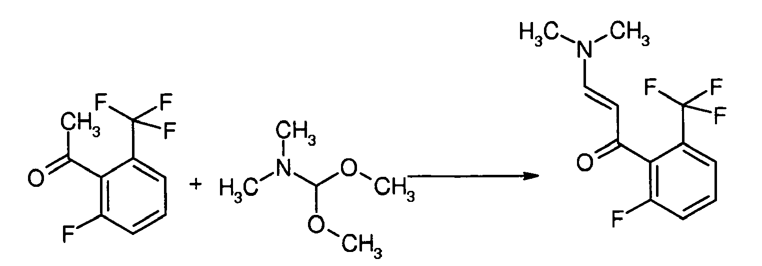

Step 1: A mixture of 2'-fluoro-6'-(trifluoromethyl)acetophenone (25.3 g, 0.123 mol) and *N,N*-dimethylformamide dimethyl acetal (200 mL, 1.51 mol) was heated at reflux for 16 h. The reaction mixture was cooled to room temperature and concentrated *in vacuo* to give 31.2 g (97% yield) of 1-(2-fluoro-6-(trifluoromethyl)phenyl)-3-dimethylamino-propenone as a brown oil. This compound was used in the next step as a crude without further purification.

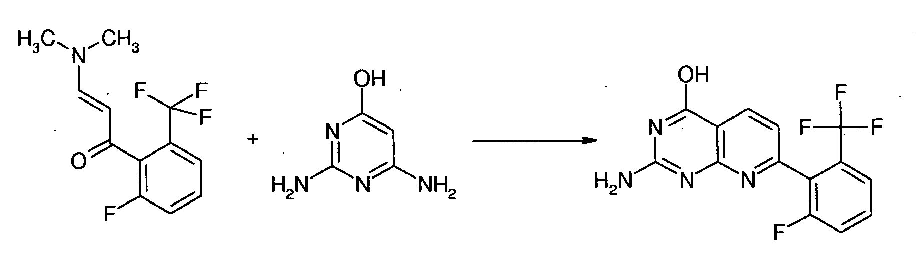

Step 2: A mixture of crude 1-(2-fluoro-6-(trifluoromethyl)phenyl)-3-dimethylamino-propenone (31.2 g, 119 mmol) and 2,4-diamino-6-hydroxypyrimidine (13.6 g, 108 mmol) in glacial acetic acid (350 mL) was heated at reflux for 2 days. The slurry was cooled to 25°C, filtered, washed with glacial acetic acid and dried *in vacuo* to afford 2-amino-7-(2-fluoro-6-(trifluoromethyl)phenyl)-pyrido[2,3-d]pyrimidin-4-ol (20.1 g, 57%) as a yellow solid; LR-MS for C₁₄H₈F₄N₄O (M+H)⁺ at m/z = 325.

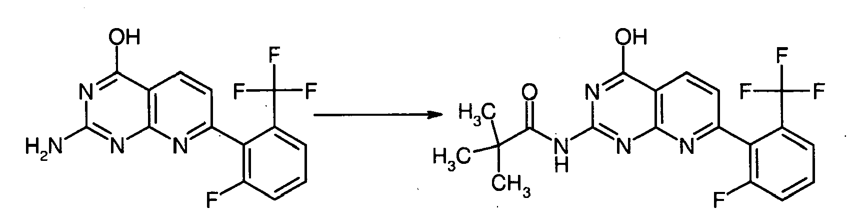

Step 3: A mixture of 2-amino-7-(2-fluoro-6-(trifluoromethyl)phenyl)-pyrido[2,3-d]pyrimidin-4-ol (20.0 g, 61.7 mmol) and trimethylacetic anhydride (33.0 mL, 161 mmol) in pyridine (200 mL) was heated to reflux for 2 days. After cooling to room temperature, the reaction mixture was concentrated *in vacuo* and recrystallization of the crude from hot ethyl acetate gave N-[7-(2-fluoro-6-(trifluoromethyl)phenyl)-4-hydroxy-pyrido[2,3-d]pyrimidin-2-yl]-2,2-dimethyl-propionamide (13.0 g, 52% yield) as a yellow solid; LR-MS for C₁₉H₁₆F₄N₄O₂ (M+H)⁺ at m/z = 409.

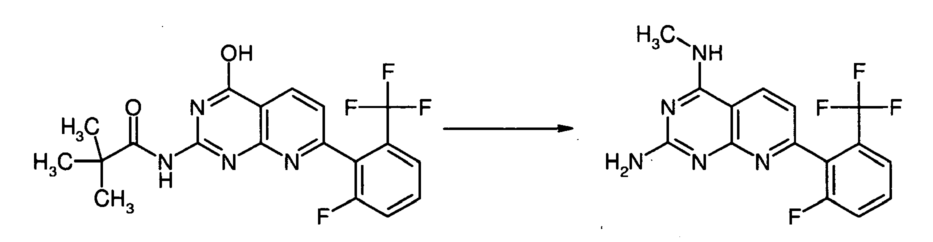

Step 4: To a mixture of phosphorous oxychloride (70 mL, 753 mmol) and N-[7-(2-fluoro-6-(trifluoromethyl)phenyl)-4-hydroxy-pyrido[2,3-d]pyrimidin-2-yl]-2,2-dimethyl-propionamide (7.10 g, 17.4 mmol) cooled in an ice bath was slowly added *N*,*N*-diisopropylethylamine (13.0 mL, 74.6 mmol). The reaction was then heated to 35°C for 18 h. After cooling to room temperature, the excess phosphorous oxychloride was distilled off *in vacuo* to afford N-[4-chloro-7-(2-fluoro-6-(trifluoromethyl)phenyl)-pyrido[2,3-d]pyrimidin-2-yl]-2,2-dimethyl-propionamide as a brown oil. To the above crude brown oil was added chilled 2-propanol (300 mL) and the solution was saturated with methylamine gas while maintaining the internal temperature <20°C. The resulting mixture was stirred at room temperature for 18 h. The mixture was concentrated *in vacuo,* taken up in hot methanol and absorbed onto silica gel. Silica gel chromatography (Merck Silica gel 60, 230-400 mesh, methylene chloride/methanol/ammonium hydroxide) afforded 2.32 g (40% yield) of 7-(2-fluoro-6-trifluoromethyl-phenyl)-N4-methyl-pyrido[2,3-d]pyrimidine-2,4-diamine as a light yellow solid. LR-MS for C₁₅H₁₁F₄N₅ (M+H)⁺ at m/z = 338.

### Example 18

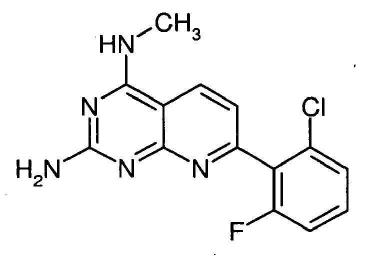

Using the same four-step sequence as shown above but starting from 2'-chloro-6'-fluoroacetophenone gave 7-(2-Chloro-6-fluoro-phenyl)-N4-methyl-pyrido[2,3-d]pyrimidine-2,4-diamine as a light yellow solid; LR-MS for C₁₄H₁₁ClFN₅ (M+H)⁺ at m/z = 304.

### Preparation of 2,4-diamino-6-methylaminopyrimidine-5-carbaldehyde

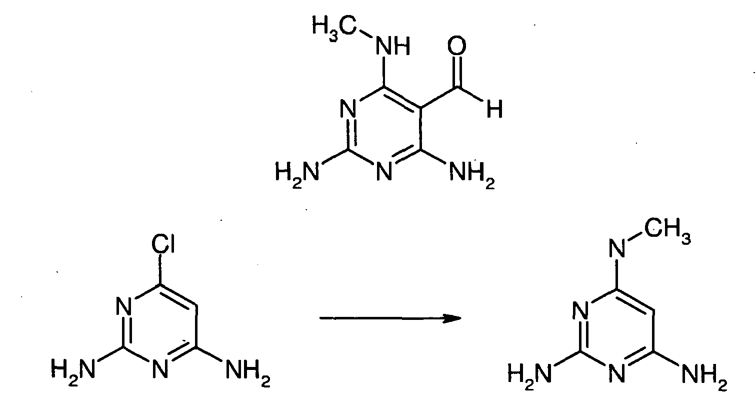

Step 1: To 6-chloro-2,4-diaminopyrimidine (5.0 g, 0.0347 mole) was added 25 ml of 25% aqueous MeNH₂ solution (0.182 mole, prepared from 40% aqueous MeNH₂ solution) in a sealed tube. The reaction was heated at 150°C for 4.5 hours. TLC (1/9/90 v/v/v conc.NH₄OH/MeOH/CH₂Cl₂) analysis indicated complete disappearance of starting material. The reaction was then cooled to room temperature and concentrated to give a crude oil. The crude was absorbed onto silica gel using methanol as solvent. The crude material on silica gel was purified using silica gel chromatography (silica gel, conc.NH₄OH/MeOH/CH₂Cl₂) to give 3.98 g of an impure material. Recrystallization of the impure material from 45 ml of hot ethanol gave 1.57 g (11.3 mmole, 33% yield) of 2,4-diamino-6-methylaminopyrimidine as an off-white solid. ¹H NMR (DMSO-d₆, 300 MHz) δ 5.9 (broad s, 1 H), 5.5 (broad s, 2H), 5.3 (broad s, 2H), 4.76 (s, 1 H), 2.60 (broad s, 3H). 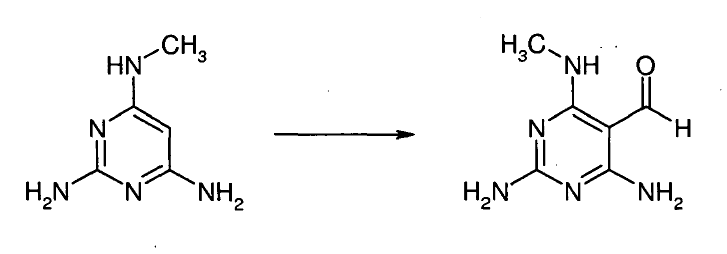 Step 2: To a 250 ml three-necked round bottom flask equipped with a magnetic stirrer, argon inlet and thermometer was added *N*,*N*-dimethylformamide (20 ml, anhydrous). The flask was cooled in a dry ice/ethylene glycol bath and phosphorus oxychloride (1.97 ml, 21.14 mmol) was added slowly at a rate so as to keep the internal temperature below 0°C. 2,4-diamino-6-methylaminopyrimidine I (2.20 g, 15.8 mmole) was then added carefully as a slurry in *N*,*N*-dimethylforamide (20 ml, anhydrous) (Exothermic!). The reaction was transferred to a 40°C oil bath and stirred for 1.5 hours. The reaction was quenched with ice (~70 g) and sodium hydroxide pellets (4 g) was added to make the solution slightly basic (pH ~ 8). The mixture was then heated in a 90°C oil bath until methylamine gas was no longer evolved from the mixture. Sodium hydroxide pellets were added as needed to keep the pH of mixture ~8. The reaction was then cooled to room temperature and concentrated to give a crude solid. The crude was absorbed onto silica gel using methanol as solvent. Silica gel chromatography (Isco silica gel 120 g, NH₄OH/MeOH/CH₂Cl₂) gave 1.23 g (47% yield) of 2,4-diamino-6-methylaminopyrimidine-5-carbaldehyde **II** as a light brown solid. ¹H NMR (DMSO-d₆, 300 MHz) δ 9.68 (s, 1 H), 9.1 (broad s, 1 H), 6.85 (broad s, 2H), 6.5 (broad s, 2H), 2.80 (broad s, 3H).

### Example 19

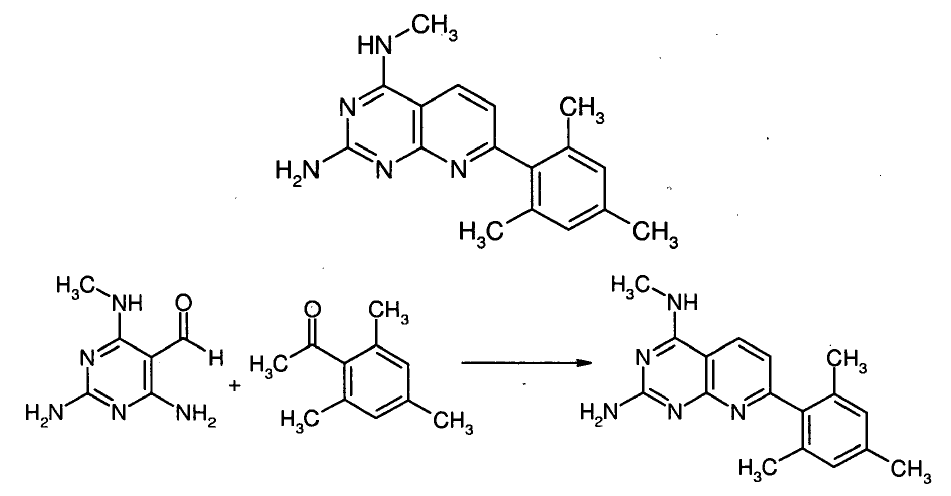

A mixture of 2,4-diamino-6-methylaminopyrimidine-5-carbaldehyde (100 mg, 0.60 mmole), 2',4',6'-trimethylacetophenone (200 mg, 1.23 mmole), potassium hydroxide pellet (100 mg, 1.79 mmole) and ethanol (4 ml) in a sealed tube was heated in a 100°C oil bath for 18 h. The reaction was cooled to room temperature, concentrated *in vacuo* and purified by silica gel chromatography (Isco 120 g, NH₄OH/MeOH/CH₂Cl₂) to give 81 mg (46% yield) of N4-Methyl-7-(2,4,6-trimethylphenyl)-pyrido[2,3-d]pyrimidine-2,4-diamine as a light yellow solid; LR-MS for C₁₇H₁₉N₅ (M+H)⁺ at m/z = 294. ¹H NMR (DMSO-d₆, 300 MHz) δ 8.3 (d, 1 H), 8.09 (broad s, 1 H), 6.87-6.95 (m, 3H), 6.38 (broad s, 2H), 2.97 (broad s, 3H), 2.26 (s, 3H), 1.97 (s, 6H).

In an analogous manner, there were obtained:

### Example 20

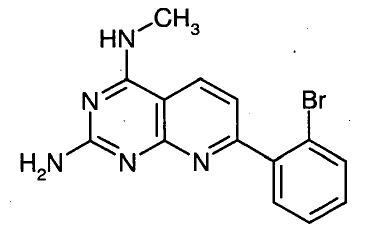

From 2,4-diamino-6-methylaminopyrimidine-5-carbaldehyde and 2'-bromoacetophenone: 7-(2-Bromo-phenyl)-N4-methyl-pyrido[2,3-d]pyrimidine-2,4-diamine as a light yellow solid; LRMS for C₁₄H₁₂BrN₅ (M+H)⁺ at m/z = 330.

### Example 21

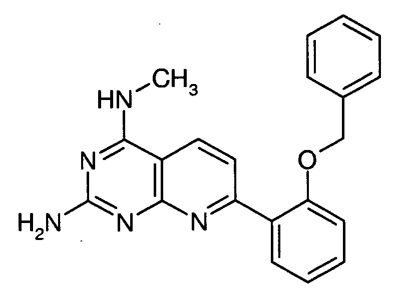

From 2,4-diamino-6-methylaminopyrimidine-5-carbaldehyde and 2'-benzyloxyacetophenone: 7-(2-Benzyloxy-phenyl)-N4-methyl-pyrido[2,3-d]pyrimidine-2,4-diamine as a light brown solid; LRMS for C₂₁H₁₉N₅O (M+H)⁺ at m/z = 358.

### Example 22

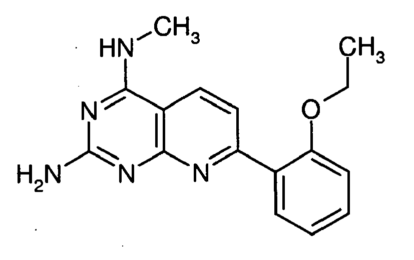

From 2,4-diamino-6-methylaminopyrimidine-5-carbaldehyde and 2'-ethoxyacetophenone: 7-(2-Ethoxy-phenyl)-N4-methyl-pyrido[2,3-d]pyrimidine-2,4-diamine trifluoroacetic acid salt as a light brown solid; LRMS for C₁₆H₁₇N₅O (M+H)⁺ at m/z = 296.

### Example 23

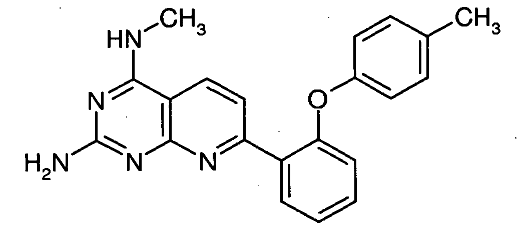

From 2,4-diamino-6-methylaminopyrimidine-5-carbaldehyde and 2-tolyloxyacetophenone: N4-Methyl-7-(2-p-tolyloxy-phenyl)-pyrido[2,3-d]pyrimidine-2,4-diamine trifluoroacetic acid salt as a light brown solid; LRMS for C₂₁H₁₉N₅O (M+H)⁺ at m/z = 358.

### Example 24

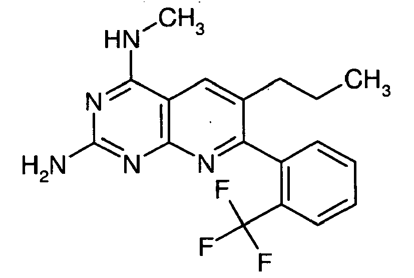

From 2,4-diamino-6-methylaminopyrimidine-5-carbaldehyde and 1-(2-Trifluoromethyl-phenyl)-pentan-1-one: N4-Methyl-6-propyl-7-(2-trifluoromethylphenyl)-pyrido[2,3-d]pyrimidine-2,4-diamine as a light brown solid; LRMS for C₁₈H₁₈F₃N₅ (M+H)⁺ at m/z = 362.

### Example 25

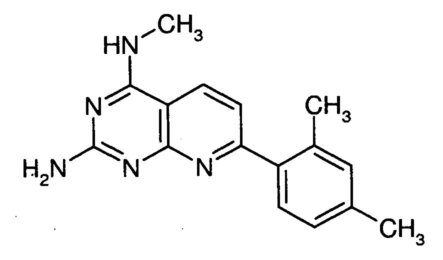

From 2,4-diamino-6-methylaminopyrimidine-5-carbaldehyde and 2',4'-dimethylacetophenone: N4-Methyl-7-(2,4-dimethyl-phenyl)-pyrido[2,3-d]pyrimidine-2,4-diamine as a light brown solid; LRMS for C₁₆H₁₇N₅ (M+H)⁺ at m/z = 280.

### Example 26

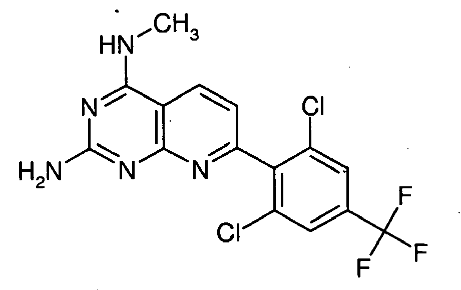

From 2,4-diamino-6-methylaminopyrimidine-5-carbaldehyde and 2',6'-dichloro-4'-(trifluoromethyl)acetophenone: 7-(2,6-Dichloro-4-trifluoromethyl-phenyl)-N4-methyl-pyrido[2,3-d]pyrimidine-2,4-diamine trifluoroacetic acid salt as a light brown solid; LRMS for C₁₅H₁₀Cl₂F₃N₅ (M+H)⁺ at m/z = 388.

### Example 27

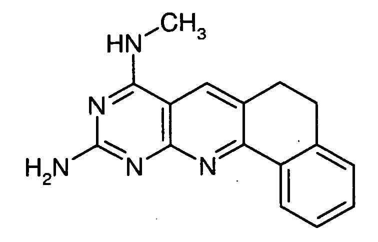

From 2,4-diamino-6-methylaminopyrimidine-5-carbaldehyde and α-tetralone: N8-Methyl-5,6-dihydro-benzo[h]pyrimido[4,5-b]quinoline-8,10-diamine as a light brown solid; LRMS for C₁₆H₁₅N₅ (M+H)⁺ at m/z = 278.

### Example 28

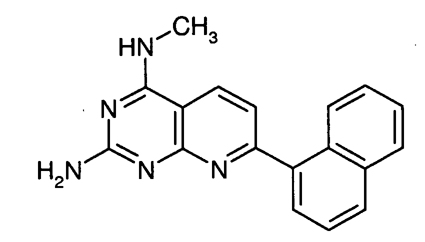

From 2,4-diamino-6-methylaminopyrimidine-5-carbaldehyde and 1'-acetonaphthone: N4-Methyl-7-naphthalen-1-yl-pyrido[2,3-d]pyrimidine-2,4-diamine as a light brown solid; LRMS for C₁₈H₁₅N₅ (M+H)⁺ at m/z = 302.

### Example 29

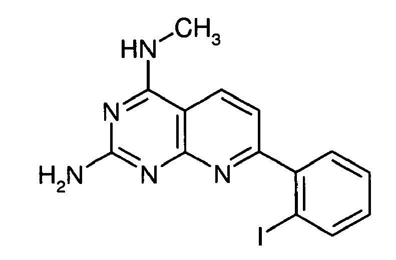

From 2,4-diamino-6-methylaminopyrimidine-5-carbaldehyde and 2'-iodoacetophenone: 7-(2-lodo-phenyl)-N4-methyl-pyrido[2,3-d]pyri midi ne-2,4-diamine trifluoroacetic acid salt as a light brown solid; LRMS for C₁₄H₁₂lN₅ (M+H)⁺ at m/z = 378.

### Example 30

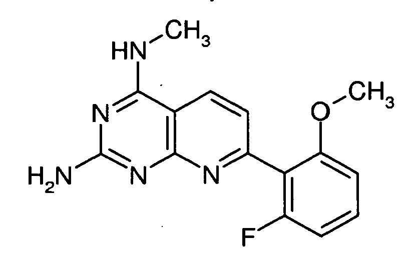

From 2,4-diamino-6-methylaminopyrimidine-5-carbaldehyde and 2',6'-difluoroacetophenone using methanol as solvent: 7-(2-Fluoro-6-methoxy-phenyl)-N4-methyl-pyrido[2,3-d]pyrimidine-2,4-diamine trifluoroacetic acid salt as a light brown solid; LRMS for C₁₅H₁₄FN₅O (M+H)⁺ at m/z = 300.

### Example 31

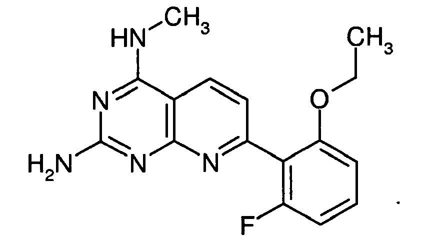

From 2,4-diamino-6-methylaminopyrimidine-5-carbaldehyde and 2',6'-difluoroacetophenone using ethanol as solvent: 7-(2-Ethoxy-6-fluoro-phenyl)-N4-methyl-pyrido[2,3-d]pyrimidine-2,4-diamine trifluoroacetic acid salt as a light brown solid; LRMS for C₁₆H₁₆FN₅O (M+H)⁺ at m/z = 314.

### Example 32

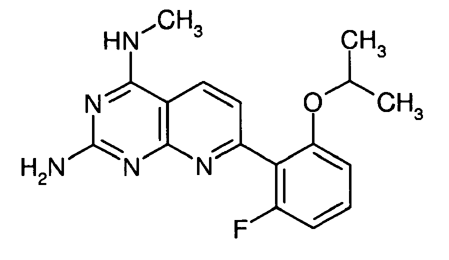

From 2,4-diamino-6-methylaminopyrimidine-5-carbaldehyde and 2',6'-difluoroacetophenone using 2-propanol as solvent: 7-(2-Fluoro-6-isopropoxyphenyl)-N4-methyl-pyrido[2,3-d]pyrimidine-2,4-diamine trifluoroacetic acid salt as a light brown solid; LRMS for C₁₇H₁₈FN₅O (M+H)⁺ at m/z = 328.

### Example 33

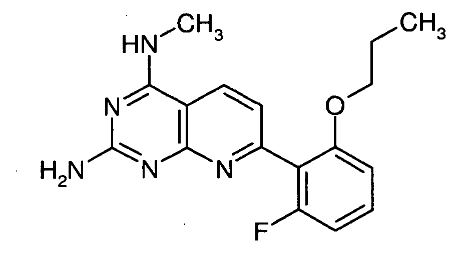

From 2,4-diamino-6-methylaminopyrimidine-5-carbaldehyde and 2',6'-difluoroacetophenone using 1-propanol as solvent: 7-(2-Fluoro-6-propoxy-phenyl)-N4-methyl-pyrido[2,3-d]pyrimidine-2,4-diamine trifluoroacetic acid salt as a light brown solid; LRMS for C₁₇H₁₈FN₅O (M+H)⁺ at m/z = 328.

### Example 34

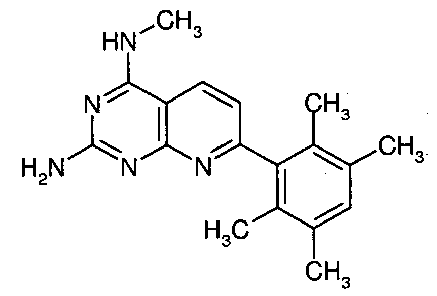

From 2,4-diamino-6-methylaminopyrimidine-5-carbaldehyde and 2',3',5',6'-tetramethylacetophenone: N4-Methyl-7-(2,3,5,6-tetramethyl-phenyl)-pyrido[2,3-d]pyrimidine-2,4-diamine trifluoroacetic acid salt as a light brown solid; LR-MS for C₁₈H₂₁N₅ (M+H)⁺ at m/z = 308.

### Example 35

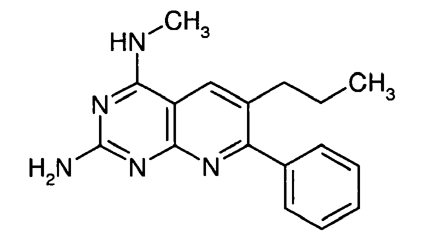

From 2,4-diamino-6-methylaminopyrimidine-5-carbaldehyde and valerophenone: N4-Methyl-7-phenyl-6-propyl-pyrido[2,3-d]pyrimidine-2,4-diamine trifluoroacetic acid salt as a light brown solid; LRMS for C₁₇H₁₉N₅ (M+H)⁺ at m/z = 294.

### Example 36

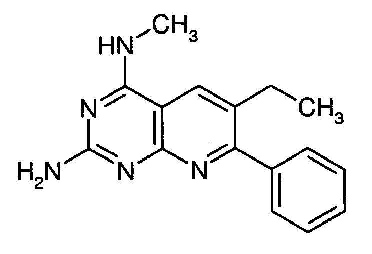

From 2,4-diamino-6-methylaminopyrimidine-5-carbaldehyde and butyrophenone: 6-Ethyl-N4-methyl-7-phenyl-pyrido[2,3-d]pyrimidine-2,4-diamine trifluoroacetic acid salt as a light brown solid; LRMS for C₁₆H₁₇N₅ (M+H)⁺ at m/z = 280.

### Example 37

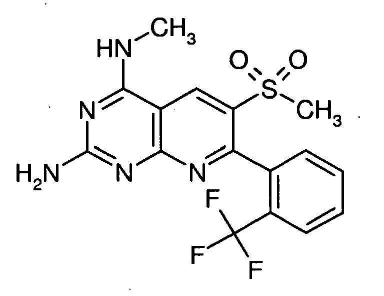

From 2,4-diamino-6-methylaminopyrimidine-5-carbaldehyde and 2-methanesulfonyl-1-(2-trifluoromethyl-phenyl)ethanone: 6-Methanesulfonyl-N4-methyl-7-(2-trifluoromethyl-phenyl)-pyrido[2,3-d]pyrimidine-2,4-diamine trifluoroacetic acid salt as a light brown solid; LRMS for C₁₆H₁₄F₃N₅O₂S (M+H)⁺ at m/z = 398.

### Example 38

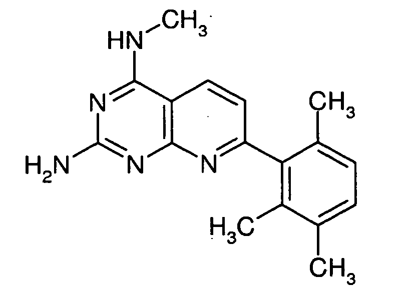

From 2,4-diamino-6-methylaminopyrimidine-5-carbaldehyde and 2',3',6'-tri methylacetophenone: N4-Methyl-7-(2,3,6-tri methyl-phenyl)-pyrido[2,3-d]pyrimidine-2,4-diamine as a light brown solid; LRMS for C₁₇H₁₉N₅ (M+H)⁺ at m/z = 294.

### Example 39

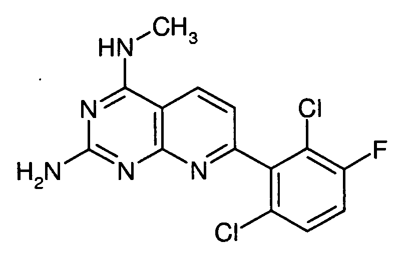

From 2,4-diamino-6-methylaminopyrimidine-5-carbaldehyde and 2',6'-dichloro-3'-fluoroacetophenone: 7-(2,6-Dichloro-3-fluoro-phenyl)-N4-methyl-pyrido[2,3-d]pyrimidine-2,4-diamine as a light brown solid; LRMS for C₁₄H₁₀Cl₂FN₅ (M+H)⁺ at m/z = 338.

### Example 40

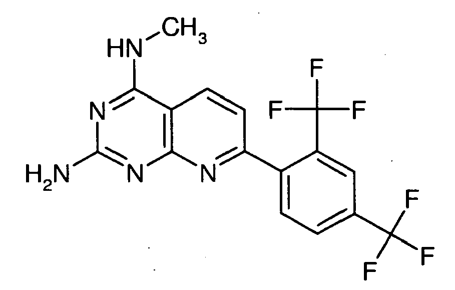

From 2,4-diamino-6-methylaminopyrimidine-5-carbaldehyde and 2'4'-bis(trifluoromethyl)acetophenone: 7-(2,4-Bis-trifluoromethyl-phenyl)-N4-methyl-pyrido[2,3-d]pyrimidine-2,4-diamine as a light brown solid; LRMS for C₁₆H₁₁F₆N₅ (M+H)⁺ at m/z = 388.

### Example 41

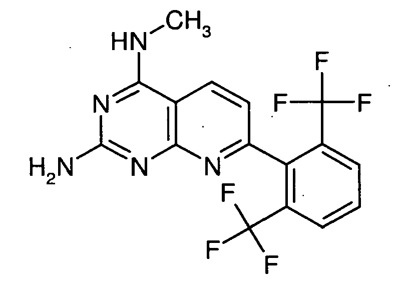

From 2,4-diamino-6-methylaminopyrimidine-5-carbaldehyde and 2',6'-bis(trifluoromethyl)acetophenone: 7-(2,6-Bis-trifluoromethyl-phenyl)-N4-methyl-pyrido[2,3-d]pyrimidine-2,4-diamine as a light brown solid; LRMS for C₁₆H₁₁F₆N₅ (M+H)⁺ at m/z = 388.

### Example 42

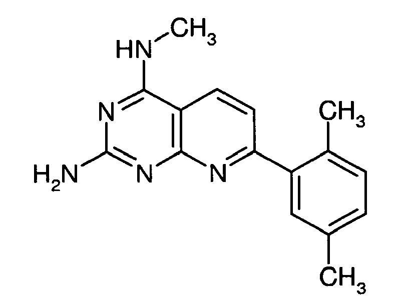

From 2,4-diamino-6-methylaminopyrimidine-5-carbaldehyde and 2',5'-di methylacetophenone: 7-(2,5-Dimethyl-phenyl)-N4-methyl-pyrido[2,3-d]pyrimidine-2,4-diamine as a light brown solid; LRMS for C₁₆H₁₇N₅ (M+H)⁺ at m/z = 280.

### Example 43

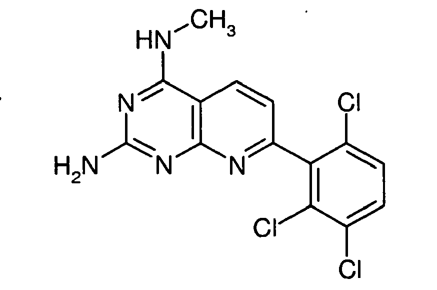

From 2,4-diamino-6-methylaminopyrimidine-5-carbaldehyde and 2',3',6'-trichloroacetophenone: N4-Methyl-7-(2,3,6-trichloro-phenyl)-pyrido[2,3-d]pyrimidine-2,4-diamine as a light brown solid; LRMS for C₁₄H₁₀Cl₃N₅ (M+H)⁺ at m/z = 354.

### Example 44

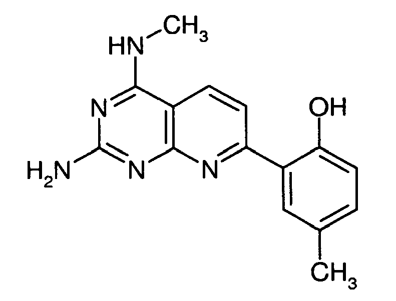

From 2,4-diamino-6-methylaminopyrimidine-5-carbaldehyde and 2'-hydroxy-5'-methylacetophenone: 2-(2-Amino-4-methylamino-pyrido[2,3-d]pyrimidin-7-yl)-4-methyl-phenol as a light brown solid; LR-MS for C₁₅H₁₅N₅O (M+H)⁺ at m/z = 282.

### Example 45

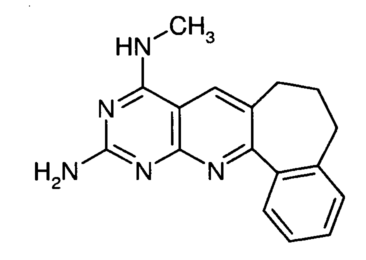

From 2,4-diamino-6-methylaminopyrimidine-5-carbaldehyde and 1-benzosuberone: N9-Methyl-6,7-di hydro-5H-10,12,13-triaza-benzo[3,4]cyclohepta[1,2-b]naphthalene-9,11-diamine trifluoroacetic acid salt as a light brown solid; LRMS for C₁₇H₁₇N₅ (M+H)⁺ at m/z = 292.

### Example 46

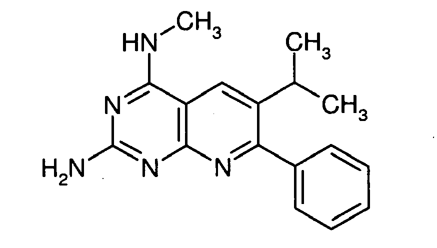

From 2,4-diamino-6-methylaminopyrimidine-5-carbaldehyde and isovalerophenone: 6-Isopropyl-N4-methyl-7-phenyl-pyrido[2,3-d]pyrimidine-2,4-diamine trifluoroacetic acid salt as a light brown solid; LRMS for C₁₇H₁₉N₅ (M+H)⁺ at m/z = 294.

### Example 47

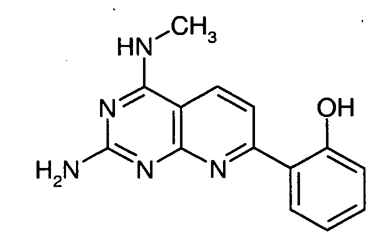

From 2,4-diamino-6-methylaminopyrimidine-5-carbaldehyde and 2'-hydroxyacetophenone: 2-(2-Amino-4-methylamino-pyrido[2,3-d]pyrimidin-7-yl)-phenol trifluoroacetic acid salt as a light brown solid; LRMS for C₁₄H₁₃N₅O (M+H)⁺ at m/z = 268.

### Example 48

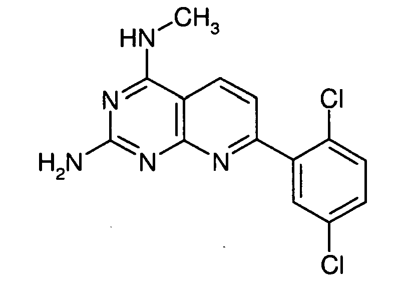

From 2,4-diamino-6-methylaminopyrimidine-5-carbaldehyde and 2',5'-dichloroacetophenone: 7-(2,5-Dichloro-phenyl)-N4-methyl-pyrido[2,3-d]pyrimidine-2,4-diamine trifluoroacetic acid salt as a light brown solid; LRMS for C₁₄H₁₁Cl₂N₅ (M+H)⁺ at m/z = 320.

### Example 49

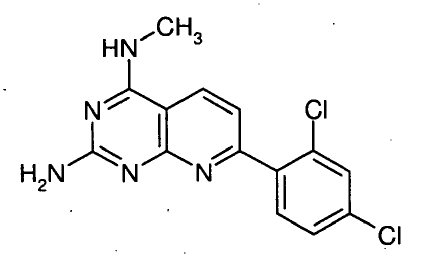

From 2,4-diamino-6-methylaminopyrimidine-5-carbaldehyde and 2',4'-dichloroacetophenone: 7-(2,4-Dichloro-phenyl)-N4-methyl-pyrido[2,3-d]pyrimidine-2,4-diamine trifluoroacetic acid salt as a light brown solid; LRMS for C₁₄H₁₁Cl₂N₅ (M+H)⁺ at m/z = 320.

### Example 50

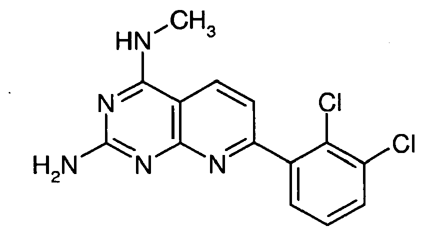

From 2,4-diamino-6-methylaminopyrimidine-5-carbaldehyde and 2',3'-dichloroacetophenone: 7-(2,3-Dichloro-phenyl)-N4-methyl-pyrido[2,3-d]pyrimidine-2,4-diamine trifluoroacetic acid salt as a light brown solid; LRMS for C₁₄H₁₁Cl₂N₅ (M+H)⁺ at m/z = 320.

### Example 51

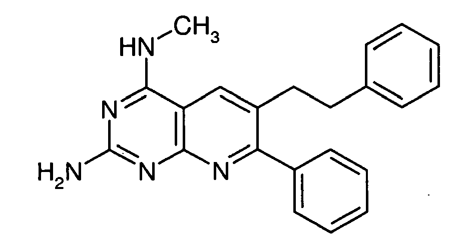

From 2,4-diamino-6-methylaminopyrimidine-5-carbaldehyde and 4-butyrylbiphenyl: N4-Methyl-6-phenethyl-7-phenyl-pyrido[2,3-d]pyrimidine-2,4-diamine trifluoroacetic acid salt as a light brown solid; LRMS for C₂₂H₂₁N₅ (M+H)⁺ at m/z = 356.

### Example 52

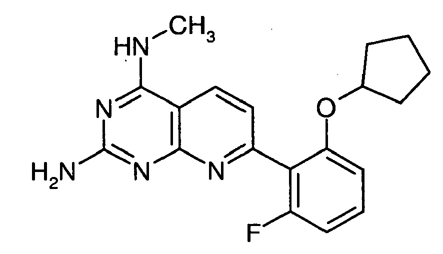

From 2,4-diamino-6-methylaminopyrimidine-5-carbaldehyde and 2',6'-difluoroacetophenone using cyclopentanol as solvent: 7-(2-Cyclopentyloxy-6-fluoro-phenyl)-N4-methyl-pyrido[2,3-d]pyrimidine-2,4-diamine trifluoroacetic acid salt as a light brown solid; LRMS for C₁₉H₂₀FN₅O (M+H)⁺ at m/z = 354.

### Example 53

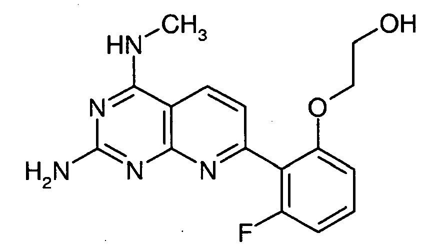

From 2,4-diamino-6-methylaminopyrimidine-5-carbaldehyde and 2',6'-difluoroacetophenone using ethylene glycol as solvent: 2-[2-(2-Amino-4-methylamino-pyrido[2,3-d]pyrimidin-7-yl)-3-fluoro-phenoxy]-ethanol trifluoroacetic acid as a light brown solid; LRMS for C₁₆H₁₆FN₅O₂ (M+H)⁺ at m/z = 330.

### Example 54

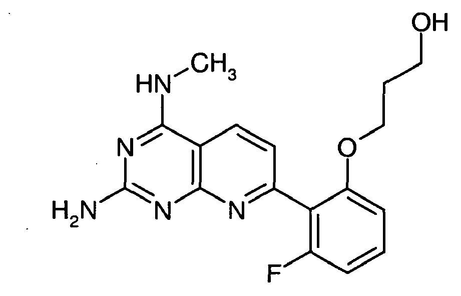

From 2,4-diamino-6-methylaminopyrimidine-5-carbaldehyde and 2',6'-difluoroacetophenone using 1,3-propanediol as solvent: 3-[2-(2-Amino-4-methylamino-pyrido[2,3-d]pyrimidin-7-yl)-3-fluoro-phenoxy]-propan-1-ol trifluoroacetic acid as a light brown solid; LRMS for C₁₇H₁₈FN₅O₂ (M+H)⁺ at m/z = 344.

### Example 55

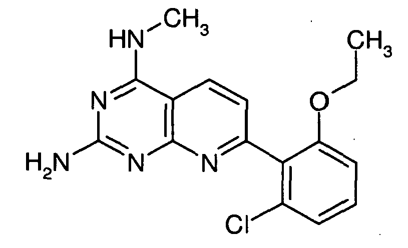

From 2,4-diami no-6-methylami nopyri midine-5-carbaldehyde, 2'-chloro-6'-fluoroacetophenone using ethanol as solvent: 7-(2-Chloro-6-ethoxy-phenyl)-N4-methyl-pyrido[2,3-d]pyrimidine-2,4-diamine trifluoroacetic acid salt as a light brown solid; LRMS for C₁₆H₁₆ClN₅O (M+H)⁺ at m/z = 330.

### Example 56

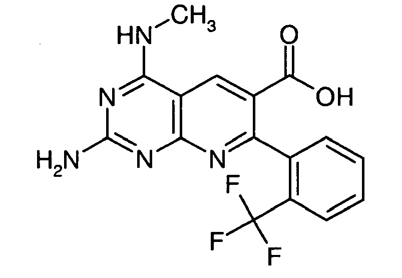

From 2,4-diamino-6-methylaminopyrimidine-5-carbaldehyde and methyl 2-(trifluoromethyl)benzoylacetate: 2-Amino-4-methylamino-7-(2-trifluoromethylphenyl)-pyrido[2,3-d]pyrimidine-6-carboxylic acid trifluoroacetic acid salt as a light brown solid; LRMS for C₁₆H₁₂F₃N₅O₂ (M+H)⁺ at m/z = 364.

### Example 57

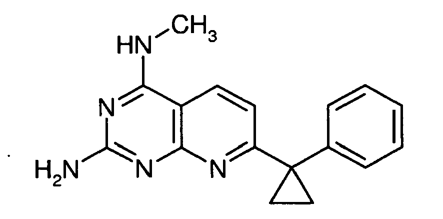

From 2,4-diamino-6-methylaminopyrimidine-5-carbaldehyde and 1-(1-phenylcyclopropyl)-ethanone: N4-Methyl-7-(1-phenyl-cyclopropyl)-pyrido[2,3-d]pyrimidine-2,4-diamine as a light brown solid; LRMS for C₁₇H₁₇N₅ (M+H)⁺ at m/z = 292.

### Example 58

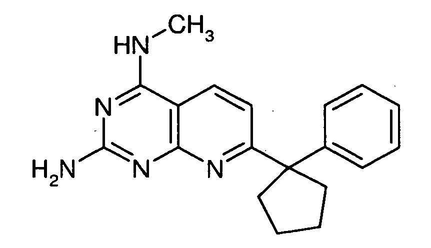

From 2,4-diamino-6-methylaminopyrimidine-5-carbaldehyde and 1-(1-phenyl-cyclopentyl)-ethanone: N4-Methyl-7-(1-phenyl-cyclopentyl)-pyrido[2,3-d]pyrimidine-2,4-diamine as a light brown solid; LRMS for C₁₉H₂₁N₅ (M+H)⁺ at m/z = 320.

### Example 59

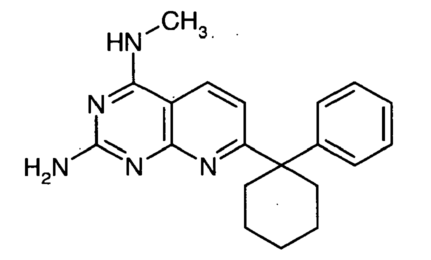

From 2,4-diamino-6-methylaminopyrimidine-5-carbaldehyde and 1-(1-phenylcyclohexyl)-ethanone: N4-Methyl-7-(1-phenyl-cyclohexyl)-pyrido[2,3-d]pyrimidine-2,4-diamine as a light brown solid; LRMS for C₂₀H₂₃N₅ (M+H)⁺ at m/z = 334.

### Example 60

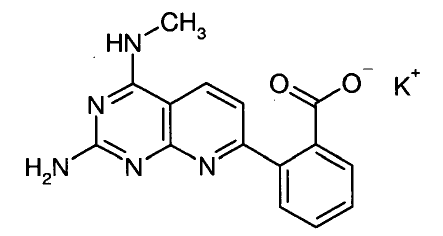

From 2,4-diamino-6-methylaminopyrimidine-5-carbaldehyde and 2-acetylbenzoic acid: potassium 2-(2-Amino-4-methylamino-pyrido[2,3-d]pyrimidin-7-yl)-benzoate as a light brown solid; LRMS for C₁₅H₁₃N₅O₂ (M+H)⁺ at m/z = 296.

### Example 61

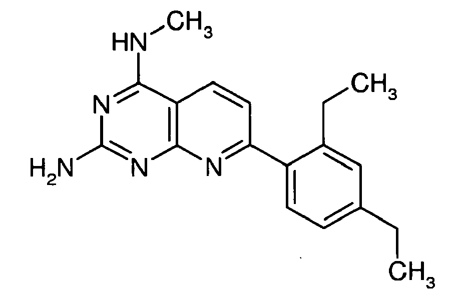

From 2,4-diamino-6-methylaminopyrimidine-5-carbaldehyde and 2',4'-diethylacetophenone: 7-(2,4-Diethyl-phenyl)-N4-methyl-pyrido[2,3-d]pyrimidine-2,4-diamine as an orange solid; LR-MS for C₁₈H₂₁N₅ (M+H)⁺ at m/z = 308.

### Example 62

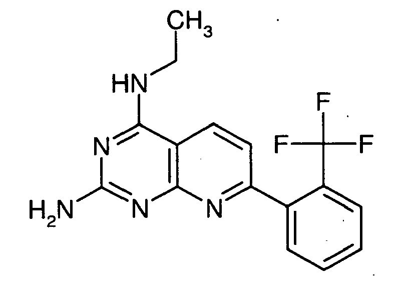

By using the 2-step procedure used in the preparation of 2,4-diamino-6-methylaminopyrimidine-5-carbaldehyde (Example 19), substituting the use of methylamine with ethylamine in step 1, gave 2,4-diamino-6-ethylaminopyrimidine-5-carbaldehyde.

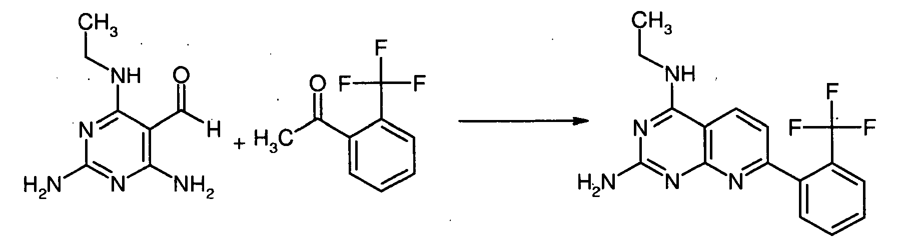

A mixture of 2,4-diamino-6-ethylaminopyrimidine-5-carbaldehyde (40 mg, 0.22 mmole), 2'-(trifluoromethyl)acetophenone (75 mg, 0.40 mmole), potassium hydroxide pellet (100 mg, 1.79 mmole) and ethanol (4 ml) in a sealed tube was heated in a 100°C oil bath for 18 h. The reaction was cooled to room temperature, concentrated *in vacuo* and purified by reversed phase HPLC to give 24 mg (24% yield) of N4-Ethyl-7-(2-trifluoromethyl-phenyl)-pyrido[2,3-d]pyrimidine-2,4-diamine trifluoroacetic acid salt as a light brown solid; LRMS for C₁₆H₁₄F₃N₅ (M+H)⁺ at m/z = 334.

In an analogous manner, there were obtained:

### Example 63

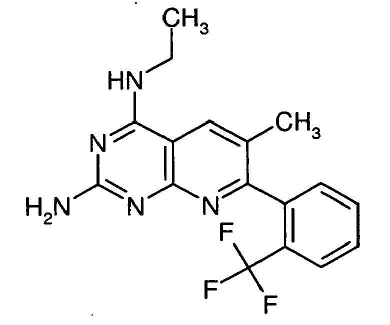

From 2,4-diamino-6-ethylaminopyrimidine-5-carbaldehyde and 2'-(trifluoromethyl)propiophenone: N4-Ethyl-6-methyl-7-(2-trifluoromethyl-phenyl)-pyrido[2,3-d]pyrimidine-2,4-diamine as a light brown solid; LRMS for C₁₇H₁₆F₃N₅ (M+H)⁺ at m/z = 348.

### Example 64

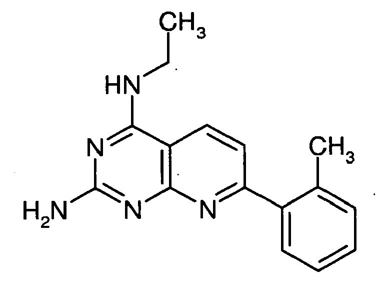

From 2,4-diamino-6-ethylaminopyrimidine-5-carbaldehyde and 2'-methylacetophenone: N4-Ethyl-7-o-tolyl-pyrido[2,3-d]pyrimidine-2,4-diamine trifluoroacetic acid salt as a light brown solid; LRMS for C₁₆H₁₇N₅ (M+H)⁺ at m/z = 280.

### Example 65

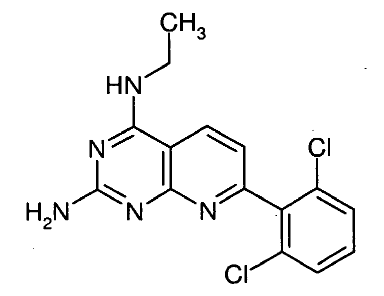

From 2,4-diamino-6-ethylaminopyrimidine-5-carbaldehyde and 2',6'-dichloroacetophenone: 7-(2,6-Dichloro-phenyl)-N4-ethyl-pyrido[2,3-d]pyrimidine-2,4-diamine trifluoroacetic acid salt as a light brown solid; LRMS for C₁₅H₁₃Cl₂N₅ (M+H)⁺ at m/z = 334.

### Example 66

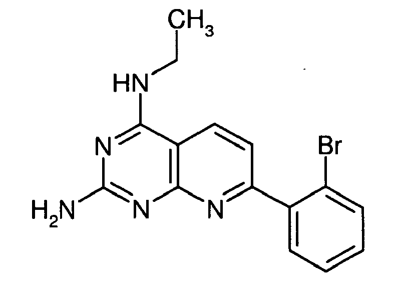

From 2,4-diamino-6-ethylaminopyrimidine-5-carbaldehyde and 2'-bromoacetophenone: 7-(2-Bromo-phenyl)-N4-ethyl-pyrido[2,3-d]pyri midi ne-2,4-diamine trifluoroacetic acid salt as a light brown solid; LRMS for C₁₅H₁₄BrN₅ (M+H)⁺ at m/z = 344.

### Example 67

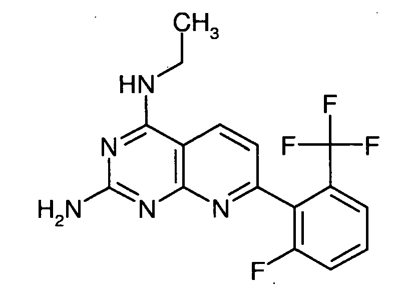

From 2,4-diamino-6-ethylaminopyrimidine-5-carbaldehyde and 2'-fluoro-6'-(trifluoromethyl)acetophenone: N4-Ethyl-7-(2-fluoro-6-trifluoromethyl-phenyl)-pyrido[2,3-d]pyrimidine-2,4-diamine trifluoroacetic acid salt as a light brown solid; LRMS for C₁₆H₁₃F₄N₅ (M+H)⁺ at m/z = 352.

### Example 68

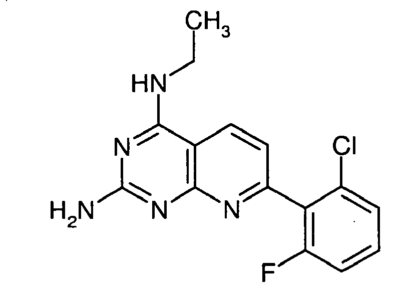

From 2,4-diamino-6-ethylaminopyrimidine-5-carbaldehyde and 2'-chloro-6'-fluoroacetophenone: 7-(2-Chloro-6-fluoro-phenyl)-N4-ethyl-pyrido[2,3-d]pyrimidine-2,4-diamine trifluoroacetic acid salt as a light brown solid; LRMS for C₁₅H₁₃ClFN₅ (M+H)⁺ at m/z = 318.

### Example 69

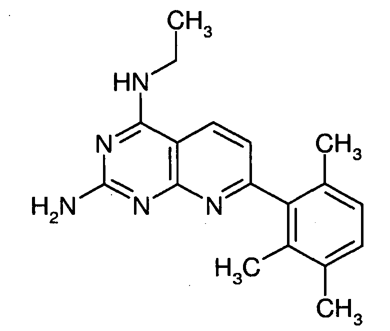

From 2,4-diamino-6-ethylaminopyrimidine-5-carbaldehyde and 2',3',6'-tri methylacetophenone: N4-Ethyl-7-(2,3,6-tri methyl-phenyl)-pyrido[2,3-d]pyrimidine-2,4-diamine trifluoroacetic acid salt as a light brown solid; LRMS for C₁₈H₂₁N₅ (M+H)⁺ at m/z = 308.

### Example 70

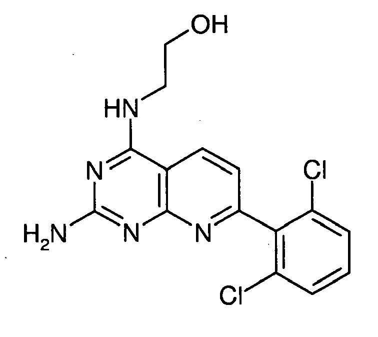

Using the 2-step procedure used in the preparation of 2,4-diamino-6-methylaminopyrimidine-5-carbaldehyde (Example 19), substituting the use of methylamine with ethanolamine in step 1, gave 2,4-Diamino-6-(2-hydroxy-ethylaminopyrimidine-5-carbaldehyde. From 2,4-Diamino-6-(2-hydroxy-ethylamino)-pyrimidine-5-carbaldehyde and 2',6'-dichloroacetophenone: 2-[2-Amino-7-(2,6-dichloro-phenyl)-pyrido[2,3-d]pyrimidin-4-ylamino]-ethanol as an orange solid; LR-MS for C₁₅H₁₃Cl₂N₅O (M+H)⁺ at m/z = 350.

### Example 71

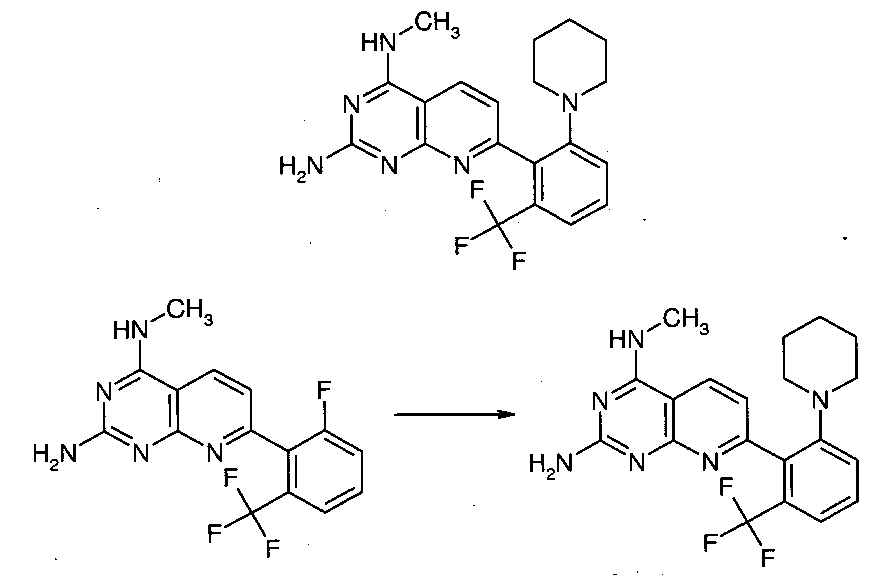

To a mixture of 7-(2-fluoro-6-trifluoromethyl-phenyl)-N4-methyl-pyrido[2,3-d]pyrimidine-2,4-diamine (30 mg, 0.089 mmole), piperidine (39 mg, 0.46 mmole) and potassium carbonate (60 mg, 0.43 mmole) in *N,N*-dimethylformamide (4 ml) or 1-methyl-2-pyrrolidinone (4 ml) in a sealed tube was heated in a 190°C oil bath overnight. After cooling to room temperature, the reaction was concentrated in *vacuo* and purified by reversed phase HPLC to give 23 mg (41 % yield) of N4-Methyl-7-(2-piperidin-1-yl-6-trifluoromethyl-phenyl)-pyrido[2,3-d]pyrimidine-2,4-diamine as a light brown solid; LRMS for C₂₀H₂₁F₃N₆ (M+H)⁺ at m/z = 403.

In an analogous manner, there were obtained:

### Example 72

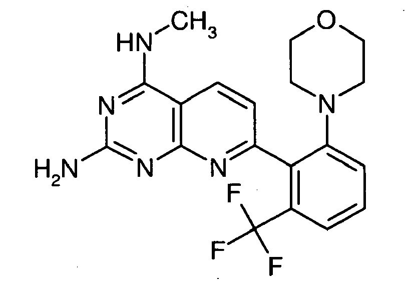

From 7-(2-fluoro-6-trifluoromethyl-phenyl)-N4-methyl-pyrido[2,3-d]pyrimidine-2,4-diamine and morpholine: N4-Methyl-7-(2-morpholin-4-yl-6-trifluoromethyl-phenyl)-pyrido[2,3-d]pyrimidine-2,4-diamine trifluoroacetic acid salt as a light brown solid; LRMS for C₁₉H₁₉F₃N₆O (M+H)⁺ at m/z = 405.

### Example 73

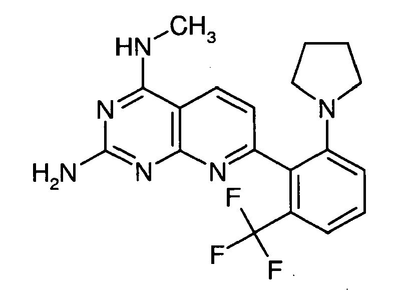

From 7-(2-fluoro-6-trifluoromethyl-phenyl)-N4-methyl-pyrido[2,3-d]pyrimidine-2,4-diamine and pyrrolidine: 7-(2,4-Dimethyl-phenyl)-N4-methyl-pyrido[2,3-d]pyrimidine-2,4-diamine as a light brown solid; LRMS for C₁₉H₁₉F₃N₆ (M+H)⁺ at m/z = 389.

### Example 74

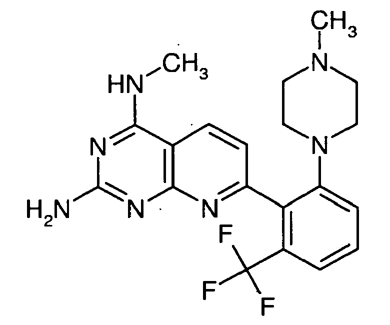

From 7-(2-fluoro-6-trifluoromethyl-phenyl)-N4-methyl-pyrido[2,3-d]pyrimidine-2,4-diamine and N-methylpiperazine: N4-Methyl-7-[2-(4-methyl-piperazin-1-yl)-6-trifluoromethyl-phenyl]-pyrido[2,3-d]pyrimidine-2,4-diamine trifluoroacetic acid salt as a light brown solid; LRMS for C₂₀H₂₂F₃N₇ (M+H)⁺ at m/z = 418.

### Example 75

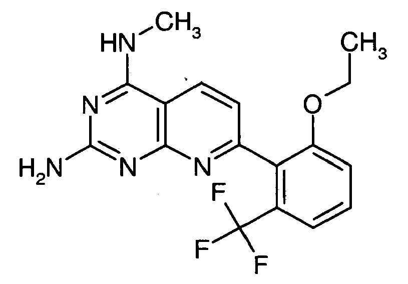

From 7-(2-fluoro-6-trifluoromethyl-phenyl)-N4-methyl-pyrido[2,3-d]pyrimidine-2,4-diamine and sodium ethoxide: 7-(2-Ethoxy-6-trifluoromethyl-phenyl)-N4-methyl-pyrido[2,3-d]pyrimidine-2,4-diamine trifluoroacetic acid salt as a light brown solid; LRMS for C₁₇H₁₆F₃N₅O (M+H)⁺ at m/z = 364.

### Example 76

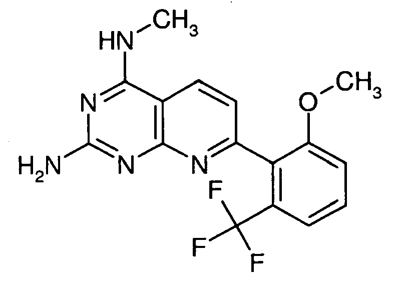

From 7-(2-fluoro-6-trifluoromethyl-phenyl)-N4-methyl-pyrido[2,3-d]pyrimidine-2,4-diamine and sodium methoxide: 7-(2-Methoxy-6-trifluoromethyl-phenyl)-N4-methyl-pyrido[2,3-d]pyrimidine-2,4-diamine trifluoroacetic acid salt as a light brown solid; LRMS for C₁₆H₁₄F₃N₅O (M+H)⁺ at m/z = 350.

### Example 77

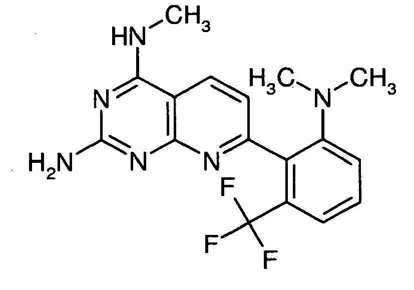

From 7-(2-fluoro-6-trifluoromethyl-phenyl)-N4-methyl-pyrido[2,3-d]pyrimidine-2,4-diamine and dimethylamine: 7-(2-Dimethylamino-6=trifluoromethyl-phenyl)-N4-methyl-pyrido[2,3-d]pyrimidine-2,4-diamine as a light brown solid; LRMS for C₁₇H₁₇F₃N₆ (M+H)⁺ at m/z = 363.

### Example 78

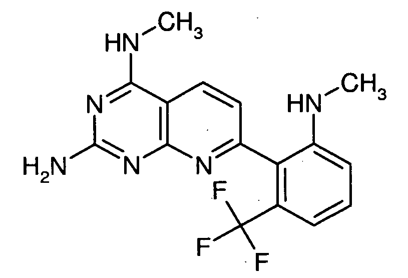

From 7-(2-fluoro-6-trifluoromethyl-phenyl)-N4-methyl-pyrido[2,3-d]pyrimidine-2,4-diamine and methylamine: N4-Methyl-7-(2-methylamino-6-trifluoromethyl-phenyl)-pyrido[2,3-d]pyrimidine-2,4-diamine trifluoroacetic acid salt as a light brown solid; LRMS for C₁₆H₁₅F₃N₆ (M+H)⁺ at m/z = 349.

### Example 79

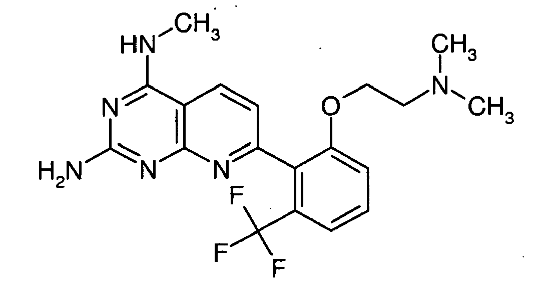

From 7-(2-fluoro-6-trifluoromethyl-phenyl)-N4-methyl-pyrido[2,3-d]pyrimidine-2,4-diamine, 2-dimethylaminoethanol and sodium hydride: 7-[2-(2-Dimethylaminoethoxy)-6-trifluoromethyl-phenyl]-N4-methyl-pyrido[2,3-d]pyrimidine-2,4-diamine trifluoroacetic acid salt as a light brown solid; LRMS for C₁₉H₂₁F₃N₆O (M+H)⁺ at m/z = 407.

### Example 80

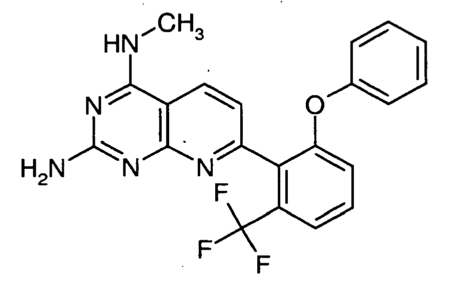

From 7-(2-fluoro-6-trifluoromethyl-phenyl)-N4-methyl-pyrido[2,3-d]pyrimidine-2,4-diamine, phenol and sodium hydride: N4-Methyl-7-(2-phenoxy-6-trifluoromethylphenyl)-pyrido[2,3-d]pyrimidine-2,4-diamine trifluoroacetic acid salt as a light brown solid; LRMS for C₂₁H₁₆F₃N₅O (M+H)⁺ at m/z = 412.

### Example 81

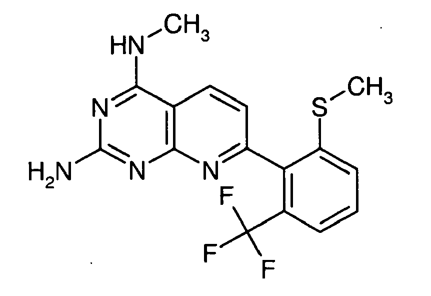

From 7-(2-fluoro-6-trifluoromethyl-phenyl)-N4-methyl-pyrido[2,3-d]pyrimidine-2,4-diamine, sodium methanethiolate: N4-Methyl-7-(2-methylsulfanyl-6-trifluoromethyl-phenyl)-pyrido[2,3-d]pyrimidine-2,4-diamine trifluoroacetic acid salt as a light brown solid; LRMS for C₁₆H₁₄F₃N₅S (M+H)⁺ at m/z = 366.

### Example 82

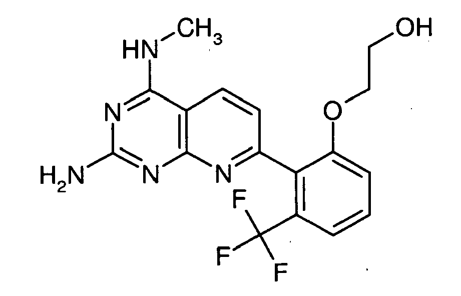

From 7-(2-fluoro-6-trifluoromethyl-phenyl)-N4-methyl-pyrido[2,3-d]pyrimidine-2,4-diamine, ethylene glycol and sodium hydride: 2-[2-(2-Amino-4-methylamino-pyrido[2,3-d]pyrimidin-7-yl)-3-trifluoromethyl-phenoxy]-ethanol trifluoroacetic acid salt as a light brown solid; LRMS for C₁₇H₁₆F₃N₅O₂ (M+H)⁺ at m/z = 380.

### Example 83

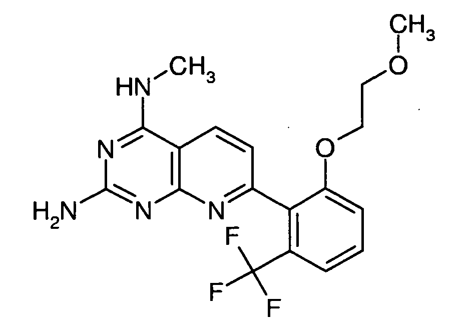

From 7-(2-fluoro-6-trifluoromethyl-phenyl)-N4-methyl-pyrido[2,3-d]pyrimidine-2,4-diamine, 2-methoxyethanol and sodium hydride: 7-[2-(2-Methoxy-ethoxy)-6-trifluoromethyl-phenyl]-N4-methyl-pyrido[2,3-d]pyrimidine-2,4-diamine trifluoroacetic acid salt as a light brown solid; LRMS for C₁₈H₁₈F₃N₅O₂ (M+H)⁺ at m/z = 394.

### Example 84

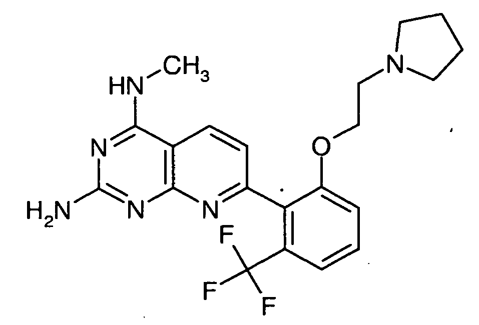

From 7-(2-fluoro-6-trifluoromethyl-phenyl)-N4-methyl-pyrido[2,3-d]pyrimidine-2,4-diamine, 1-(2-hydroxyethyl)pyrrolidine and sodium hydride: N4-Methyl-7-[2-(2-pyrrolidin-1-yl-ethoxy)-6-trifluoromethyl-phenyl]-pyrido[2,3-d]pyrimidine-2,4-diamine trifluoroacetic acid salt as a light brown solid; LRMS for C₂₁H₂₃F₃N_{6O} (M+H)⁺ at m/z = 433.

### Example 85

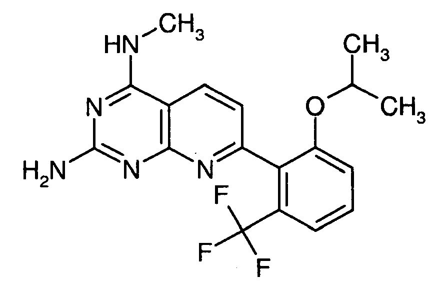

From 7-(2-fluoro-6-trifluoromethyl-phenyl)-N4-methyl-pyrido[2,3-dJpyrimidine-2,4-diamine, 2-propanol and sodium hydride: 7-(2-Isopropoxy-6-trifluoromethylphenyl)-N4-methyl-pyrido[2,3-d]pyrimidine-2,4-diamine trifluoroacetic acid salt as a light brown solid; LRMS for C₁₈H₁₈F₃N₅O (M+H)⁺ at m/z = 378.

### Example 86

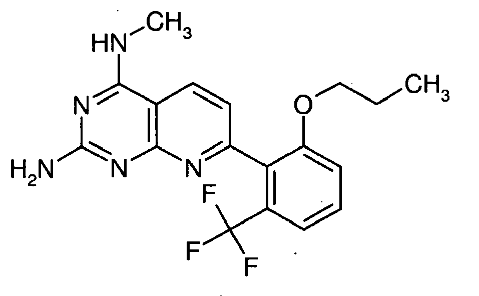

From 7-(2-fluoro-6-trifluoromethyl-phenyl)-N4-methyl-pyrido[2,3-d]pyrimidine-2,4-diamine, 1-propanol and sodium hydride: N4-Methyl-7-(2-propoxy-6-trifluoromethyl-phenyl)-pyrido[2,3-d]pyrimidine-2,4-diamine trifluoroacetic acid salt as a light brown solid; LRMS for C₁₈H₁₈F₃N₅O (M+H)⁺ at m/z = 378.

### Example 87

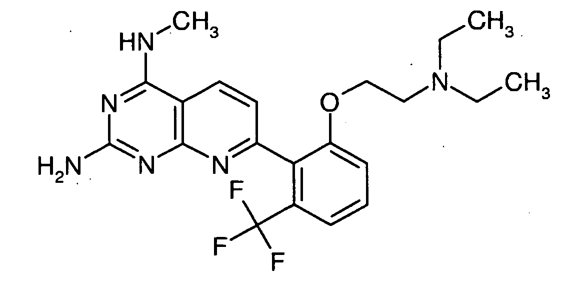

From 7-(2-fluoro-6-trifluoromethyl-phenyl)-N4-methyl-pyrido[2,3-d]pyrimidine-2,4-diamine, 2-diethylaminoethanol and sodium hydride: 7-[2-(2-Diethylamino-ethoxy)-6-trifluoromethyl-phenyl]-N4-methyl-pyrido[2,3-d]pyrimidine-2,4-diamine trifluoroacetic acid salt as a light brown solid; LRMS for C₂₁H₂₅F₃NeO (M+H)⁺ at m/z = 435.

### Example 88

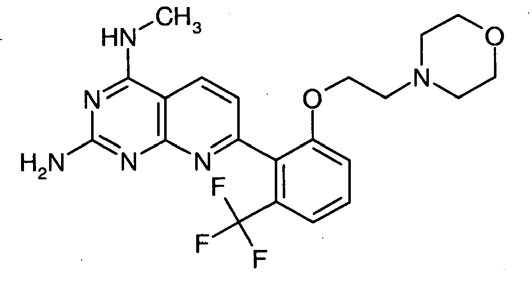

From 7-(2-fluoro-6-trifluoromethyl-phenyl)-N4-methyl-pyrido[2,3-d]pyrimidine-2,4-diamine, N-(2-hydroxyethyl)morpholine and sodium hydride: N4-Methyl-7-[2-(2-morpholin-4-yl-ethoxy)-6-trifluoromethyl-phenyl]-pyrido[2,3-d]pyrimidine-2,4-diamine trifluoroacetic acid salt as a light brown solid; LRMS for C₂₁H₂₃F₃N₆O₂ (M+H)⁺ at m/z = 449.

### Example 89

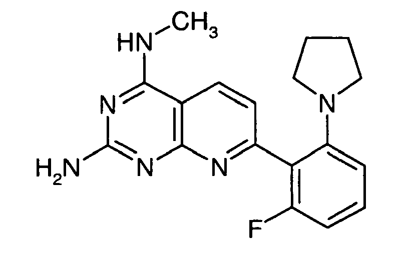

From 7-(2,6-difluoro-phenyl)-N4-methyl-pyrido[2,3-d]pyrimidine-2,4-diamine, and pyrrolidine: 7-(2-fluoro-6-pyrrolidin-1-yl-phenyl)-N4-methyl-pyrido[2,3-d]pyrimidine-2,4-diamine as a dark-yellow solid; (ES)⁺-HRMS m/e calcd for C₁₈H₁₉FN₆ (M+H)⁺ 339.1730, found 339.1728.

### Example 90

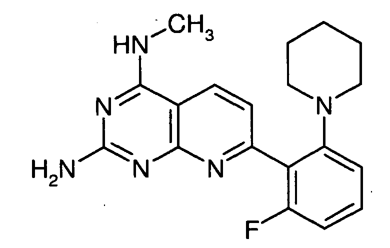

From 7-(2,6-difluoro-phenyl)-N4-methyl-pyrido[2,3-d]pyrimidine-2,4-diamine and piperidine: 7-(2-Fluoro-6-piperidin-1-yl-phenyl)-N4-methyl-pyrido[2,3-d]pyrimidine-2,4-diamine as a yellow solid; EI-HRMS m/e calcd for C₁₉H₂₁FN₆ (M⁺) 352.1812, found 352.1813.

### Example 91

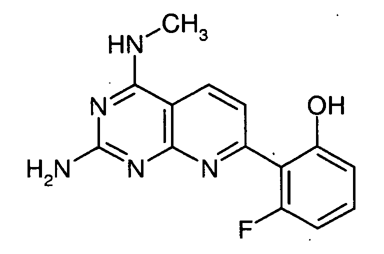

Obtained as a by-product from 7-(2,6-difluoro-phenyl)-N4-methyl-pyrido[2,3-d]pyrimidine-2,4-diamine, phenol and sodium hydride: 2-(2-amino-4-methylamino-pyrido[2,3-d]pyrimidin-7-yl)-3-fluoro-phenol as a yellow solid; EI-HRMS m/e calcd for C₁₄H₁₂FN₅O (M⁺) 285.1029, found 285.1026.

### Example 92

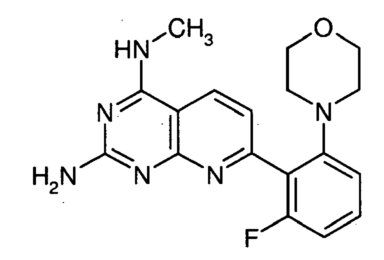

From 7-(2,6-difluoro-phenyl)-N4-methyl-pyrido[2,3-d]pyrimidine-2,4-diamine and morpholine: 7-(2-Fluoro-6-morpholino-4-yl-phenyl)-N4-methyl-pyrido[2,3-d]pyrimidine-2,4-diamine as a yellow solid; LRMS for C₁₈H₁₉FN₆O (M+H)⁺ at m/z = 355.

### Example 93

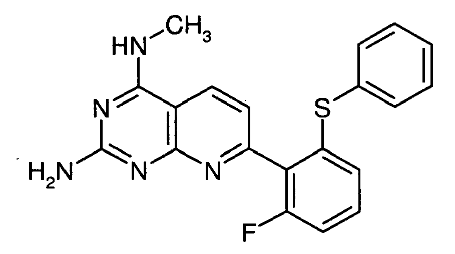

From 7-(2,6-difluoro-phenyl)-N4-methyl-pyrido[2,3-d]pyrimidine-2,4-diamine and Benzenethiol: 7-(2-Fluoro-6-phenylsulfanyl-phenyl)-N4-methyl-pyrido[2,3-d]pyrimidine-2,4-diamine trifluoroacetic acid salt as a brwon solid; (ES)⁺-HRMS m/e calcd for C₂₀H₁₆FN₅S (M+H)⁺ 378.1183, found 378.1181.

### Example 94

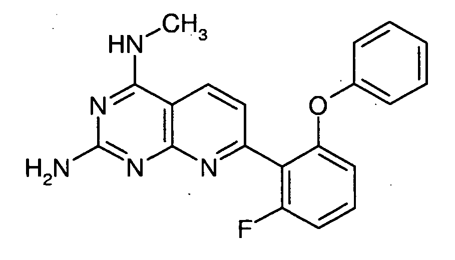

From 7-(2,6-difluoro-phenyl)-N4-methyl-pyrido[2,3-d]pyrimidine-2,4-diamine and Phenol: 7-(2-Fluoro-6-phenoxy-phenyl)-N4-methyl-pyrido[2,3-d]pyri midi ne-2,4-diamine trifluoroacetic acid salt as a brown solid; (ES)⁺-HRMS m/e calcd for C₂₀H₁₆FN₅O (M+H)⁺ 362.1412, found 362.1410.

### Example 95

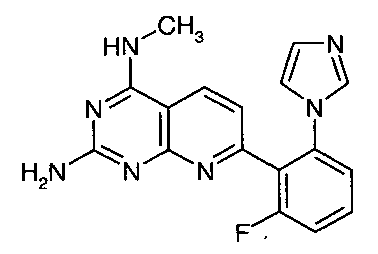

From 7-(2,6-Difluoro-phenyl)-N4-methyl-pyrido[2,3-d]pyrimidine-2,4-diamine and 1 H-Imidazole: 7-(2-Fluoro-6-imidazol-1-yl-phenyl)-N4-methyl-pyrido[2,3-d]pyrimidine-2,4-diamine as a light yellow solid; (ES)⁺-HRMS m/e calcd for C₁₇H₁₄FN₇ (M+H)⁺ 336.1368, found 336.1370.

### Example 96

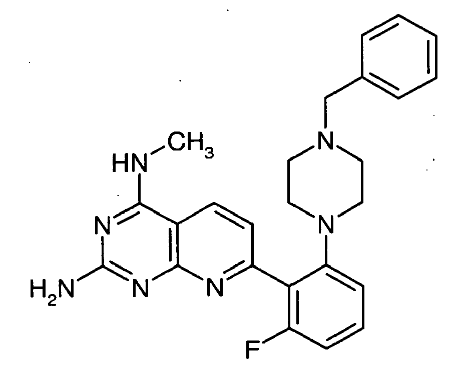

From 7-(2,6-difluoro-phenyl)-N4-methyl-pyrido[2,3-d]pyrimidine-2,4-diamine and 1-Benzyl-piperazi ne: 7-[2-(4-Benzyl-piperazin-1-yl)-6-fluoro-phenyl]-N4-methyl-pyrido[2,3-d]pyrimidine-2,4-diamine trifluoroacetic acid salt as a brown solid; (ES)⁺-HRMS m/e calcd for C₂₅H₂₆FN₇ (M+H)⁺ 444.2307, found 444.2305.

### Example 97

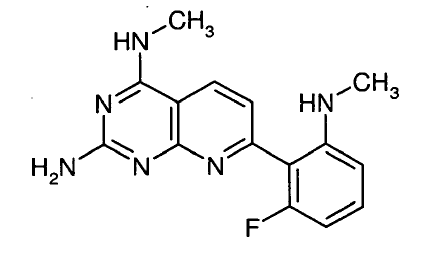

From 7-(2,6-difluoro-phenyl)-N4-methyl-pyrido[2,3-d]pyrimidine-2,4-diamine and Methylamine: 7-(2-Fluoro-6-methylami no-phenyl)-N4-methyl-pyrido[2,3-d]pyrimidine-2,4-diamine trifluoroacetic acid salt as a brown solid; (ES)⁺-HRMS m/e calcd for C₁₅H₁₅FN₆ (M+H)⁺ 299.1415, found 299.1417.

### Example 98

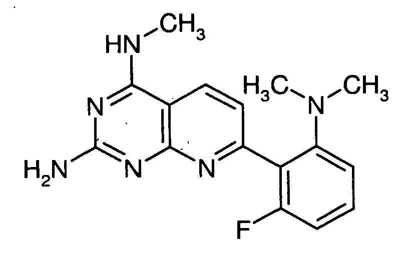

From 7-(2,6-difluoro-phenyl)-N4-methyl-pyrido[2,3-d]pyrimidine-2,4-diamine and Dimethylamine: 7-(2-Dimethylamino-6-fluoro-phenyl)-N4-methyl-pyrido[2,3-d]pyrimidine-2,4-diamine trifluoroacetic acid salt as a brown solid; (ES)⁺-HRMS m/e calcd for C₁₆H₁₇FN₆ (M+H)⁺ 313.1572, found 313.1570.

### Example 99

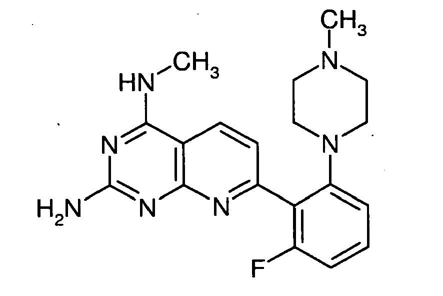

From 7-(2,6-difluoro-phenyl)-N4-methyl-pyrido[2,3-d]pyrimidine-2,4-diamine and 1-Methyl-piperazine: 7-[2-Fluoro-6-(4-methyl-piperazin-1-yl)-phenyl]-N4-methyl-pyrido[2,3-d]pyrimidine-2,4-diamine trifluoroacetic acid salt as a brown solid; (ES)⁺-HRMS m/e calcd for C₁₉H₂₂FN₇ (M+H)⁺ 368.1994, found 368.1992.

### Example 100

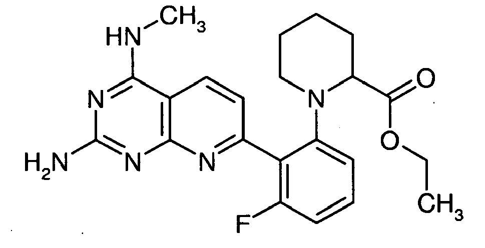

From 7-(2,6-Difluoro-phenyl)-N4-methyl-pyrido[2,3-d]pyrimidine-2,4-diamine and Piperidine-2-carboxylic acid ethyl ester: 1-[2-(2-Amino-4-methylamino-pyrido[2,3-d]pyrimidin-7-yl)-3-fluoro-phenyl]-piperidine-2-carboxylic acid ethyl ester trifluoroacetic acid salt as a yellow solid; (ES)⁺-HRMS m/e calcd for C₂₂H₂₅FN₆O₂ (M+H)⁺ 425.2098, found 425.2096.

### Example 101'

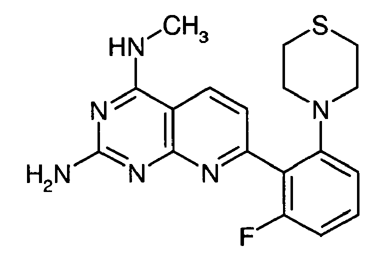

From 7-(2,6-difluoro-phenyl)-N4-methyl-pyrido[2,3-d]pyrimidine-2,4-diamine and Thiomorpholine: 7-(2-Fluoro-6-thiomorpholin-4-yl-phenyl)-N4-methyl-pyrido[2,3-d]pyrimidine-2,4-diamine trifluoroacetic acid salt as a brown solid; (ES)⁺-HRMS m/e calcd for C₁₈H₁₉FN₆S (M+H)⁺ 371.1449, found 371.1451.

### Example 102

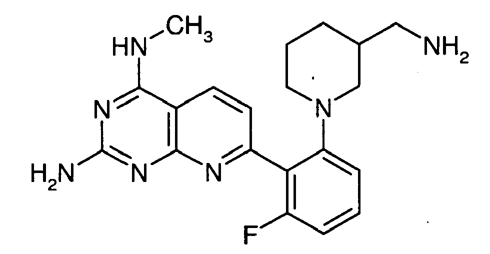

From 7-(2,6-difluoro-phenyl)-N4-methyl-pyrido[2,3-d]pyrimidine-2,4-diamine and C-Piperidin-3-yl-methylamine: 7-[2-(3-Aminomethyl-piperidin-1-yl)-6-fluoro-phenyl]-N4-methyl-pyrido[2,3-d]pyrimidine-2,4-diamine trifluoroacetic acid salt as a brown solid; (ES)⁺-HRMS m/e calcd for C₂₀H₂₄FN₇ (M+H)⁺ 382.2150, found 382.2152.

### Example 103

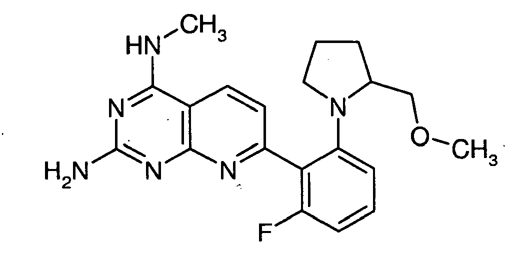

From 7-(2,6-difluoro-phenyl)-N4-methyl-pyrido[2,3-d]pyrimidine-2,4-diamine and 2-Methoxymethyl-pyrrolidi ne: 7-[2-Fluoro-6-(2-methoxymethyl-pyrrolidin-1-yl)-phenyl]-N4-methyl-pyrido[2,3-d]pyrimidine-2,4-diamine trifluoroacetic acid salt as a brown solid; (ES)⁺-HRMS m/e calcd for C₂₀H₂₃FN₆O (M+H)⁺ 383.1990, found 383.1993.

### Example 104

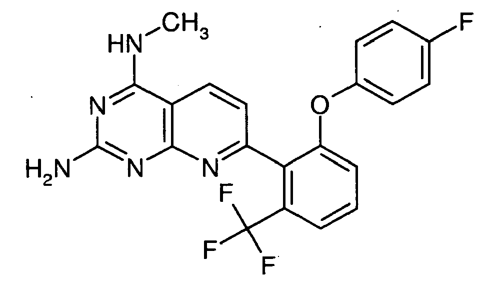

From 7-(2-fluoro-6-trifluoromethyl-phenyl)-N4-methyl-pyrido[2,3-d]pyrimidine-2,4-diamine, 4-fluorophenol and sodium hydride: 7-[2-(4-Fluoro-phenoxy)-6-. trifluoromethyl-phenyl]-N4-methyl-pyrido[2,3-d]pyrimidine-2,4-diamine trifluoroacetic acid salt as a light brown solid; LRMS for C₂₁H₁₅F₄N₅O (M+H)⁺ at m/z = 430.

### Example 105

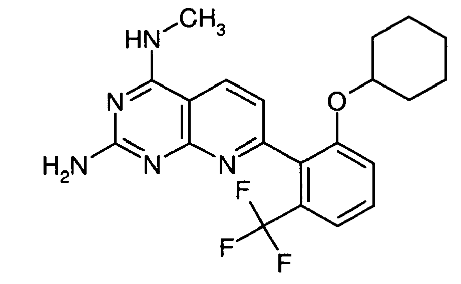

From 7-(2-fluoro-6-trifluoromethyl-phenyl)-N4-methyl-pyrido[2,3-d]pyrimidine-2,4-diamine, cyclohexanol and sodium hydride: 7-(2-Cyclohexyloxy-6-trifluoromethylphenyl)-N4-methyl-pyrido[2,3-d]pyrimidine-2,4-diamine trifluoroacetic acid salt as a light brown solid; LRMS for C₂₁H₂₂F₃N₅O (M+H)⁺ at m/z = 418.

### Example 106

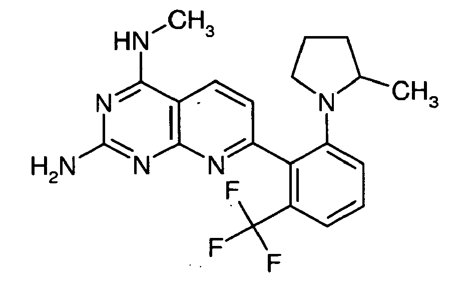

From 7-(2-fluoro-6-trifluoromethyl-phenyl)-N4-methyl-pyrido[2,3-d]pyrimidine-2,4-diamine and 2-methylpyrrolidine (racemic): N4-Methyl-7-[2-(2-methyl-pyrrolidin-1-yl)-6-trifluoromethyl-phenyl]-pyrido[2,3-d]pyrimidine-2,4-diamine as a light brown solid; LRMS for C₂₀H₂₁F₃N₆ (M+H)⁺ at m/z = 403.

### Example 107

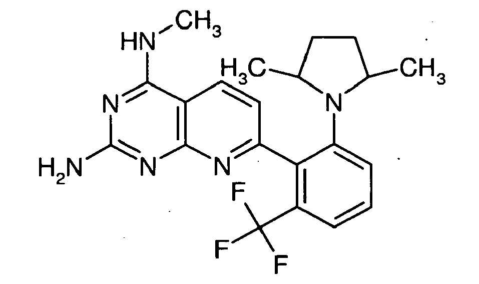

From 7-(2-fluoro-6-trifluoromethyl-phenyl)-N4-methyl-pyrido[2,3-d]pyrimidine-2,4-diamine and 2,5-dimethylpyrrolidine (mixture of *cis-* and *trans*-)*:* 7-[2-(2,5-Dimethyl-pyrrolidin-1-yl)-6-trifluoromethyl-phenyl]-N4-methyl-pyrido[2,3-d]pyrimidine-2,4-diamine trifluoroacetic acid salt as a light brown solid; LRMS for C₂₁H₂₃F₃N₆ (M+H)⁺ at m/z = 417.

### Example 108

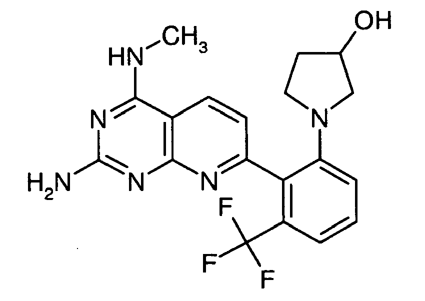

From 7-(2-fluoro-6-trifluoromethyl-phenyl)-N4-methyl-pyrido[2,3-d]pyrimidine-2,4-diamine and racemic 3-hydroxypyrrolidine: 1-[2-(2-Amino-4-methylamino-pyrido[2,3-d]pyrimidin-7-yl)-3-trifluoromethyl-phenyl]-pyrrolidin-3-ol trifluoroacetic acid salt as a light brown solid; LRMS for C₁₉H₁₉F₃N₆O (M+H)⁺ at m/z = 405.

### Example 109

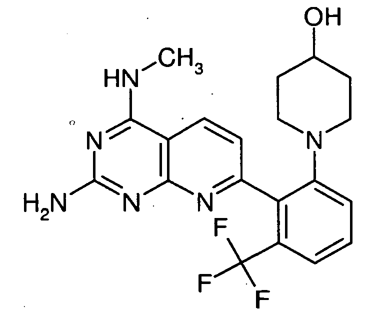

From 7-(2-fluoro-6-trifluoromethyl-phenyl)-N4-methyl-pyrido[2,3-d]pyrimidine-2,4-diamine and 4-hydroxypiperidine: 1-[2-(2-Amino-4-methylamino-pyrido[2,3-d]pyrimidin-7-yl)-3-trifluoromethyl-phenyl]-piperidin-4-ol trifluoroacetic acid salt as a light brown solid; LRMS for C₂₀H₂₁F₃N₆O (M+H)⁺ at m/z = 419.

### Example 110

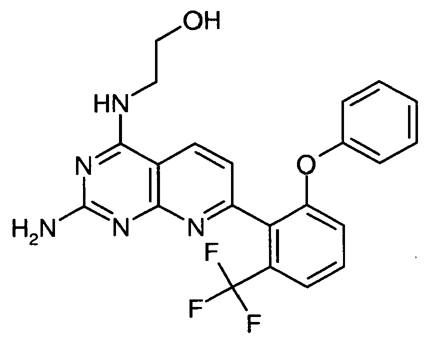

From 2-[2-Amino-7-(2-fluoro-6-trifluoromethyl-phenyl)-pyrido[2,3-d]pyrimidin-4-ylamino]-ethanol, phenol and sodium hydride: 2-[2-Amino-7-(2-phenoxy-6-trifluoromethyl-phenyl)-pyrido[2,3-d]pyrimidin-4-ylamino]-ethanol trifluoroacetic acid salt as a light brown solid; LRMS for C₂₂H₁₈F₃N₅O₂ (M+H)⁺ at m/z = 442.

### Example 111

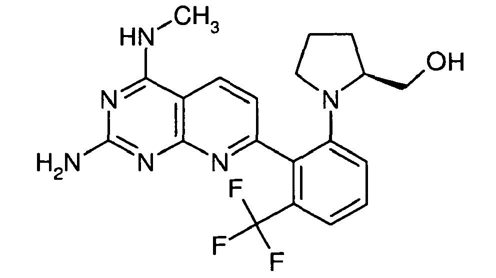

From 7-(2-fluoro-6-trifluoromethyl-phenyl)-N4-methyl-pyrido[2,3-d]pyrimidine-2,4-diamine and (L)-prolinol: {1-[2-(2-Amino-4-methylamino-pyrido[2,3-d]pyrimidin-7-yl)-3-trifluoromethyl-phenyl]-pyrrolidin-2-yl}-methanol trifluoroacetic acid salt as a light brown solid; LRMS for C₂₀H₂₁F₃N₆O (M+H)⁺ at m/z = 419.

### Example 112

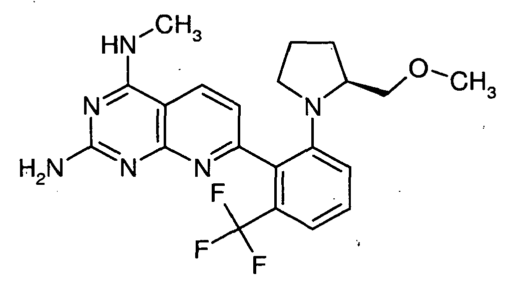

From 7-(2-fluoro-6-trifluoromethyl-phenyl)-N4-methyl-pyrido[2,3-d]pyrimidine-2,4-diamine and (S)-2-(methoxymethyl)pyrrolidine: 7-[2-(2-Methoxymethyl-pyrrolidin-1-yl)-6-trifluoromethyl-phenyl]-N4-methyl-pyrido[2,3-d]pyrimidine-2,4-diamine trifluoroacetic acid salt as a light brown solid; LRMS for C₂₁H₂₃F₃N₆O (M+H)⁺ at m/z = 433.

### Example 113

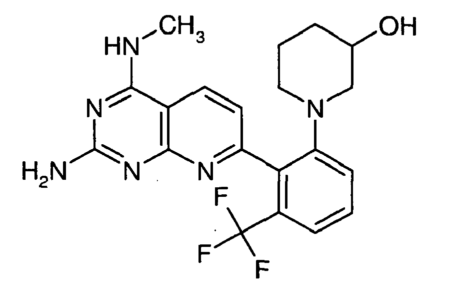

From 7-(2-fluoro-6-trifluoromethyl-phenyl)-N4-methyl-pyrido[2,3-d]pyrimidine-2,4-diamine and racemic 3-hydroxypiperidine: 1-[2-(2-Amino-4-methylamino-pyrido[2,3-d]pyrimidin-7-yl)-3-trifluoromethyl-phenyl]-piperidin-3-ol trifluoroacetic acid salt as a light brown solid; LRMS for C₂₀H₂₁F₃N₆O (M+H)⁺ at m/z =419.

### Example 114

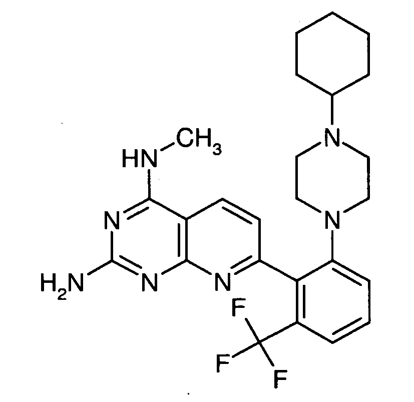

From 7-(2-fluoro-6-trifluoromethyl-phenyl)-N4-methyl-pyrido[2,3-d]pyrimidine-2,4-diamine and 1-cyclohexylpiperazine: 7-[2-(4-Cyclohexyl-piperazin-1-yl)-6-trifluoromethyl-phenyl]-N4-methyl-pyrido[2,3-d]pyrimidine-2,4-diamine trifluoroacetic acid salt as a light brown solid; LRMS for C₂₅H₃₀F₃N₇ (M+H)⁺ at m/z = 486.

### Example 115

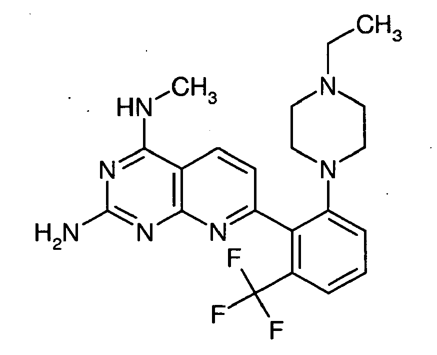

From 7-(2-fluoro-6-trifluoromethyl-phenyl)-N4-methyl-pyrido[2,3-d]pyrimidine-2,4-diamine and 1-ethylpiperazine: 7-[2-(4-Ethyl-piperazin-1-yl)-6-trifluoromethylphenyl]-N4-methyl-pyrido[2,3-d]pyrimidine-2,4-diamine trifluoroacetic acid salt as a light brown solid; LRMS for C₂₁H₂₄F₃N₇ (M+H)⁺ at m/z = 432.

### Example 116

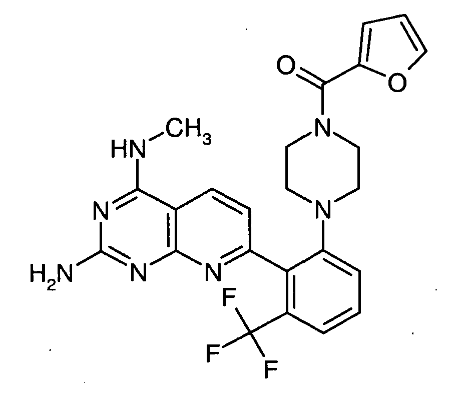

From 7-(2-fluoro-6-trifluoromethyl-phenyl)-N4-methyl-pyrido[2,3-d]pyrimidine-2,4-diamine and 1-(2-furoyl)piperazine: {4-[2-(2-Amino-4-methylamino-pyrido[2,3-d]pyrimidin-7-yl)-3-trifluoromethyl-phenyl]-piperazin-1-yl}-furan-2-yl-methanone trifluoroacetic acid salt as a light brown solid; LRMS for C₂₄H₂₂F₃N₇O₂ (M+H)⁺ at m/z = 498.

### Example 117

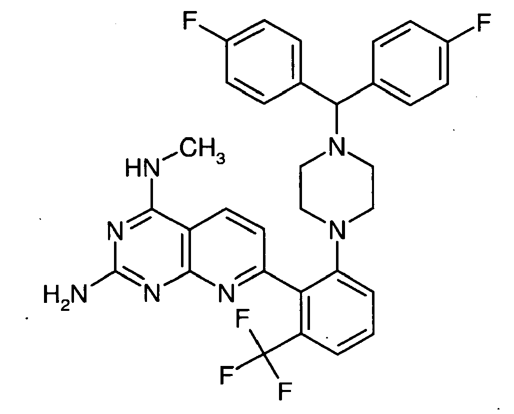

From 7-(2-fluoro-6-trifluoromethyl-phenyl)-N4-methyl-pyrido[2,3-d]pyrimidine-2,4-diamine and 1-(4,4'-difluorobenzhydryl)piperazine: 7-(2-(4-[Bis-(4-fluoro-phenyl)-methyl]-piperazin-1-yl}-6-trifluoromethyl-phenyl)-N4-methyl-pyrido[2,3-d]pyrimidine-2,4-diamine trifluoroacetic acid salt as a light brown solid; LRMS for C₃₂H₂₈F₅N₇ (M+H)⁺ at m/z = 606.

### Example 118

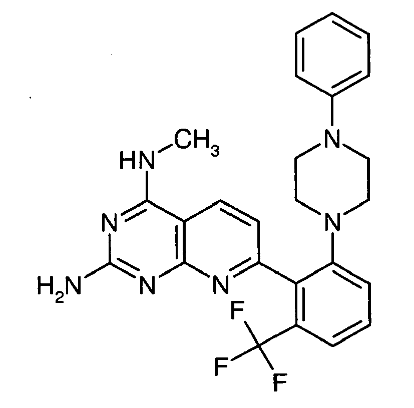

From 7-(2-fluoro-6-trifluoromethyl-phenyl)-N4-methyl-pyrido[2,3-d]pyrimidine-2,4-diamine and 1-phenylpiperazine: N4-Methyl-7-[2-(4-phenyl-piperazin-1-yl)-6-trifluoromethyl-phenyl]-pyrido[2,3-d]pyrimidine-2,4-diamine trifluoroacetic acid salt as a light brown solid; LRMS for C₂₅H₂₄F₃N₇ (M+H)⁺ at m/z = 480.

### Example 119

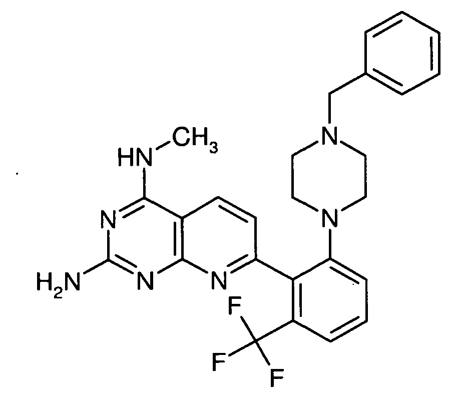

From 7-(2-fluoro-6-trifluoromethyl-phenyl)-N4-methyl-pyrido[2,3-d]pyrimidine-2,4-diamine and 1-benzylpiperazine: 7-[2-(4-Benzyl-piperazin-1-yl)-6-trifluoromethylphenyl]-N4-methyl-pyrido[2,3-d]pyrimidine-2,4-diamine trifluoroacetic acid salt as a light brown solid; LRMS for C₂₆H₂₆F₃N₇ (M+H)⁺ at m/z = 494.

### Example 120

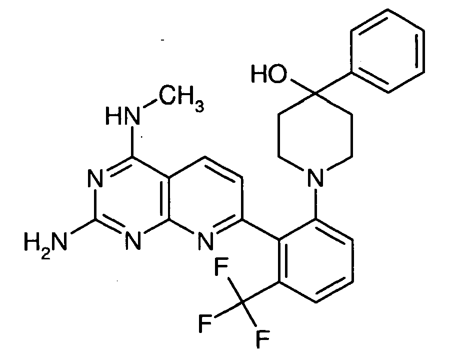

From 7-(2-fluoro-6-trifluoromethyl-phenyl)-N4-methyl-pyrido[2,3-d]pyrimidine-2,4-diamine and 4-hydroxy-4-phenylpiperidine: 1-[2-(2-Amino-4-methylamino-pyrido[2,3-d]pyrimidin-7-yl)-3-trifluoromethyl-phenyl]-4-phenyl-piperidin-4-ol trifluoroacetic acid salt as a light brown solid; LRMS for C₂₆H₂₅F₃N₆O (M+H)⁺ at m/z = 495.

### Example 121

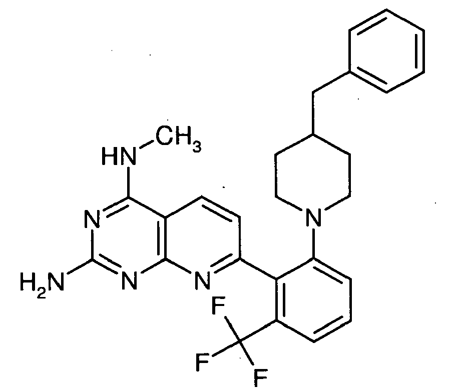

From 7-(2-fluoro-6-trifluoromethyl-phenyl)-N4-methyl-pyrido[2,3-d]pyrimidine-2,4-diamine and 4-benzylpiperidine: 7-[2-(4-Benzyl-piperidin-1-yl)-6-trifluoromethylphenyl]-N4-methyl-pyrido[2,3-d]pyrimidine-2,4-diamine trifluoroacetic acid salt as a light brown solid; LRMS for C₂₇H₂₇F₃N₆ (M+H)⁺ at m/z = 493.

### Example 122

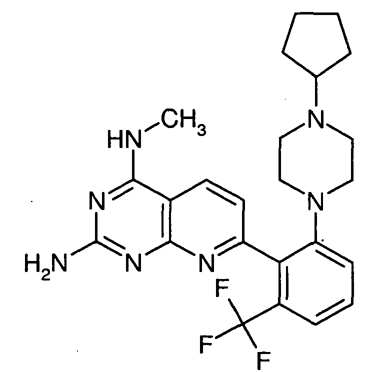

From 7-(2-fluoro-6-trifluoromethyl-phenyl)-N4-methyl-pyrido[2,3-d]pyrimidine-2,4-diamine and 1-cyclopentylpiperazine: 7-(2-(4-Cyclopentyl-piperazin-1-yl)-6-trifluoromethyl-phenyl]-N4-methyl-pyrido[2,3-d]pyrimidine-2,4-diamine trifluoroacetic acid salt as a light brown solid; LRMS for C₂₄H₂₈F₃N₇ (M+H)⁺ at m/z = 472.

### Example 123

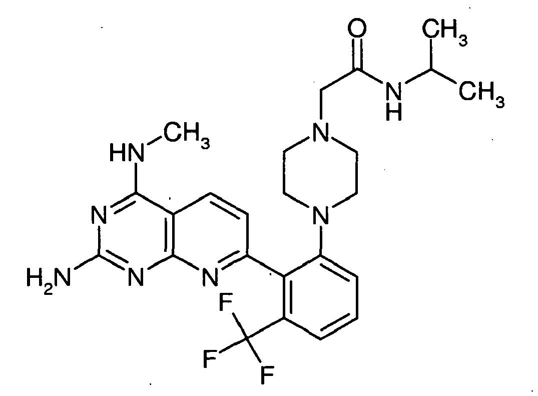

From 7-(2-ffuoro-6-trifluoromethyl-phenyl)-N4-methyl-pyrido[2,3-d]pyrimidine-2,4-diamine and N-isopropyl-1-piperazineacetamide: 2-{4-[2-(2-Amino-4-methylamino-pyrido[2,3-d]pyrimidin-7-yl)-3-trifluoromethyl-phenyl]-piperazin-1-yl}-N-isopropyl-acetamide trifluoroacetic acid salt as a light brown solid; LRMS for C₂₄H₂₉F₃N₈O (M+H)⁺ at m/z = 503.

### Example 124

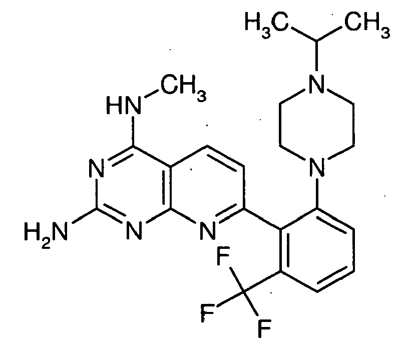

From 7-(2-fluoro-6-trifluoromethyl-phenyl)-N4-methyl-pyrido[2,3-d]pyrimidine-2,4-diamine and 1-isopropylpiperazine: 7-[2-(4-Isopropyl-piperazin-1-yl)-6-trifluoromethyl-phenyl]-N4-methyl-pyrido[2,3-d]pyrimidine-2,4-diamine trifluoroacetic acid salt as a light brown solid; LRMS for C₂₂H₂₆F₃N₇ (M+H)⁺ at m/z = 446.

### Example 125

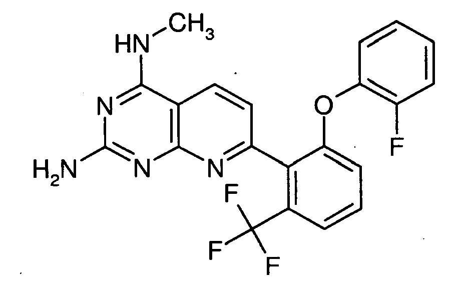

From 7-(2-fluoro-6-trifluoromethyl-phenyl)-N4-methyl-pyrido[2,3-d]pyrimidine-2,4-diamine, 2-fluorophenol and sodium hydride: 7-[2-(2-Fluoro-phenoxy)-6-trifluoromethyl-phenyl]-N4-methyl-pyrido[2,3-d]pyrimidine-2,4-diamine trifluoroacetic acid salt as a light brown solid; LRMS for C₂₁H₁₅F₄N₅O (M+H)⁺ at m/z = 430.

### Example 126

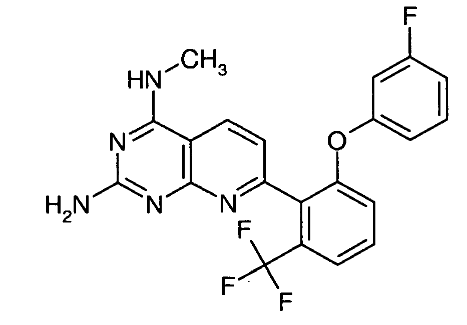

From 7-(2-fluoro-6-trifluoromethyl-phenyl)-N4-methyl-pyrido[2,3-d]pyrimidine-2,4-diamine, 3-fluorophenol and sodium hydride: 7-[2-(3-Fluoro-phenoxy)-6-trifluoromethyl-phenyl]-N4-methyl-pyrido[2,3-d]pyri midi ne-2,4-di ami ne trifluoroacetic acid salt as a light brown solid; LRMS for C₂₁H₁₅F₄N₅O (M+H)⁺ at m/z = 430.

### Example 127

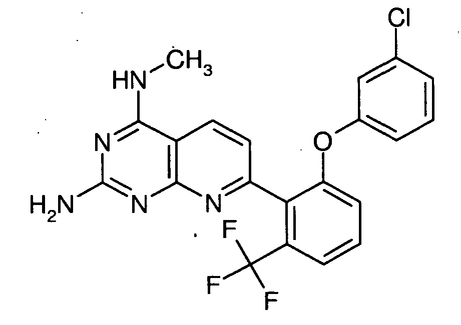

From 7-(2-fluoro-6-trifluoromethyl-phenyl)-N4-methyl-pyrido[2,3-d]pyrimidine-2,4-diamine, 3-chlorophenol and sodium hydride: 7-[2-(3-Chloro-phenoxy)-6-trifluoromethyl-phenyl]-N4-methyl-pyrido[2,3-d]pyrimidine-2,4-diamine trifluoroacetic acid salt as a light brown solid; LRMS for C₂₁H₁₅ClF₃N₅O (M+H)⁺ at m/z = 446.

### Example 128

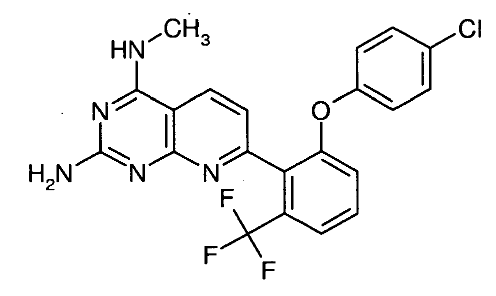

From 7-(2-fluoro-6-trifluoromethyl-phenyl)-N4-methyl-pyrido[2,3-d]pyrimidine-2,4-diamine, 4-chlorophenol and sodium hydride: 7-[2-(4-Chloro-phenoxy)-6-trifluoromethyl-phenyl]-N4-methyl-pyrido[2,3-d]pyrimidine-2,4-diamine trifluoroacetic acid salt as a light brown solid; LRMS for C₂₁H₁₅CIF₃N₅O (M+H)⁺ at m/z = 446.

### Example 129

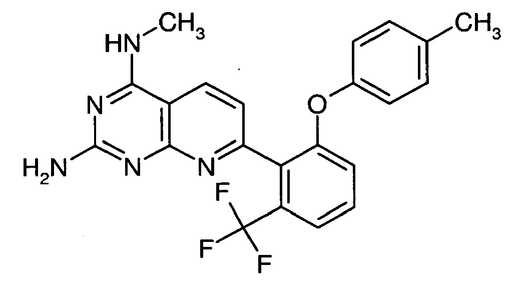

From 7-(2-fluoro-6-trifluoromethyl-phenyl)-N4-methyl-pyrido[2,3-d]pyrimidine-2,4-diamine, p-cresol and sodium hydride: N4-Methyl-7-(2-p-tolyloxy-6-trifluoromethylphenyl)-pyrido[2,3-d]pyrimidine-2,4-diamine trifluoroacetic acid salt as a light brown solid; LRMS for C₂₂H₁₈F₃N₅O (M+H)⁺ at m/z = 426.

### Example 130

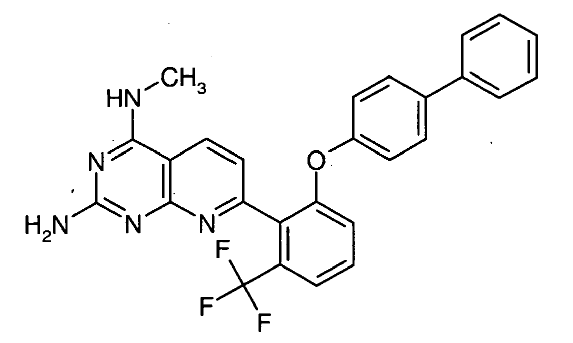

From 7-(2-fluoro-6-trifluoromethyl-phenyl)-N4-methyl-pyrido[2,3-d]pyrimidine-2,4-diamine, 4-phenylphenol and sodium hydride: 7-[2-(Biphenyl-4-yloxy)-6-trifluoromethyl-phenyl]-N4-methyl-pyrido[2,3-d]pyrimidine-2,4-diamine trifluoroacetic acid salt as a light brown solid; LRMS for C₂₇H₂₀F₃N₅O (M+H)⁺ at m/z = 488.

### Example 131

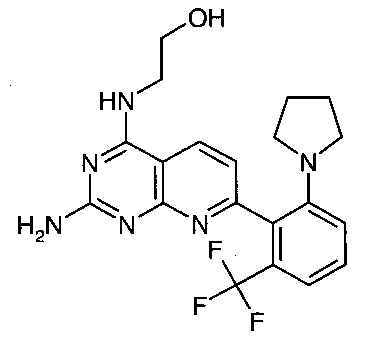

From 2-[2-Amino-7-(2-fluoro-6-trifluoromethyl-phenyl)-pyrido[2,3-d]pyrimidin-4-ylamino]-ethanol and pyrrolidine: 2-[2-Amino-7-(2-pyrrolidin-1-yl-6-trifluoromethylphenyl)-pyrido[2,3-d]pyrimidin-4-ylamino]-ethanol trifluoroacetic acid salt as a light brown solid; LRMS for C₂₀H₂₁FN₆O (M+H)⁺ at m/z = 419.

### Example 132

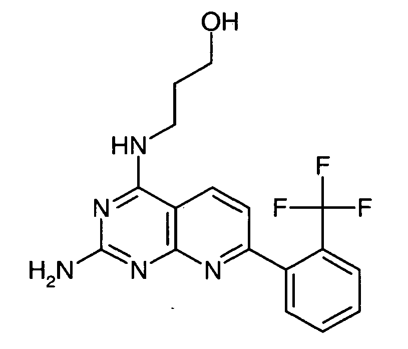

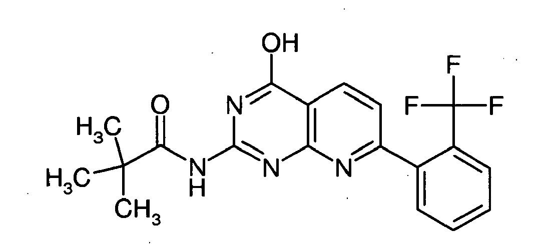

Using steps 1-3 of the four-step sequence of Example 17 but starting from 2'-(trifluoromethyl)acetophenone gave N-[7-(2-(trifluoromethyl)phenyl)-4-hydroxy-pyrido[2,3-d]pyrimidin-2-yl]-2,2-dimethyl-propionamide as a light brown solid. LR-MS for C₁₉H₁₇F₃N₄O₂ (M+H)⁺ at m/z = 391.

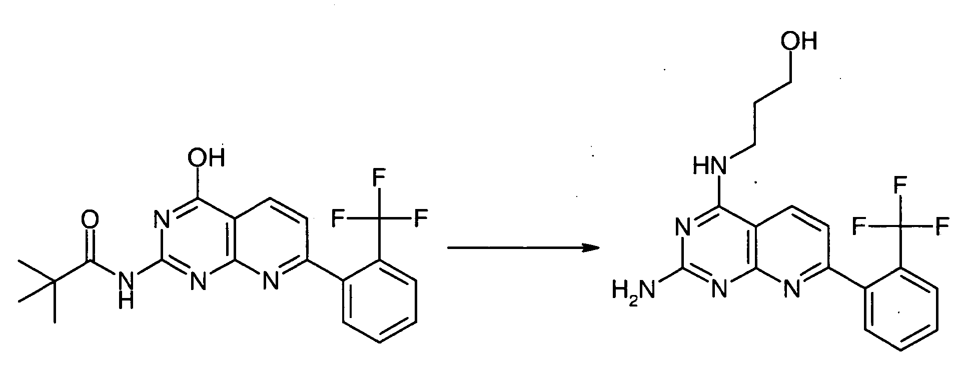

To a mixture of phosphorous oxychloride (26 ml, 280 mmol) and N-[7-(2-(trifluoromethyl)phenyl)-4-hydroxy-pyrido[2,3-d]pyrimidin-2-yl]-2,2-dimethyl-propionamide (2.5 g, 6.4 mmol) cooled in an ice bath was slowly added N,N-diisopropylethylamine (5.2 ml, 29.9 mmol). The reaction was then heated in a 35°C oil bath for 24 h. After cooling to room temperature, phosphorous oxychloride was distilled off *in vacuo* to afford N-[4-chloro-7-(6-(trifluoromethyl)phenyl)-pyrido[2,3-d]pyrimidin-2-yl]-2,2-dimethyl-propionamide as a brown oil. To a portion of the crude N-[4-chloro-7-(6-(trifluoromethyl)phenyl)-pyrido[2,3-d]pyrimidin-2-yl]-2,2-dimethyl-propionamide prepared above (750 mg, 1.84 mmol) in a sealed tube was added 2-propanol (60 ml), *N,N-*diisopropylethylamine (1.50 ml, 8.63 mmol) and 3-amino-1-propanol (270 mg, 3.60 mmol) at 0°C. The reaction was stirred at room temperature for three days. The reaction was concentrated *in vacuo* and purified by reversed phase HPLC to give 139 mg (16% yield) of 4-[2-amino-7-(2-trifluoromethyl-phenyl)-pyrido[2,3-d]pyrimidin-4-ylamino]-propan-1-ol trifluoroacetic acid salt as a white solid; LRMS for C₁₇H₁₆F₃N₅O (M+H)⁺ at m/z = 364.

In an analogous manner, the following compounds were also obtained:

### Example 133

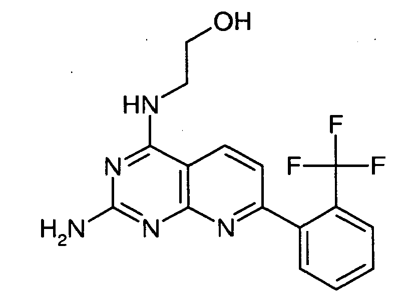

From N-[4-chloro-7-(6-(trifluoromethyl)phenyl)-pyrido[2,3-d]pyrimidin-2-yl]-2,2-dimethyl-propionamide and ethanolamine: 2-[2-Amino-7-(2-trifluoromethylphenyl)-pyrido[2,3-d]pyrimidin-4-ylamino]-ethanol as a light brown solid; LR-MS for C₁₆H₁₄F₃N₅O (M+H)⁺ at m/z = 350.

### Example 134

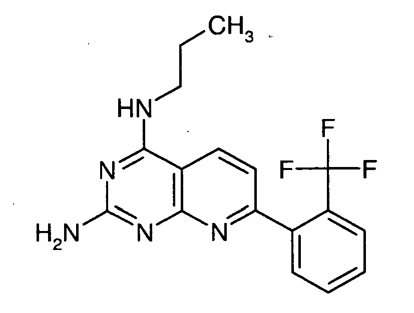

From N-[4-chloro-7-(6-(trifluoromethyl)phenyl)-pyrido[2,3-d]pyrimidin-2-yl]-2,2-dimethyl-propionamide and n-propylamine: N4-Propyl-7-(2-trifluoromethyl-phenyl)-pyrido[2,3-d]pyrimidine-2,4-diamine trifluoroacetic acid salt as a white solid; LRMS for C₁₇H₁₆F₃N₅ (M+H)⁺ at m/z = 348.

### Example 135

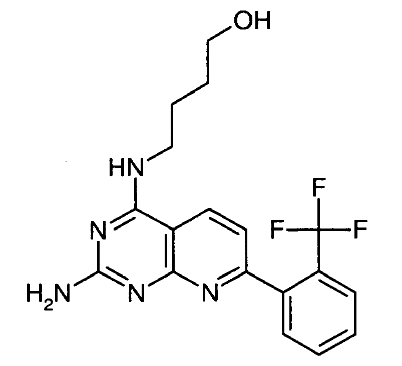

From N-[4-chloro-7-(6-(trifluoromethyl)phenyl)-pyrido[2,3-d]pyrimidin-2-yl]-2,2-dimethyl-propionamide and 4-amino-1-butanol: 4-[2-Amino-7-(2-trifluoromethylphenyl)-pyrido[2,3-d]pyrimidin-4-ylamino]-butan-1-ol trifluoroacetic acid salt as a white solid; LRMS for C₁₈H₁₈F₃N₅O (M+H)⁺ at m/z = 378.

### Example 136

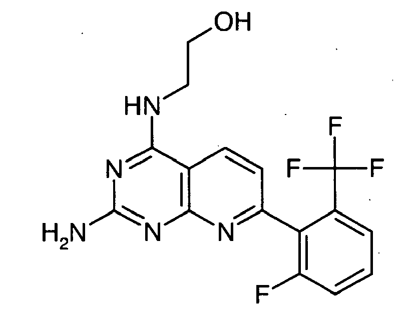

From N-[7-(2-fluoro-6-(trifluoromethyl)phenyl)-4-chloro-pyrido[2,3-d]pyrimidin-2-yl]-2,2-dimethyl-propionamide and ethanolamine: 2-[2-Amino-7-(2-fluoro-6-trifluoromethyl-phenyl)-pyrido[2,3-d]pyrimidin-4-ylamino]-ethanol as a light brown solid; LRMS for C₁₆H₁₃F₄N₅O (M+H)⁺ at m/z = 368.

### Example 137

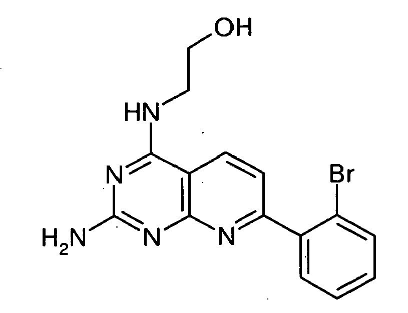

Analogously, substituting 2'-bromoacetophenone for 2'-(trifluoromethyl)acetophenone in the above procedures gave N-[7-(2-bromophenyl)-4-hydroxy-pyrido[2,3-d]pyrimidin-2-yl]-2,2-dimethyl-propionamide as a light brown solid. LR-MS for C₁₈H₁₇BrN₄O₂ (M+H)⁺ at m/z = 401. From the resulting N-[7-(2-bromophenyl)-4-chloro-pyrido[2,3-d]pyrimidin-2-yl]-2,2-dimethyl-propionamide and ethanolamine: 2-[2-Amino-7-(2-bromo-phenyl)-pyrido[2,3-d]pyrimidin-4-ylamino]-ethanol as a light brown solid; LRMS for C₁₅H₁₄BrN₅O (M+H)⁺ at m/z = 360.

### Example 138

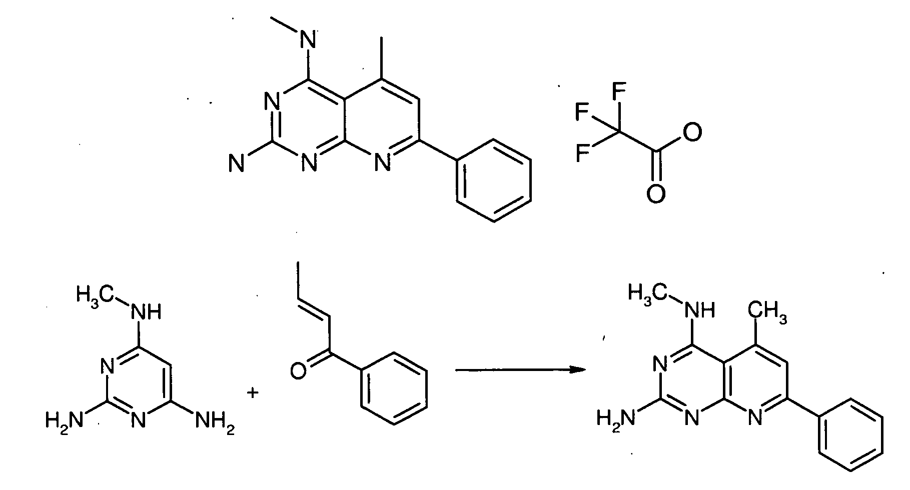

A mixture of N-Methyl-pyrimidine-2,4,6-triamine (40 mg, 0.29 mmole) and 1-Phenyl-but-2-en-1-one (53 mg, 0.36 mmole) in 1-methyl-2-pyrrolidinone (2 mL) was heated at reflux overnight. The reaction mixture was blown to dryness and the crude was purified by reversed phase HPLC to give 10 mg (9% yield) of 5,N*4*-Dimethyl-7-phenyl-pyrido[2,3-d]pyrimidine-2,4-diamine trifluoroacetate as a light brown solid; LR-MS for C₁₅H₁₅N₅ (M+H)⁺ at m/z = 266.

### Example 139

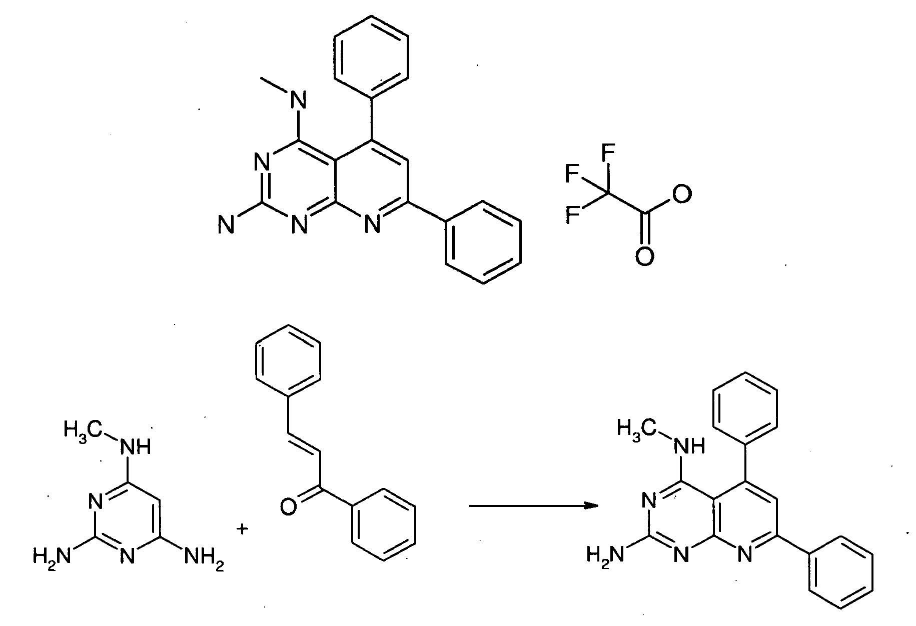

A mixture of N-Methyl-pyrimidine-2,4,6-triamine (40 mg, 0.29 mmole), 1,3-Diphenyl-propenone (75 mg, 0.36 mmole) in 1-methyl-2-pyrrolidinone (2 mL) was heated at reflux overnight. The reaction was blown to dryness and the crude was purified by reversed phase HPLC to give 15 mg (12% yield) of N*4*-Methyl-5,7-diphenyl-pyrido[2,3-d]pyrimidine-2,4-diamine trifluoroacetate as a light brown solid; LR-MS for C₂₀H₁₇N₅ (M+H)⁺ at m/z = 328.

### Example 140

### In vitro inhibition of PTP1 B

### Enzymes

Human PTP1 B (1-321) was cloned from a human cDNA library using conventional molecular biology techniques. The cDNA sequence was identical to the published human PTP1 B sequence (Accession number M33689). The protein was expressed and purified from E. coli as described by Barford D. et.al J. Mol Biol (1994) 239, 726-730.

### PTPase assays

The measurement of PTPase activity was carried out using one of two methods:
The first method for the measurement of PTP1 B inhibitory activity a tyrosine phosphorylated peptide based on the amino acid sequence of insulin receptor tyrosine autophosphorylation site 1146 (TRDI(pY)E) was used as substrate. The reaction conditions were as follows:
   PTP1 B (0.5-2nM) was incubated with compound for 15 min in buffer containing 37.5 mM Bis-Tris buffer pH 6.2, 140mMNaCl, 0.05% BSA and 2mM DTT. The reaction was started by the addition of 50µM substrate. After 20 min at room temperature (22-25°C), the reaction was stopped with KOH and the amount of free phosphate measured using Malachite Green as previously described (Harder et al. 1994 Biochem J. 298; 395).
The second method was used for the measurement of general PTPase inhibitory activity across a panel of PTPases the substrate (6,8-difluoro-4-methylumbelliferyl phosphate (DiFMUP; from Molecular Probes) was used at the Km for each enzyme. The buffer conditions were identical as in the Malachite Green assay. The reaction was stopped with KOH. In this case the dephosphoryated product becomes fluorescent and the fluorescense read (Excitiation:360mM/Emmission: 460nM).

For kinetic experiments, the same buffer conditions were used except that the reaction was started using enzyme and the reaction stopped after 10 minutes.

The IC50 values (in µM) for the PTP1 B inhibitory activity of the compounds in the present application are in the range of about 0.14µM to about 80µM. The following Table lists IC50 results for several of the above exemplified compounds:

| **Example** | **IC50 (µM)** |
|---|---|
| 1 | 1.66 |
| 3 | 0.51 |
| 9 | 1.41 |
| 13 | 3.57 |
| 14 | 2.56 |
| 27 | 4.22 |
| 28 | 1.15 |
| 56 | 0.25 |
| 59 | 10.80 |
| 80 | 0.17 |
| 92 | 2.33 |
| 107 | 0.80 |
| 116 | 0.30 |
| 121 | 2.05 |
| 139 | 52.44 |

### Example 141

### Glucose Uptake Assay

The day before the assay the SKMC media was changed to high glucose DMEM , 25mM Hepes, pH 7.0 and 2% Charcoal/dextran treated FBS for 19 hours.

On the morning of the assay, cells were starved for max. 2 hours in low glucose (5.5mM glucose) DMEM, 25 mM Hepes, pH 7.0 and 0.5% BSA. The starvation medium was removed and replaced with test medium (150mMNaCl, 25mM Hepes, pH 7.0) containing either 1% DMSO, or test compound diluted in DMSO or Porcine Insulin to a final concentrations of 1, 0.1, 0.05, 0.01 and 0.01 µM. Each assay point was performed in triplicate. The cells were incubated for 45 min at 37°C. 10µM Cytochalasin B (CB) was added to appropriate wells to stop the active glucose transport (i.e., GLUT 1 & 4). At this point 2-Deoxy-D(U-¹⁵C)glucose (Amersham, Code CFB195, 200uCi/ml) was added to all wells to a final concentration of 0.8 µCi/ml. The cells were incubated for an additional 45 minutes at 37°C in an incubator. Cells were then very gently washed for three times in PBS (RT). The cells were then lysed with the addition of 0.05% NaOH solution for 20 min at RT. The lysate was transferred to a scintillation vial containing 5 ml of scintillation fluid and counted in a Beckman LS6500 Scintillation counter. Analysis of results: The counts obtained with CB (passive glucose transport values) were subtracted from every value obtained with PI (or compounds) in order to evaluate only active glucose transport. Fold increase was calculated by dividing values in the presence of PI (or compounds) by the value obtained in the presence of DMSO (control). Compounds were considered to be active when they increase glucose uptake at least 25% of the Porcine Insulin response at 0.05 µM.

*In vivo* inhibition of PTP1 B: The anti-diabetic effect of compounds can be confirmed in well established rodent *in vivo* models of type 2 diabetes and obesity as set forth in the following procedures:

### Example 142

### Mouse Models:

***Diet Induced Obese (DIO) Mouse Model:*** A majority of male C57BL/6J mice fed a diet consisting of 35.5% fat for 3 months develop obesity, hyperinsulinemia and hyperglycemia. DIO mice are probably a better model for human type-2 diabetes than are genetic mutations with multiple neuroendocrine abnormalities. Furthermore, the DIO mice probably develop type-2 diabetes in a manner similar to most cases of type-2 diabetes in humans, e.g. only those predisposed individuals who become obese after access to a diabetogenic diet.

***B6.C-m Lep^{db}*/*++*/*****J:*** Mice homozygous for the diabetes spontaneous mutation *(Lepr^{db})* become identifiably obese around 3 to 4 weeks of age. Elevations of plasma insulin begin at 10 to 14 days and of blood sugar at 4 to 8 weeks. Homozygous mutant mice are polyphagic, polydipsic, and polyuric. The course of the disease is markedly influenced by genetic background. A number of features are observed on the C57BLKS background, including an uncontrolled rise in blood sugar, severe depletion of the insulin-producing beta-cells of the pancreatic islets, and death by 10 months of age. Exogenous insulin fails to control blood glucose levels and gluconeogenic enzyme activity increases. Peripheral neuropathy and myocardial disease are seen in C57BLKS *Lepr^{db}* homozygotes.

***B6.V-Lep^{ob}*/*****J:*** Mice homozygous for the obese spontaneous mutation, (*Lep^{ob}* commonly referred to as *ob* or *ob*/*ob*), are first recognizable at about 4 weeks of age. Homozygous mutant mice increase in weight rapidly and may reach three times the normal weight of wildtype controls. In addition to obesity, mutant mice exhibit hyperphagia, a diabetes-like syndrome of hyperglycemia, glucose intolerance, elevated plasma insulin, subfertility, impaired wound healing, and an increase in hormone production from both pituitary and adrenal glands. They are also hypometabolic and hypothermic. The obesity is characterized by an increase in both number and size of adipocytes. Although hyperphagia contributes to the obesity, homozygotes gain excess weight and deposit excess fat even when restricted to a diet sufficient for normal weight maintenance in lean mice. Hyperinsulinemia does not develop until after the increase body weight and is probably the result of it. Homozygotes do have an abnormally low threshold for stimulation of pancreatic islet insulin secretion even in very young preobese animals. Female homozygotes exhibit decreased uterine and ovarian weights, decreased ovarian hormone production and hypercytolipidemia in follicular granulosa and endometrial epithelial tissue layers (Garris et al., 2004).

### Mouse Criteria:

**DIO Mouse Model:** Mice used in these studies are at least 18 weeks of age and maintained on a high fat diet (BioServ F3282) for at least 12 weeks, The mice are weighed on the day prior to the study and sorted into treatment groups. Because of the variability in body weights, the DIO mice having the most extreme (i.e. highest or lowest) body weights are excluded.

**B6.C-m Lep^{db}/++/J:** Mice used in these studies are at least 9 weeks of age and maintained on Purina Lab Diet 5008 starting at 6 weeks of age. Two to three days prior to the study blood glucose levels of the mice are determined following a two hour fast. The mice are sorted into treatment groups. Because of the variability in blood glucose levels, the mice having the most extreme (i.e. highest or lowest) blood glucose levels are excluded with the goal of achieving an average blood glucose level between 160-190mg/dl.

**B6.V-Lep^{ob}/J:** Mice used in these studies are at least 7 weeks of age and maintained on Purina Lab Diet 5001. Two to three days prior to the study blood glucose levels of the mice are determined following a two hour fast. The mice are sorted into treatment groups. Because of the variability in blood glucose levels, the mice having the most extreme (i.e. highest or lowest) blood glucose levels are excluded. In some instances mice are sorted based on body weights, the ob/ob mice having the most extreme (i.e. highest or lowest) body weights were excluded.

### Experimental Parameters:

***Oral Glucose Tolerance Test (OGTT):*** Mice are placed into individual cages and fasted for 15 hours. After 15 hours the mice are treated orally by gavage with vehicle or compound using a dose volume of 5ml/kg. An oral glucose challenge (1-2g/kg) is administered four hours following treatment. Blood is collected from the tail vein into a 20ul heparinized microhematocrit tube immediately prior to dosing with vehicle or compound, immediately prior to the OGTT and 0.5, 1, 1.5, 2 and sometimes up to 4 hours following the OGTT. The blood is transferred immediately to a microfuge tube. Blood glucose is measured with the YSI 2700 Select Glucose Analyzer. In some instances mice are fasted for only 2 hours prior to dosing with vehicle or compound and the OGTT is administered 4 hours post dose.

***Acute Efficacy Study:*** Mice are placed into individual cages and fasted for 2 hours. After 2 hours the mice are treated orally by gavage with vehicle or compound using a dose volume of 5ml/kg. Blood is collected from the tail vein into a 20 ul heparinized microhematocrit tube immediately prior to dosing with vehicle or compound and 2, 4, 6 and 8 hours following treatment. The blood is transferred immediately to a microfuge tube. Blood glucose is measured with the YSI 2700 Select Glucose Analyzer

Mice that have type 2 diabetes are generated by maintaining them on a high fat diet for 4-6 months (Diabetes vol. 37 Sept 1988). Male C57BL/6J mice (age 3 - 4 weeks) are placed on high fat diet for 4-6 months. At this time they are hyperglycemic and hyperinsulinemic and weighed 40-50 g. DIO mice (n=10) are weighed and fasted for a two hour period prior to oral treatment. Immediately prior to dosing a pre-dose blood glucose reading is taken by snipping off a portion of the tail and collecting blood from the tail vein. Mice are treated either with a single dose of compound (acute) or once a day for 5 days (sub-chronic). For the acute studies, glucose is generally measured at 2h, 4h, 6h, 8h post treatment. Compounds are considered active if the compounds demonstrated AUC (Area under the curve) show a statistically significant (p ≤ 0.05) glucose lowering (>15%) compared to the vehicle treated animals.

For sub-chronic (5 day) studies mice are dosed once a day by gavage as described above. On day five, glucose is measured prior to dosing (0 time) and 2 hours after dosing. Insulin and triglycerides are measured at 2 hour post dose. Compounds are considered active if the compounds demonstrated AUC (Area under the curve) show a statistically significant (p ≤ 0.05) glucose, insulin and triglyceride lowering compared to the vehicle treated animals.

### Example A

Film coated tablets containing the following ingredients can be manufactured in a conventional manner:

| Ingredients | | |
|---|---|---|
| | *Per tablet* | |
| **Kernel:** | | |
| Compound of formula (I) | 10.0 mg | 200.0 mg |
| Microcrystalline cellulose | 23.5 mg | 43.5 mg |
| Lactose hydrous | 60.0 mg | 70.0 mg |
| Polyvinylpyrrolidone K30 | 12.5 mg | 15.0 mg |
| Sodium starch glycolate | 12.5 mg | 17.0 mg |
| Magnesium stearate | 1.5 mg | 4.5 mg |
| (Kernel Weight) | 120.0 mg | 350.0 mg |
| **Film Coat:** | | |
| Hydroxypropyl methyl cellulose | 3.5 mg | 7.0 mg |
| Polyethylene glycol 6000 | 0.8 mg | 1.6 mg |
| Talc | 1.3 mg | 2.6 mg |
| Iron oxide (yellow) | 0.8 mg | 1.6 mg |
| Titanium dioxide | 0.8 mg | 1.6 mg |

The active ingredient is sieved and mixed with microcristalline cellulose and the mixture is granulated with a solution of polyvinylpyrrolidone in water. The granulate is mixed with sodium starch glycolate and magesiumstearate and compressed to yield kernels of 120 or 350 mg respectively. The kernels are lacquered with an aqueous solution / suspension of the above mentioned film coat.

### Example B

Capsules containing the following ingredients can be manufactured in a conventional manner:

| Ingredients | Per capsule |
|---|---|
| Compound of formula (I) | 25.0 mg |
| Lactose | 150.0 mg |
| Maize starch | 20.0 mg |
| Talc | 5.0 mg |

The components are sieved and mixed and filled into capsules of size 2.

### Example C

Injection solutions can have the following composition:

| | |
|---|---|
| Compound of formula (I) | 3.0 mg |
| Polyethylene glycol 400 | 150.0 mg |
| Acetic Acid | q.s. ad pH 5.0 |
| Water for injection solutions | ad 1.0 ml |

The active ingredient is dissolved in a mixture of polyethylene glycol 400 and water for injection (part). The pH is adjusted to 5.0 by acetic acid. The volume is adjusted to 1.0 ml by addition of the residual amount of water. The solution is filtered, filled into vials using an appropriate overage and sterilized.

### Example D

Soft gelatin capsules containing the following ingredients can be manufactured in a conventional manner:

| **Capsule contents** | |
|---|---|
| Compound of formula (I) | 5.0 mg |
| Yellow wax | 8.0 mg |
| Hydrogenated Soya bean oil | 8.0 mg |
| Partially hydrogenated plant oils | 34.0 mg |
| Soya bean oil | 110.0 mg |
| Weight of capsule contents | 165.0 mg |
| | |

| **Gelatin capsule** | |
|---|---|
| Gelatin | 75.0 mg |
| Glycerol 85% | 32.0 mg |
| Karion 83 | 8.0 mg (dry matter) |
| Titanium dioxide | 0.4 mg |
| Iron oxide yellow | 1.1 mg |

The active ingredient is dissolved in a warm melting of the other ingredients and the mixture is filled into soft gelatin capsules of appropriate size. The filled soft gelatin capsules are treated according to the usual procedures.

### Example E

Sachets containing the following ingredients can be manufactured in a conventional manner:

| | |
|---|---|
| Compound of formula (I) | 50.0 mg |
| Lactose, fine powder | 1015.0 mg |
| Microcrystalline cellulose (AVICEL PH 102) | 1400.0 mg |
| Sodium carboxymethyl cellulose | 14.0 mg |
| Polyvinylpyrrolidone K 30 | 10.0 mg |
| Magnesium stearate | 10.0 mg |
| Flavoring additives | 1.0 mg |

The active ingredient is mixed with lactose, microcristalline cellulose and sodium carboxymethyl cellulose and granulated with a mixture of polyvinylpyrrolidone in water. The granulate is mixed with magnesium stearate and the flavouring additives and filled into sachets.

## Claims

1. Compounds of the formula (I): 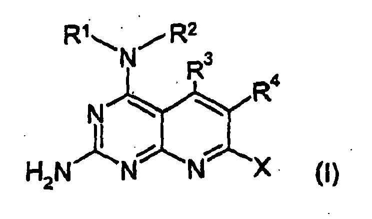
wherein X is a group X-1 of the formula: 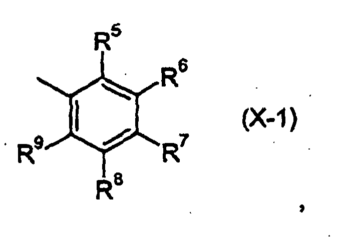
or X is a group X-2 of the formula: 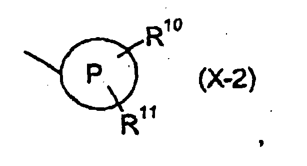
or X is a group X-3 of the formula: 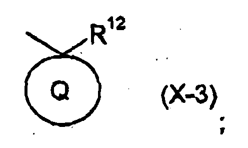
R¹ and R² are independently selected from the group consisting of hydrogen, lower alkyl, methoxy lower alkyl and hydroxy lower alkyl, except that R¹ and R² may not both be hydrogen;
R³ is hydrogen, lower alkyl or phenyl;
R⁴ is hydrogen, lower alkyl, lower alkylsulfonyl, phenyl, carboxy or together with R⁵ forms a 5-7 membered carbocyclic ring;
R⁵ when not fused in a ring with R⁴ is hydrogen, lower alkyl, substituted lower alkyl, lower alkoxy, substituted lower alkoxy, hydroxy, carboxy, halogen, lower alkylthio, lower alkylsulfinyl, lower alkylsulfonyl, aminosulfonyl, cyano, nitro, lower alkanoyl, aryl, aroyl, aryloxy, arylthio, perfluoro lower alkyl, lower alkylamino, lower alkanoylamino, sulfonylamino, cycloalkyl, cycloalkoxy, heterocyclyl, heterocyclyloxy, heterocyclylcarbonyl, heteroaryl, or together with R⁶ forms a second fused 5 or 6 membered aromatic ring;
R⁶ when not fused in a ring with R⁵ is hydrogen, lower alkyl, substituted lower alkyl, lower alkoxy, substituted lower alkoxy, hydroxy, halogen, lower alkylthio, lower alkylsulfinyl, lower alkylsulfonyl, aminosulfonyl, cyano, nitro, lower alkanoyl, aryl, aroyl, aryloxy, lower alkylamino, lower alkanoylamino, sulfonylamino, cycloalkyl, heterocyclyl, heterocyclyloxy or heterocyclylcarbonyl;
R⁷ is hydrogen, lower alkyl, lower alkoxy, alkoxy lower alkyl, alkoxy lower alkoxy, hydroxy lower alkyl, hydroxy, hydroxyalkoxy, halogen, lower alkylthio, lower alkylsulfinyl, lower alkylsulfonyl, perfluoro lower alkyl, lower alkanoyl, aroyl or lower alkanoylamino;
R⁸ and R⁹ are each independently selected from the group consisting of hydrogen, lower alkyl, substituted lower alkyl, lower alkoxy, substituted lower alkoxy, hydroxy, halogen, lower alkylthio, lower alkylsulfinyl, lower alkylsulfonyl, aminosulfonyl, cyano, nitro, lower alkanoyl, aryl, aroyl, aryloxy, lower alkylamino, lower alkanoylamino, sulfonylamino, cycloalkyl, heterocyclyl, heterocyclyloxy and heterocyclylcarbonyl;
P is a 5 or 6 membered heteroaromatic ring containing from 1 to 2 hetero atoms selected from the group consisting of oxygen, sulfur and nitrogen;
R¹⁰ and R¹¹ are each independently selected from the group consisting of hydrogen, lower alkyl, lower alkoxy, perfluoro lower alkyl, halogen, aryl lower alkyl, aryl and aryl lower alkoxy;
Q is a 3-6 membered cycloalkyl ring; and
R¹² is hydrogen or aryl;
or the pharmaceutically acceptable salts or esters thereof,
wherein the term "lower alkyl", alone or in combination, means a straight-chain or branched-chain alkyl group containing a maximum of six carbon atoms;
wherein the term "lower alkoxy" means a lower alkyl group as defined above) bonded through an oxygen atom:
wherein the term "lower alkylthio" means a lower alkyl group bonded through a divalent sulfur atom: and
wherein the term "lower alkanoyl" means a lower alkyl group bonded to the rest of the molecule via a carbonyl group.

2. Compounds of claim 1 of the formula (Ia): 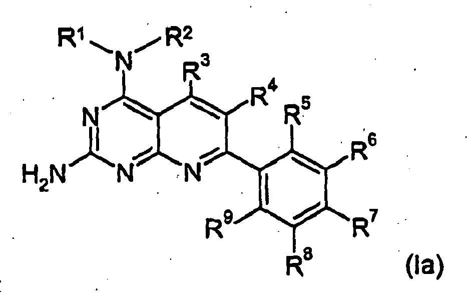
wherein R³ is hydrogen and R⁴ is hydrogen, lower alkyl, lower alkylsulfonyl, phenyl or carboxy, and R¹, R², R⁵, R⁶, R⁷, R⁸ and R⁹ are as defined in claim 1.

3. Compounds of claim 2 wherein R⁶, R⁷ and R⁸ are each independently hydrogen, halogen, lower alkyl, lower alkoxy, hydroxy, hydroxy lower alkyl, lower alkylthio, lower alkyl sulfinyl, lower alkyl sulfonyl or perfluoro lower alkyl.

4. Compounds of claim 3 wherein R⁷ is hydrogen or flourine.

5. Compounds of claim 4 wherein one of R⁶ and R⁸ is hydrogen or flourine.

6. Compounds of claim 4 wherein one of R⁶ and R⁸ is hydrogen or fluorine and the other is halogen, tower alkyl, lower alkoxy, hydroxy, hydroxy lower alkyl, lower alkylthio, lower alkyl sulfinyl, lower alkyl sulfonyl or perfluoro lower alkyl.

7. Compounds of claim 5 wherein R⁶, R⁷ and R⁸ are hydrogen.

8. Compounds of any of claims 2 to 7 wherein R⁵ and R⁹ are each independently selected from the group consisting of hydrogen, halogen, lower alkyl, lower alkoxy, alkoxy lower alkoxy, nitro, hydroxy, hydroxy lower alkoxy, hydroxy lower alkyl, lower alkylthio, lower alkylamino, lower alkyl sulfonyl, lower alkyl sulfinyl, perfluoro lower alkyl, cycloalkyl, cycloalkoxy, aryl, heteroaryl, aryloxy, arylthio and heterocyclyl.

9. Compounds of any of claims 5 to 7 wherein R⁵ and R⁹ are each independently selected from the group consisting of chlorine, fluorine, trifluoromethyl, C1-4 alkyl, C1-3 alkylthio, C1-3 alkylsulfonyl, C1-3 alkoxy, phenoxy, phenoxy mono-substituted with fluorine, chlorine or oxygen, and C1-3 alkoxy substituted with hydroxy, methoxy or ethoxy.

10. Compounds of claim 2 wherein R¹ or R² is hydrogen.

11. Compound of claim 10 wherein the R¹ or R² which is substituted is substituted with C1-4 alkyl or hydroxy C1-3 alkyl.

12. Compounds of claim 10 wherein R⁶, R⁷ and R⁸ are each independently hydrogen, halogen, lower alkyl, lower alkoxy, hydroxy, hydroxy lower alkyl, lower alkylthio, lower alkyl sulfinyl, lower alkyl sulfonyl or perfluoro lower alkyl.

13. Compounds of claim 10 wherein R⁷ is hydrogen or flourine.

14. Compounds of claim 13 wherein one of R⁶ and R⁸ is hydrogen or flourine.

15. Compounds of claim 13 wherein one of R⁶ and R⁸ is hydrogen or fluorine and the other is halogen, lower alkyl, lower alkoxy, hydroxy, hydroxy lower alkyl, lower alkylthio, lower alkyl sulfinyl, lower alkyl sulfonyl or perfluoro lower alkyl.

16. Compounds of claim 14 wherein R⁶, R⁷ and R⁸ are hydrogen.

17. Compounds according to claim 16 wherein the R¹ or R² which is substituted is substituted with C1-4 alkyl or hydroxy C1-3 alkyl.

18. Compounds of any of claims 10 to 15 wherein R⁵ and R⁹ are each independently selected from the group consisting of hydrogen, halogen, lower alkyl, lower alkoxy, alkoxy lower alkoxy, nitro, hydroxy, hydroxy lower alkoxy, hydroxy lower alkyl, lower alkylthio, lower alkylamino, lower alkyl sulfonyl, lower alkyl sulfinyl, perfluoro lower alkyl, cycloalkyl, cycloalkoxy, aryl, heteroaryl, aryloxy, arylthio and heterocyclyl.

19. Compounds of claim 18 wherein the R¹ or R² which is substituted is substituted with C1-4 alkyl or hydroxy C1-3 alkyl.

20. Compounds of claim 16 wherein R⁵ and R⁹ are each independently selected from the group consisting of chlorine, fluorine, trifluoromethyl, C1-4 alkyl, C1-3 alkylthio, C1-3 alkylsulfonyl, C1-3 alkoxy, phenoxy, phenoxy mono-substituted with fluorine, chlorine or oxygen, and C1-3 alkoxy substituted with hydroxy, methoxy or ethoxy.

21. Compounds of claim 20 wherein the R¹ or R² which is substituted is substituted with C1-4 alkyl or hydroxy C1-3 alkyl.

22. Compounds of claim 1 wherein R⁴ and R⁵ form a 5-7 membered carbocyclic ring.

23. Compounds of claim 22 wherein R¹ or R² is hydrogen.

24. Compounds of claim 23 wherein R⁷ is hydrogen or flourine.

25. Compounds of claim 24 wherein one of R⁶ and R⁸ is hydrogen.

26. Compounds of claim 24 wherein one of R⁶ and R⁸ is hydrogen or fluorine and the other is halogen, lower alkyl, lower alkoxy, hydroxy, hydroxy lower alkyl, lower alkylthio, lower alkyl sulfinyl, lower alkyl sulfonyl or perfluoro lower alkyl.

27. Compounds of claim 25 wherein R⁶, R⁷ and R⁸ are hydrogen.

28. Compounds according to claim 27 wherein the R¹ or R² which is substituted is substituted with C1-4 alkyl or hydroxy C1-3 alkyl.

29. Compounds of claim 23 wherein R⁵ and R⁹ are each independently hydrogen, halogen, lower alkyl, lower alkoxy, alkoxy lower alkoxy, nitro, hydroxy, hydroxy lower alkoxy, hydroxy lower alkyl, lower alkylthio, lower alkyl sulfinyl, lower alkyl sulfonyl, and perfluoro lower alkyl.

30. Compounds of claim 23 or 25 wherein the R¹ or R² which is substituted is substituted with C1-4 alkyl or hydroxy C1-3 alkyl.

31. Compounds of claim 1 of the formula (Ib): 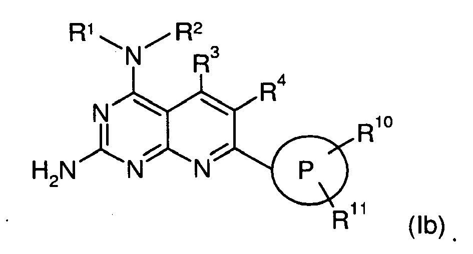
wherein R¹, R², R³, R⁴, P, R¹⁰ and R¹¹ are as defined in claim 1.

32. Compounds of claim 31 wherein R¹ or R² is hydrogen.

33. Compounds of claim 32 wherein R3 is hydrogen and R⁴ is hydrogen, lower alkyl, lower alkylsulfonyl, phenyl or carboxy.

34. Compounds of claim 32 or 33 wherein R¹⁰ and R¹¹ are each independently lower alkyl, lower alkoxy, perfluoro lower alkyl or halogen.

35. Compounds of claim 34 wherein the R¹ or R² which is substituted is substituted with C1-4 alkyl or hydroxy C1-3 alkyl.

36. Compounds of claim 34 wherein the R¹ or R² which is substituted is substituted with C1-4 alkyl or hydroxy C1-3 alkyl.

37. Compounds of claim 1 of the formula (Ic): 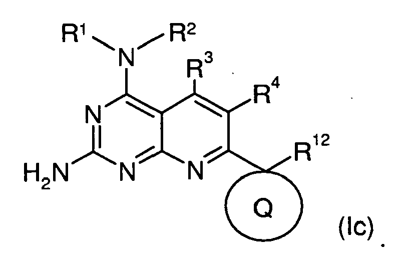
wherein R¹, R², R³, R⁴, Q and R¹² are as defined in claim 1.

38. Compounds of claim 37 wherein R¹ or R² is hydrogen.

39. Compounds of claim 38 wherein R³ is hydrogen and R⁴ is hydrogen, lower alkyl, lower alkylsulfonyl, phenyl or carboxy.

40. Compounds of claim 38 wherein R¹² is unsubstituted or substituted phenyl.

41. Compounds of claim 38 wherein R¹² is mono-substituted phenyl.

42. Compounds of claim 38 or 40 wherein the R¹ or R² which is substituted is substituted with C1-4 alkyl or hydroxy C1-3 alkyl.

43. Compounds of any of claims 1 to 42, selected from the group consisting of
N4-Methyl-7-o-tolyl-pyrido[2,3-d]pyrimidine-2,4-diamine,
N4-Methyl-7-(2-trifluoromethyl-phenyl)-pyrido[2,3-d]pyrimidine-2,4-diamine,
7-(2,6-Dichloro-phenyl)-N4-methyl-pyrido[2,3-d]pyrimidine-2,4-diamine,
7-(2-Chloro-phenyl)-N4-methyl-pyrido[2,3-d]pyrimidine-2,4-diamine,
7-(2,6-Difluoro-phenyl)-N4-methyl-pyrido[2,3-d]pyrimidine-2,4-diamine,
7-tert-Butyl-N4-methyl-pyrido(2,3-d]pyrimidine-2,4-diamine,
2-chloro-6-fluorophenyl-N4-methyl-pyrido[2,3-d]pyrimidine-2,4-diamine,
7-(2-Fluoro-6-trifluoromethyl-phenyl)-N4-methyl-pyrido[2,3-d]pyri midi ne-2,4-diamine,
7-Cyclohexyl-N4-methyl-pyrido[2,3-d]pyrimidine-2,4-diamine,
7-(2-Methoxy-phenyl)-N4-methyl-pyrido[2,3-d]pyrimidine-2,4-diamine,
N4-Methyl-7-(2-nitro-phenyl)-pyrido[2,3-d]pyri midi ne-2,4-diami ne,
6,N4-Dimethyl-7-(2-trifluoromethyl-phenyl)-pyrido[2,3-d]pyrimidine-2,4-diamine,
N4-Methyl-7-thiophen-2-yl-pyrido[2,3-d]pyrimidine-2,4-diamine,
N4-Methyl-6,7-diphenyl-pyrido[2,3-d]pyrimidine-2,4-diamine,
7-(2,6-Dimethyl-phenyl)-N4-methyl-pyrido[2,3-d]pyrimidine-2,4-diamine,
7-(2,6-dimethyl-phenyl)-N4-ethyl-pyrido[2,3-d]pyrimidine-2,4-diamine,
7-(2-fluoro-6-trifluoromethyl-phenyl)-N4-methyl-pyrido[2,3-d]pyrimidine-2,4-diamine,
7-(2-Chloro-6-fluoro-phenyl)-N4-methyl-pyrido[2,3-d]pyri midi ne-2,4-diami ne,
N4-Methyl-7-(2,4,6-tri methyl-phenyl)-pyrido[2,3-d]pyri midi ne-2,4-diami ne,
7-(2-Bromo-phenyl)-N4-methyl-pyrido[2,3-d]pyrimidine-2,4-diamine,
7-(2-Benzyloxy-phenyl)-N4-methyl-pyrido[2,3-d]pyrimidine-2,4-diamine,
7-(2-Ethoxy-phenyl)-N4-methyl-pyrido[2,3-d]pyrimidine-2,4-diamine trifluoroacetic acid salt,
N4-Methyl-7-(2-p-tolyloxy-phenyl)-pyrido[2,3-d]pyri midi ne-2,4-diami ne trifluoroacetic acid salt,
N4-Methyl-6-propyl-7-(2-trifluoromethyl-phenyl)-pyrido[2,3-d]pyrimidine-2,4-diamine,
N4-Methyl-7-(2,4-dimethyl-phenyl)-pyrido[2,3-d]pyri midi ne-2,4-diami ne,
7-(2,6-Dichloro-4-trifluoromethyl-phenyl)-N4-methyl-pyrido[2,3-d]pyrimidine-2,4-diamine trifluoroacetic acid salt,
N8-Methyl-5,6-di hydro-benzo[h]pyri mido[4,5-b]qui noli ne-8,10-diami ne,
N4-Methyl-7-naphthalen-1-yl-pyrido[2,3-d]pyrimidine-2,4-diamine,
7-(2-lodo-phenyl)-N4-methyl-pyrido[2,3-d]pyrimidine-2,4-diamine trifluoroacetic acid salt,
7-(2-Fluoro-6-methoxy-phenyl)-N4-methyl-pyrido[2,3-d]pyrimidine-2,4-diamine trifluoroacetic acid salt,
7-(2-Ethoxy-6-fluoro-phenyl)-N4-methyl-pyrido[2,3-d]pyrimidine-2,4-diamine trifluoroacetic acid salt,
7-(2-Fluoro-6-isopropoxy-phenyl)-N4-methyl-pyrido[2,3-d]pyrimidine-2,4-diamine trifluoroacetic acid salt,
7-(2-Fluoro-6-propoxy-phenyl)-N4-methyl-pyrido[2,3-d]pyrimidine-2,4-diamine trifluoroacetic acid salt,
N4-Methyl-7-(2,3,5,6-tetramethyl-phenyl)-pyrido[2,3-d]pyrimidine-2,4-diamine trifluoroacetic acid salt,
N4-Methyl-7-phenyl-6-propyl-pyrido[2,3-d]pyrimidine-2,4-diamine trifluoroacetic acid salt,
6-Ethyl-N4-methyl-7-phenyl-pyrido[2,3-d]pyrimidine-2,4-diamine trifluoroacetic acid salt,
6-Methanesulfonyl-N4-methyl-7-(2-trifluoromethyl-phenyl)-pyrido[2,3-d]pyrimidine-2,4-diamine trifluoroacetic acid salt,
N4-Methyl-7-(2,3,6-trimethyl-phenyl)-pyrido[2,3-d]pyrimidine-2,4-diamine,
7-(2,6-Dichloro-3-fluoro-phenyl)-N4-methyl-pyrido[2,3-d]pyrimidine-2,4-diamine,
7-(2,4-Bis-trifluoromethyl-phenyl)-N4-methyl-pyrido[2,3-d]pyrimidine-2,4-diamine,
7-(2,6-Bis-trifluoromethyl-phenyl)-N4-methyl-pyrido[2,3-d]pyrimidine-2,4-diamine,
7-(2,5-Dimethyl-phenyl)-N4-methyl-pyrido[2,3-d]pyrimidine-2,4-diamine,
N4-Methyl-7-(2,3,6-trichloro-phenyl)-pyrido[2,3-d]pyrimidine-2,4-diamine,
2-(2-Amino-4-methylamino-pyrido[2,3-d]pyrimidin-7-yl)-4-methyl-phenol,
N9-Methyl-6,7-dihydro-5H-10,12,13-triaza-benzo[3,4]cyclohepta[1,2-b]naphthalene-9,11-diamine trifluoroacetic acid salt,
6-Isopropyl-N4-methyl-7-phenyl-pyrido[2,3-d]pyrimidine-2,4-diamine trifluoroacetic acid salt,
2-(2-Amino-4-methylamino-pyrido[2,3-d]pyrimidin-7-yl)-phenol trifluoroacetic acid salt,
7-(2,5-Dichloro-phenyl)-N4-methyl-pyrido[2,3-d]pyrimidine-2,4-diamine trifluoroacetic acid salt,
7-(2,4-Dichloro-phenyl)-N4-methyl-pyrido[2,3-d]pyri midi ne-2,4-diami ne trifluoroacetic acid salt,
7-(2,3-Dichloro-phenyl)-N4-methyl-pyrido[2,3-d]pyrimidine-2,4-diamine trifluoroacetic acid salt,
N4-Methyl-6-phenethyl-7-phenyl-pyrido[2,3-d]pyrimidine-2,4-diamine trifluoroacetic acid salt,
7-(2-Cyclopentyloxy-6-fluoro-phenyl)-N4-methyl-pyrido[2,3-d]pyrimidine-2,4-diamine trifluoroacetic acid salt,
2-[2-(2-Amino-4-methylamino-pyrido[2,3-d]pyrimidin-7-yl)-3-fluoro-phenoxy]-ethanol trifluoroacetic acid,
3-[2-(2-Amino-4-methylamino-pyrido[2,3-d]pyrimidin-7-yl)-3-fluoro-phenoxy]-propan-1-ol trifluoroacetic acid,
7-(2-Chloro-6-ethoxy-phenyl)-N4-methyl-pyrido[2,3-d]pyrimidine-2,4-diamine trifluoroacetic acid salt,
2-Amino-4-methylamino-7-(2-trifluoro-methyl-phenyl)-pyrido[2,3-d]pyrimidine-6-carboxylic acid trifluoroacetic acid salt,
N4-Methyl-7-(1-phenyl-cyclopropyl)-pyrido[2,3-d]pyrimidine-2,4-diamine,
N4-Methyl-7-(1-phenyl-cyclopentyl)-pyrido[2,3-d]pyrimidine-2,4-diamine,
N4-Methyl-7-(1-phenyl-cyclohexyl)-pyrido[2,3-d]pyrimidine-2,4-diamine, potassium 2-(2-Amino-4-methylamino-pyrido[2,3-d]pyrimidin-7-yl)-benzoate,
7-(2,4-Diethyl-phenyl)-N4-methyl-pyrido[2,3-d]pyri midi ne-2,4-diami ne,
N4-Ethyl-7-(2-trifluoromethyl-phenyl)-pyrido[2,3-d]pyri midi ne-2,4-diami ne trifluoroacetic acid salt,
N4-Ethyl-6-methyl-7-(2-trifluoromethyl-phenyl)-pyrido[2,3-d]pyrimidine-2,4-diamine,
N4-Ethyl-7-o-tolyl-pyrido[2,3-d]pyrimidine-2,4-diamine trifluoroacetic acid salt,
7-(2,6-Dichloro-phenyl)-N4-ethyl-pyrido[2,3-d]pyrimidine-2,4-diamine trifluoroacetic acid salt,
7-(2-Bromo-phenyl)-N4-ethyl-pyrido[2,3-d]pyrimidine-2,4-diamine trifluoroacetic acid salt,
N4-Ethyl-7-(2-fluoro-6-trifluoromethyl-phenyl)-pyrido[2,3-d]pyrimidine-2,4-diamine trifluoroacetic acid salt,
7-(2-Chloro-6-fluoro-phenyl)-N4-ethyl-pyrido[2,3-d]pyrimidine-2,4-diamine trifluoroacetic acid salt,
N4-Ethyl-7-(2,3,6-trimethyl-phenyl)-pyrido[2,3-d]pyrimidine-2,4-diamine trifluoroacetic acid salt,
2-[2-Amino-7-(2,6-dichloro-phenyl)-pyrido[2,3-d]pyrimidin-4-ylamino]-ethanol,
N4-Methyl-7-(2-pi peridi n-1-yl-6-trifluoromethyl-phenyl)-pyrido[2,3-d]pyri midi ne-2,4-diamine,
N4-Methyl-7-(2-morpholi n-4-yl-6-trifl uoromethyl-phenyl)-pyrido[2,3-d]pyri midi ne-2,4-diamine trifluoroacetic acid salt,
7-(2,4-Dimethyl-phenyl)-N4-methyl-pyrido[2,3-d]pyrimidine-2,4-diamine,
N4-Methyl-7-[2-(4-methyl-piperazin-1-yl)-6-trifluoromethyl-phenyl]-pyrido[2,3-d]pyrimidine-2,4-diamine trifluoroacetic acid salt,
7-(2-Ethoxy-6-trifluoromethyl-phenyl)-N4-methyl-pyrido[2,3-d]pyrimidine-2,4-diamine trifluoroacetic acid salt,
7-(2-Methoxy-6-trifluoromethyl-phenyl)-N4-methyl-pyrido[2,3-d]pyrimidine-2,4-diamine trifluoroacetic acid salt,
7-(2-Dimethylamino-6-trifluoromethyl-phenyl)-N4-methyl-pyrido[2,3-d]pyrimidine-2,4-diamine,
N4-Methyl-7-(2-methylamino-6-trifluoromethyl-phenyl)-pyrido[2,3-d]pyrimidine-2,4-diamine trifluoroacetic acid salt,
7-[2-(2-Di methylamino-ethoxy)-6-trifluoromethyl-phenyl]-N4-methyl-pyrido[2,3-d]pyrimidine-2,4-diamine trifluoroacetic acid salt,
N4-Methyl-7-(2-phenoxy-6-trifluoromethyl-phenyl)-pyrido[2,3-d]pyri midi ne-2,4-diamine trifluoroacetic acid salt,
N4-Methyl-7-(2-methylsulfanyl-6-trifluoromethyl-phenyl)-pyrido[2,3-d]pyrimidine-2,4-diamine trifluoroacetic acid salt,
2-[2-(2-Amino-4-methylamino-pyrido[2,3-d]pyrimidin-7-yl)-3-trifluoromethyl-phenoxy]-ethanol trifluoroacetic acid salt,
7-[2-(2-Methoxy-ethoxy)-6-trifluoromethyl-phenyl]-N4-methyl-pyrido[2,3-d]pyrimidine-2,4-diamine trifluoroacetic acid salt,
N4-Methyl-7-[2-(2-pyrrolidi n-1-yl-ethoxy)-6-trifluoromethyl-phenyl]-pyrido[2,3-d]pyrimidine-2,4-diamine trifluoroacetic acid salt,
7-(2-Isopropoxy-6-trifluoromethyl-phenyl)-N4-methyl-pyrido[2,3-d]pyrimidine-2,4-diamine trifluoroacetic acid salt,
N4-Methyl-7-(2-propoxy-6-trifluoromethyl-phenyl)-pyrido[2,3-d]pyrimidine-2,4-diamine trifluoroacetic acid salt,
7-[2-(2-Diethylamino-ethoxy)-6-trifluoromethyl-phenyl]-N4-methyl-pyrido[2,3-d]pyrimidine-2,4-diamine trifluoroacetic acid salt,
N4-Methyl-7-[2-(2-morpholin-4-yl-ethoxy)-6-trifluoromethyl-phenyl]-pyrido[2,3-d]pyrimidine-2,4-diamine trifluoroacetic acid salt,
7-(2-fluoro-6-pyrrolidi n-1-yl-phenyl)-N4-methyl-pyrido[2,3-d]pyri midi ne-2,4-diami ne,
7-(2-Fluoro-6-piperidin-1-yl-phenyl)-N4-methyl-pyrido[2,3-d]pyrimidine-2,4-diamine,
2-(2-amino-4-methylamino-pyrido[2,3-d]pyrimidin-7-yl)-3-fluoro-phenol,
7-(2-Fluoro-6-morpholino-4-yl-phenyl)-N4-methyl-pyrido[2,3-d]pyrimidine-2,4-diamine,
7-(2-Fluoro-6-phenylsulfanyl-phenyl)-N4-methyl-pyrido[2,3-d]pyrimidine-2,4-diamine trifluoroacetic acid salt,
7-(2-Fluoro-6-phenoxy-phenyl)-N4-methyl-pyrido[2,3-d]pyrimidine-2,4-diami*ne trifluoroacetic acid,
7-(2-Fluoro-6-imidazol-1-yl-phenyl)-N4-methyl-pyrido[2,3-d]pyrimidine-2,4-diamine,
7-[2-(4-Benzyl-piperazin-1-yl)-6-fluoro-phenyl]-N4-methyl-pyrido[2,3-d]pyrimidine-2,4-diamine trifluoroacetic acid salt,
7-(2-Fluoro-6-methylamino-phenyl)-N4-methyl-pyrido[2,3-d]pyrimidine-2,4-diamine trifluoroacetic acid salt,
7-(2-Dimethylamino-6-fluoro-phenyl)-N4-methyl-pyrido[2,3-d]pyrimidine-2,4-diamine trifluoroacetic acid salt,
7-[2-Fluoro-6-(4-methyl-piperazin-1-yl)-phenyl]-N4-methyl-pyrido[2,3-d]pyrimidine-2,4-diamine trifluoroacetic acid salt,
1-[2-(2-Amino-4-methylamino-pyrido[2,3-d]pyrimidin-7-yl)-3-fluoro-phenyl]-piperidine-2-carboxylic acid ethyl ester trifluoroacetic acid salt,
7-(2-Fluoro-6-thiomorpholin-4-yl-phenyl)-N4-methyl-pyrido[2,3-d]pyrimidine-2,4-diamine trifluoroacetic acid salt,
7-[2-(3-Aminomethyl-piperidin-1-yl)-6-fluoro-phenyl]-N4-methyl-pyrido[2,3-d]pyrimidine-2,4-diamine trifluoroacetic acid salt, 7-[2-Fluoro-6-(2-methoxymethyl-pyrrolidin-1-yl)-phenyl]-N4-methyl-pyrido[2,3-d]pyrimidine-2,4-diamine trifluoroacetic acid salt,
7-[2-(4-Fluoro-phenoxy)-6-trifluoromethyl-phenyl]-N4-methyl-pyrido[2,3-d]pyrimidine-2,4-diamine trifluoroacetic acid salt,
7-(2-Cyclohexyloxy-6-trifluoromethyl-phenyl)-N4-methyl-pyrido[2,3-d]pyrimidine-2,4-diamine trifluoroacetic acid salt,
N4-Methyl-7-[2-(2-methyl-pyrrolidi n-1-yl)-6-trifluoromethyl-phenyl]-pyrido[2,3-d]pyrimidine-2,4-diamine,
7-[2-(2,5-Di methyl-pyrrolidi n-1-yl)-6-trifluoro methyl-phenyl]-N4-methyl-pyrido[2,3-d]pyrimidine-2,4-diamine trifluoroacetic acid salt,
1-[2-(2-Amino-4-methylamino-pyrido[2,3-d]pyrimidin-7-yl)-3-trifluoromethyl-phenyl]-pyrrolidin-3-ol trifluoroacetic acid salt,
1-[2-(2-Amino-4-methylamino-pyrido[2,3-d]pyrimidin-7-yl)-3-trifluoromethyl-phenyl]-piperidin-4-ol trifluoroacetic acid salt,
2-[2-Amino-7-(2-phenoxy-6-trifluoromethyl-phenyl)-pyrido[2,3-d]pyrimidin-4-ylamino]-ethanol trifluoroacetic acid salt,
{1-[2-(2-Amino-4-methylamino-pyrido[2,3-d]pyrimidin-7-yl)-3-trifluoromethylphenyl]-pyrrolidin-2-yl}-methanol trifluoroacetic acid salt,
7-[2-(2-Methoxymethyl-pyrrolidi n-1-yl)-6-trifluoromethyl-phenyl]-N4-methyl-pyrido[2,3-d]pyrimidine-2,4-diamine trifluoroacetic acid salt,
1-[2-(2-Amino-4-methylamino-pyrido[2,3-d]pyrimidin-7-yl)-3-trifluoromethyl-phenyl]-piperidin-3-ol trifluoroacetic acid salt,
7-[2-(4-Cyclohexyl-piperazin-1-yl)-6-trifluoromethyl-phenyl]-N4-methyl-pyrido[2,3-d]pyrimidine-2,4-diamine trifluoroacetic acid salt,
7-[2-(4-Ethyl-piperazin-1-yl)-6-trifluoromethyl-phenyl]-N4-methyl-pyrido[2,3-d]pyrimidine-2,4-diamine trifluoroacetic acid salt,
{4-[2-(2-Amino-4-methylamino-pyrido[2,3-d]pyrimidin-7-yl)-3-trifluoromethylphenyl]-piperazin-1-yl}-furan-2-yl-methanone trifluoroacetic acid salt,
7-(2-{4-[Bis-(4-fluoro-phenyl)-methyl]-piperazin-1-yl}-6-trifluoromethyl-phenyl)-N4-methyl-pyrido[2,3-d]pyrimidine-2,4-diamine trifluoroacetic acid salt,
N4-Methyl-7-[2-(4-phenyl-piperazin-1-yl)-6-trifluoromethyl-phenyl]-pyrido[2,3-d]pyrimidine-2,4-diamine trifluoroacetic acid salt,
7-[2-(4-Benzyl-piperazin-1-yl)-6-trifluoromethyl-phenyl]-N4-methyl-pyrido[2,3-d]pyrimidine-2,4-diamine trifluoroacetic acid salt,
1-[2-(2-Amino-4-methylamino-pyrido[2,3-d]pyrimidin-7-yl)-3-trifluoromethyl-phenyl]-4-phenyl-piperidin-4-ol trifluoroacetic acid salt,
7-[2-(4-Benzyl-piperidin-1-yl)-6-trifluoromethyl-phenyl]-N4-methyl-pyrido[2,3-d]pyrimidine-2,4-diamine trifluoroacetic acid salt,
7-[2-(4-Cyclopentyl-piperazin-1-yl)-6-trifluoromethyl-phenyl]-N4-methyl-pyrido[2,3-d]pyrimidine-2,4-diamine trifluoroacetic acid salt,
2-{4-[2-(2-Amino-4-methylamino-pyrido[2,3-d]pyrimidin-7-yl)-3-trifluoromethylphenyl]-piperazin-1-yl}-N-isopropyl-acetamide trifluoroacetic acid salt,
7-[2-(4-Isopropyl-piperazin-1-yl)-6-trifluoromethyl-phenyl]-N4-methyl-pyrido[2,3-d]pyrimidine-2,4-diamine trifluoroacetic acid salt,
7-[2-(2-Fluoro-phenoxy)-6-trifluoromethyl-phenyl]-N4-methyl-pyrido[2,3-d]pyrimidine-2,4-diamine trifluoroacetic acid salt,
7-[2-(3-Fluoro-phenoxy)-6-trifluoromethyl-phenyl]-N4-methyl-pyrido[2,3-d]pyrimidine-2,4-diamine trifluoroacetic acid salt,
7-[2-(3-Chloro-phenoxy)-6-trifluoromethyl-phenyl]-N4-methyl-pyrido[2,3-d]pyrimidine-2,4-diamine trifluoroacetic acid salt,
7-[2-(4-Chloro-phenoxy)-6-trifluoromethyl-phenyl]-N4-methyl-pyrido[2,3-d]pyrimidine-2,4-diamine trifluoroacetic acid salt,
N4-Methyl-7-(2-p-tolyloxy-6-trifluoromethyl-phenyl)-pyrido[2,3-d]pyri midi ne-2,4-diamine trifluoroacetic acid salt,
7-[2-(Biphenyl-4-yloxy)-6-trifluoromethyl-phenyl]-N4-methyl-pyrido[2,3-d]pyrimidine-2,4-diamine trifluoroacetic acid salt,
2-[2-Amino-7-(2-pyrrolidin-1-yl-6-trifluoromethyl-phenyl)-pyrido[2,3-d]pyrimidin-4-ylamino]-ethanol trifluoroacetic acid salt,
4-[2-amino-7-(2-trifluoromethyl-phenyl)-pyrido[2,3-d]pyrimidin-4-ylamino]-propan-1-ol trifluoroacetic acid salt,
2-[2-Amino-7-(2-trifluoromethyl-phenyl)-pyrido[2,3-d]pyrimidin-4-ylamino]-ethanol,
N4-Propyl-7-(2-trifluoromethyl-phenyl)-pyrido[2,3-d]pyrimidine-2,4-diamine trifluoroacetic acid salt,
4-[2-Ami no-7-(2-trifluoromethyl-phenyl)-pyrido[2,3-d]pyri midi n-4-ylami no]-butan-1-ol trifluoroacetic acid salt,
2-[2-Amino-7-(2-fluoro-6-trifluoromethyl-phenyl)-pyrido[2,3-d]pyrimidin-4-ylamino]-ethanol,
2-[2-Amino-7-(2-bromo-phenyl)-pyrido[2,3-d]pyrimidin-4-ylamino]-ethanol,
5,N-4-Dimethyl-7-phenyl-pyrido[2,3-d]pyrimidine-2,4-diamine trifluoroacetate, and
N-4-Methyl-5,7-diphenyl-pyrido[2,3-d]pyrimidine-2,4-diamine trifluoroacetate,
and pharmaceutically acceptable salts and esters thereof.

44. Compounds of any of claims 1 to 43, selected from the group consisting of
N4-Methyl-7-(2-trifluoromethyl-phenyl)-pyrido[2,3-d]pyrimidine-2,4-diamine,
7-(2,6-Dichloro-phenyl)-N4-methyl-pyrido[2,3-d]pyrimidine-2,4-diamine,
2-chloro-6-fluorophenyl-N4-methyl-pyrido[2,3-d]pyrimidine-2,4-diamine,
7-(2-Fluoro-6-trifluoromethyl-phenyl)-N4-methyl-pyrido[2,3-d]pyrimidine-2,4-diamine,
7-(2-Chloro-6-fluoro-phenyl)-N4-methyl-pyrido[2,3-d]pyrimidine-2,4-diamine,
7-(2,6-Dichloro-3-fluoro-phenyl)-N4-methyl-pyrido[2,3-d]pyrimidine-2,4-diamine,
7-(2,6-Bis-trifluoromethyl-phenyl)-N4-methyl-pyrido[2,3-d]pyrimidine-2,4-diamine,
7-(2-Chloro-6-ethoxy-phenyl)-N4-methyl-pyrido[2,3-d]pyrimidine-2,4-diamine trifluoroacetic acid salt,
2-Amino-4-methylamino-7-(2-trifluoromethyl-phenyl)-pyrido[2,3-d]pyrimidine-6-carboxylic acid trifluoroacetic acid salt,
7-(2,4-Dimethyl-phenyl)-N4-methyl-pyrido[2,3-d]pyrimidine-2,4-diamine,
7-(2-Ethoxy-6-trifluoromethyl-phenyl)-N4-methyl-pyrido[2,3-d]pyrimidine-2,4-diamine trifluoroacetic acid salt,
N4-Methyl-7-(2-phenoxy-6-trifluoromethyl-phenyl)-pyrido[2,3-d]pyrimidine-2,4-diamine trifluoroacetic acid salt,
7-(2-Isopropoxy-6-trifluoromethyl-phenyl)-N4-methyl-pyrido[2,3-d]pyrimidine-2,4-diamine trifluoroacetic acid salt,
7-[2-(4-Fluoro-phenoxy)-6-trifluoromethyl-phenyl]-N4-methyl-pyrido[2,3-d]pyrimidine-2,4-diamine trifluoroacetic acid salt,
7-(2-Cyclohexyloxy-6-trifluoromethyl-phenyl)-N4-methyl-pyrido[2,3-d]pyrimidine-2,4-diamine trifluoroacetic acid salt,
7-[2-(2-Fluoro-phenoxy)-6-trifluoromethyl-phenyl]-N4-methyl-pyrido[2,3-d]pyrimidine-2,4-diamine trifluoroacetic acid salt,
7-[2-(3-Fluoro-phenoxy)-6-trifluoromethyl-phenyl]-N4-methyl-pyrido[2,3-d]pyrimidine-2,4-diamine trifluoroacetic acid salt,
7-[2-(4-Chloro-phenoxy)-6-trifluoromethyl-phenyl]-N4-methyl-pyrido[2,3-d]pyrimidine-2,4-diamine trifluoroacetic acid salt, and
N4-Methyl-7-(2-p-tolyloxy-6-trifluoromethyl-phenyl)-pyrido[2,3-d]pyrimidine-2,4-diamine trifluoroacetic acid salt,
and pharmaceutically acceptable salts and esters thereof.

45. A process for the preparation of compounds according to any of claims 1 to 44, comprising reacting a compound of formula (II) 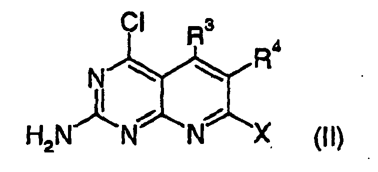 with a compound HN(R¹,R²), wherein R¹, R², R³, R⁴ and X are as defined in any of claims 1 to 44.

46. Pharmaceutical compositions comprising a compound according to any of claims 1 to 44 and a pharmaceutically acceptable carrier and/or adjuvant.

47. Compounds according to any of claims 1 to 44 for use as therapeutic active substances.

48. Compounds according to any of claims 1 to 44 useful for the therapeutic and/or prophylactic treatment of diabetes.

49. The use of compounds according to any of claims 1 to 44 for the preparation of medicaments for the therapeutic and/or prophylactic treatment of diabetes.

## Patentansprüche

1. Verbindungen der Formel (I): 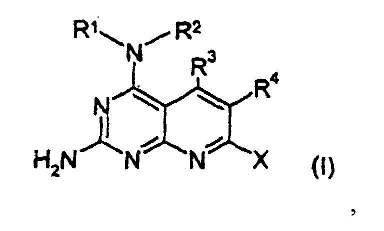
worin X eine Gruppe X-1 der Formel: 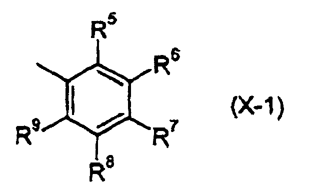 ist,
oder X eine Gruppe X-2 der Formel: 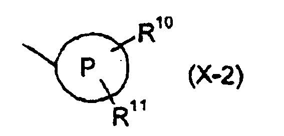 ist,
oder X eine Gruppe X-3 der Formel: 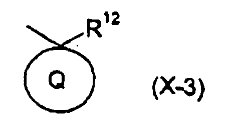 ist;
R¹ und R² unabhängig voneinander ausgewählt sind aus der Gruppe, bestehend aus Wasserstoff, Niederalkyl, Methoxyniederalkyl und Hydroxyniederalkyl, außer dass R¹ und R² nicht beide Wasserstoff sein können;
R³ Wasserstoff, Niederalkyl oder Phenyl ist;
R⁴ Wasserstoff, Niederalkyl, Niederalkylsulfonyl, Phenyl, Carboxy ist oder zusammen mit R⁵ einen 5- bis 7gliedrigen carbocyclischen Ring bildet;
R⁵ sofern nicht in einem Ring mit R⁴ kondensiert, Wasserstoff, Niederalkyl, substituiertes Niederalkyl, Niederalkoxy, substituiertes Niederalkoxy, Hydroxy, Carboxy, Halogen, Niederalkylthio, Niederalkylsulfinyl, Niederalkylsulfonyl, Aminosulfonyl, Cyano, Nitro, Niederalkanoyl, Aryl, Aroyl, Aryloxy, Arylthio, Perfluorniederalkyl, Niederalkylamino, Niederalkanoylamino, Sulfonylamino, Cycloalkyl, Cycloalkoxy, Heterocyclyl, Heterocyclyloxy, Heterocyclylcarbonyl, Heteroaryl ist oder zusammen mit R⁶ einen zweiten kondensierten 5- oder 6gliedrigen aromatischen Ring bildet;
R⁶, sofern nicht in einem Ring mit R⁵ kondensiert, Wasserstoff, Niederalkyl, substituiertes Niederalkyl, Niederalkoxy, substituiertes Niederalkoxy, Hydroxy, Halogen, Niederalkylthio, Niederalkylsulfinyl, Niederalkylsulfonyl, Aminosulfonyl, Cyano, Nitro, Niederalkanoyl, Aryl, Aroyl, Aryloxy, Niederalkylamino, Niederalkanoylamino, Sulfonylamino, Cycloalkyl, Heterocyclyl, Heterocyclyloxy oder Heterocyclylcarbonyl ist;
R⁷ Wasserstoff, Niederalkyl, Niederalkoxy, Alkoxyniederalkyl, Alkoxyniederalkoxy, Hydroxyniederalkyl, Hydroxy, Hydroxyalkoxy, Halogen, Niederalkylthio, Niederalkylsulfinyl, Niederalkylsulfonyl, Perfluorniederalkyl, Niederalkanoyl, Aroyl oder Niederalkanoylamino ist;
R⁸ und R⁹ jeweils unabhängig voneinander ausgewählt sind aus der Gruppe, bestehend aus Wasserstoff, Niederalkyl, substituiertem Niederalkyl, Niederalkoxy, substituiertem Niederalkoxy, Hydroxy, Halogen, Niederalkylthio, Niederalkylsulfinyl, Niederalkylsulfonyl, Aminosulfonyl, Cyano, Nitro, Niederalkanoyl, Aryl, Aroyl, Aryloxy, Niederalkylamino, Niederalkanoylamino, Sulfonylamino, Cycloalkyl, Heterocyclyl, Heterocyclyloxy und Heterocyclylcarbonyl;
P ein 5- oder 6gliedriger heteroaromatischer Ring, enthaltend 1 bis 2 Heteroatome, ausgewählt aus der Gruppe, bestehend aus Sauerstoff, Schwefel und Stickstoff, ist;
R¹⁰ und R¹¹ jeweils unabhängig voneinander ausgewählt sind aus der Gruppe, bestehend aus Wasserstoff, Niederalkyl, Niederalkoxy, Perfluorniederalkyl, Halogen, Arylniederalkyl, Aryl und Arylniederalkoxy;
Q ein 3- bis 6gliedriger Cycloalkylring ist; und
R¹² Wasserstoff oder Aryl ist;
oder die pharmazeutisch akzeptablen Salze oder Ester davon,
wobei der Ausdruck "Niederalkyl", allein oder in Kombination, eine geradkettige oder verzweigtkettige Alkylgruppe, enthaltend höchstens sechs Kohlenstoffatome, bedeutet; wobei der Ausdruck "Niederalkoxy" eine Niederalkylgruppe (wie oben definiert), gebunden durch ein Sauerstoffatom, bedeutet;
wobei der Ausdruck "Niederalkylthio" eine Niederalkylgruppe, gebunden durch ein zweiwertiges Schwefelatom, bedeutet; und
wobei der Ausdruck "Niederalkanoyl" eine Niederalkylgruppe, gebunden an den Rest des Moleküls über eine Carbonylgruppe, bedeutet.

2. Verbindungen nach Anspruch 1 der Formel (Ia): 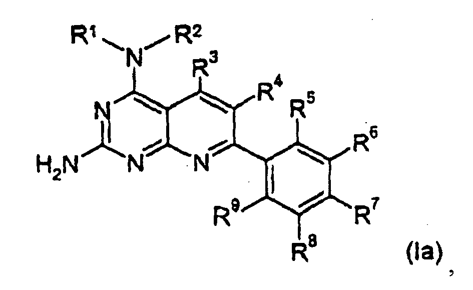
worin R³ Wasserstoff ist und R⁴ Wasserstoff, Niederalkyl, Niederalkylsulfonyl, Phenyl oder Carboxy ist, und R¹, R², R⁵, R⁶, R⁷, R⁸ und R⁹ wie in Anspruch 1 definiert sind.

3. Verbindungen nach Anspruch 2, wobei R⁶, R⁷ und R⁸ jeweils unabhängig voneinander Wasserstoff, Halogen, Niederalkyl, Niederalkoxy, Hydroxy, Hydroxyniederalkyl, Niederalkylthio, Niederalkylsulfinyl, Niederalkylsulfonyl oder Perfluorniederalkyl sind.

4. Verbindungen nach Anspruch 3, wobei R⁷ Wasserstoff oder Fluor ist.

5. Verbindungen nach Anspruch 4, wobei einer von R⁶ und R⁸ Wasserstoff oder Fluor ist.

6. Verbindungen nach Anspruch 4, wobei einer von R⁶ und R⁸ Wasserstoff oder Fluor ist und der andere Halogen, Niederalkyl, Niederalkoxy, Hydroxy, Hydroxyniederalkyl, Niederalkylthio, Niederalkylsulfinyl, Niederalkylsulfonyl oder Perfluorniederalkyl ist.

7. Verbindungen nach Anspruch 5, wobei R⁶, R⁷ und R⁸ Wasserstoff sind.

8. Verbindungen nach einem der Ansprüche 2 bis 7, wobei R⁵ und R⁹ jeweils unabhängig voneinander ausgewählt sind aus der Gruppe, bestehend aus Wasserstoff, Halogen, Niederalkyl, Niederalkoxy, Alkoxyniederalkoxy, Nitro, Hydroxy, Hydroxyniederalkoxy, Hydroxyniederalkyl, Niederalkylthio, Niederalkylamino, Niederalkylsulfonyl, Niederalkylsulfinyl, Perfluorniederalkyl, Cycloalkyl, Cycloalkoxy, Aryl, Heteroaryl, Aryloxy, Arylthio und Heterocyclyl.

9. Verbindungen nach einem der Ansprüche 5 bis 7, wobei R⁵ und R⁹ jeweils unabhängig voneinander ausgewählt sind aus der Gruppe, bestehend aus Chlor, Fluor, Trifluormethyl, C₁₋₄-Alkyl, C₁₋₃-Alkylthio, C₁₋₃-Alkylsulfonyl, C₁₋₃-Alkoxy, Phenoxy, Phenoxy, monosubstituiert mit Fluor, Chlor oder Sauerstoff, und C₁₋₃-Alkoxy, substituiert mit Hydroxy, Methoxy oder Ethoxy.

10. Verbindungen nach Anspruch 2, wobei R¹ oder R² Wasserstoff ist.

11. Verbindung nach Anspruch 10, wobei der substituierte R¹ oder R² mit C₁₋₄-Alkyl oder Hydroxy-C₁₋₃-alkyl substituiert ist.

12. Verbindungen nach Anspruch 10, wobei R⁶, R⁷ und R⁸ jeweils unabhängig voneinander Wasserstoff, Halogen, Niederalkyl, Niederalkoxy, Hydroxy, Hydroxyniederalkyl, Niederalkylthio, Niederalkylsulfinyl, Niederalkylsulfonyl oder Perfluorniederalkyl sind.

13. Verbindungen nach Anspruch 10, wobei R⁷ Wasserstoff oder Fluor ist.

14. Verbindungen nach Anspruch 13, wobei einer von R⁶ und R⁸ Wasserstoff oder Fluor ist.

15. Verbindungen nach Anspruch 13, wobei einer von R⁶ und R⁸ Wasserstoff oder Fluor ist und der andere Halogen, Niederalkyl, Niederalkoxy, Hydroxy, Hydroxyniederalkyl, Niederalkylthio, Niederalkylsulfinyl, Niederalkylsulfonyl oder Perfluorniederalkyl ist.

16. Verbindungen nach Anspruch 14, wobei R⁶, R⁷ und R⁸ Wasserstoff sind.

17. Verbindungen nach Anspruch 16, wobei der substituierte R¹ oder R² mit C₁₋₄-Alkyl oder Hydroxy-C₁₋₃-alkyl substituiert ist.

18. Verbindungen nach einem der Ansprüche 10 bis 15, wobei R⁵ und R⁹ jeweils unabhängig voneinander ausgewählt sind aus der Gruppe, bestehend aus Wasserstoff, Halogen, Niederalkyl, Niederalkoxy, Alkoxyniederalkoxy, Nitro, Hydroxy, Hydroxyniederalkoxy, Hydroxyniederalkyl, Niederalkylthio, Niederalkylamino, Niederalkylsulfonyl, Niederalkylsulfinyl, Perfluorniederalkyl, Cycloalkyl, Cycloalkoxy, Aryl, Heteroaryl, Aryloxy, Arylthio und Heterocyclyl.

19. Verbindungen nach Anspruch 18, wobei der substituierte R¹ oder R² mit C₁₋₄-Alkyl oder Hydroxy-C₁₋₃-alkyl substituiert ist.

20. Verbindungen nach Anspruch 16, wobei R⁵ und R⁹ jeweils unabhängig voneinander ausgewählt sind aus der Gruppe, bestehend aus Chlor, Fluor, Trifluormethyl, C₁₋₄-Alkyl, C₁₋₃-Alkylthio, C₁₋₃-Alkylsulfonyl, C₁₋₃-Alkoxy, Phenoxy, Phenoxy, monosubstituiert mit Fluor, Chlor oder Sauerstoff, und C₁₋₃-Alkoxy, substituiert mit Hydroxy, Methoxy oder Ethoxy.

21. Verbindungen nach Anspruch 20, wobei der substituierte R¹ oder R² mit C₁₋₄-Alkyl oder Hydroxy-C₁₋₃-alkyl substituiert ist.

22. Verbindungen nach Anspruch 1, wobei R⁴ und R⁵ einen 5- bis 7gliedrigen carbocyclischen Ring bilden.

23. Verbindungen nach Anspruch 22, wobei R¹ oder R² Wasserstoff ist.

24. Verbindungen nach Anspruch 23, wobei R⁷ Wasserstoff oder Fluor ist.

25. Verbindungen nach Anspruch 24, wobei einer von R⁶ und R⁸ Wasserstoff ist.

26. Verbindungen nach Anspruch 24, wobei einer von R⁶ und R⁸ Wasserstoff oder Fluor ist und der andere Halogen, Niederalkyl, Niederalkoxy, Hydroxy, Hydroxyniederalkyl, Niederalkylthio, Niederalkylsulfinyl, Niederalkylsulfonyl oder Perfluorniederalkyl ist.

27. Verbindungen nach Anspruch 25, wobei R⁶, R⁷ und R⁸ Wasserstoff sind.

28. Verbindungen nach Anspruch 27, wobei der substituierte R¹ oder R² mit C₁₋₄-Alkyl oder Hydroxy-C₁₋₃-alkyl substituiert ist.

29. Verbindungen nach Anspruch 23, wobei R⁵ und R⁹ jeweils unabhängig voneinander Wasserstoff, Halogen, Niederalkyl, Niederalkoxy, Alkoxyniederalkoxy, Nitro, Hydroxy, Hydroxyniederalkoxy, Hydroxyniederalkyl, Niederalkylthio, Niederalkylsulfinyl, Niederalkylsulfonyl und Perfluorniederalkyl sind.

30. Verbindungen nach Anspruch 23 oder 25, wobei der substituierte R¹ oder R² mit C₁₋₄-Alkyl oder Hydroxy-C₁₋₃-alkyl substituiert ist.

31. Verbindungen nach Anspruch 1 der Formel (Ib): 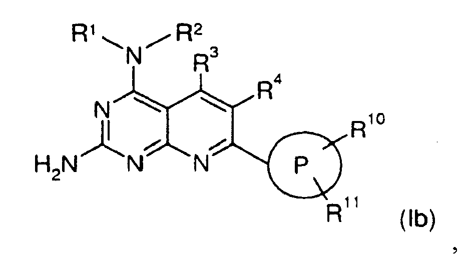
worin R¹, R², R³, R⁴, P, R¹⁰ und R¹¹ wie in Anspruch 1 definiert sind.

32. Verbindungen nach Anspruch 31, wobei R¹ oder R² Wasserstoff ist.

33. Verbindungen nach Anspruch 32, wobei R³ Wasserstoff ist und R⁴ Wasserstoff, Niederalkyl, Niederalkylsulfonyl, Phenyl oder Carboxy ist.

34. Verbindungen nach Anspruch 32 oder 33, wobei R¹⁰ und R¹¹ jeweils unabhängig voneinander Niederalkyl, Niederalkoxy, Perfluorniederalkyl oder Halogen sind.

35. Verbindungen nach Anspruch 34, wobei der substituierte R¹ oder R² mit C₁₋₄-Alkyl oder Hydroxy-C₁₋₃-alkyl substituiert ist.

36. Verbindungen nach Anspruch 34, wobei der substituierte R¹ oder R² mit C₁₋₄-Alkyl oder Hydroxy-C₁₋₃-alkyl substituiert ist.

37. Verbindungen nach Anspruch 1 der Formel (Ic): 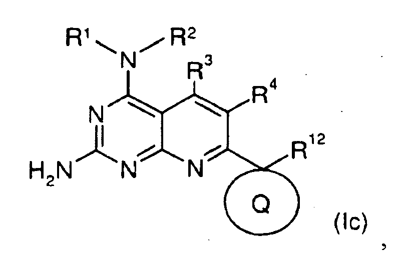
worin R¹, R², R³, R⁴, Q und R¹² wie in Anspruch 1 definiert sind.

38. Verbindungen nach Anspruch 37, wobei R¹ oder R² Wasserstoff ist.

39. Verbindungen nach Anspruch 38, wobei R³ Wasserstoff ist und R⁴ Wasserstoff, Niederalkyl, Niederalkylsulfonyl, Phenyl oder Carboxy ist.

40. Verbindungen nach Anspruch 38, wobei R¹² unsubstituiertes oder substituiertes Phenyl ist.

41. Verbindungen nach Anspruch 38, wobei R¹² monosubstituiertes Phenyl ist.

42. Verbindungen nach Anspruch 38 oder 40, wobei der substituierte R¹ oder R² mit C₁₋₄-Alkyl oder Hydroxy-C₁₋₃-alkyl substituiert ist.

43. Verbindungen nach einem der Ansprüche 1 bis 42, ausgewählt aus der Gruppe, bestehend aus
N4-Methyl-7-o-tolyl-pyrido[2,3-d]pyrimidin-2,4-diamin,
N4-Methyl-7-(2-trifluormethyl-phenyl)-pyrido[2,3-d]pyrimidin-2,4-diamin,
7-(2,6-Dichlor-phenyl)-N4-methyl-pyrido[2,3-d]pyrimidin-2,4-diamin,
7-(2-Chlor-phenyl)-N4-methyl-pyrido[2,3-d]pyrimidin-2,4-diamin,
7-(2,6-Difluor-phenyl)-N4-methyl-pyrido[2,3-d]pyrimidin-2,4-diamin,
7-tert-Butyl-N4-methyl-pyrido[2,3-d]pyrimidin-2,4-diamin,
2-Chlor-6-fluorphenyl-N4-methyl-pyrido[2,3-d]pyrimidin-2,4-diamin,
7-(2-Fluor-6-trifluormethyl-phenyl)-N4-methyl-pyrido[2,3-d]pyrimidin-2,4-diamin,
7-Cyclohexyl-N4-methyl-pyrido[2,3-d]pyrimidin-2,4-diamin,
7-(2-Methoxy-phenyl)-N4-methyl-pyrido[2,3-d]pyrimidin-2,4-diamin,
N4-Methyl-7-(2-nitro-phenyl)-pyrido[2,3-d]pyrimidin-2,4-diamin,
6,N4-Dimethyl-7-(2-trifluormethyl-phenyl)-pyrido[2,3-d]pyrimidin-2,4-diamin,
N4-Methyl-7-thiophen-2-yl-pyrido[2,3-d]pyrimidin-2,4-diamin,
N4-Methyl-6,7-diphenyl-pyrido[2,3-d]pyrimidin-2,4-diamin,
7-(2,6-Dimethyl-phenyl)-N4-methyl-pyrido[2,3-d]pyrimidin-2,4-diamin,
7-(2,6-Dimethyl-phenyl)-N4-ethyl-pyrido[2,3-d]pyrimidin-2,4-diamin,
7-(2-Fluor-6-trifluormethyl-phenyl)-N4-methyl-pyrido[2,3-d]pyrimidin-2,4-diamin,
7-(2-Chlor-6-fluor-phenyl)-N4-methyl-pyrido[2,3-d]pyrimidin-2,4-diamin,
N4-Methyl-7-(2,4,6-trimethyl-phenyl)-pyrido[2,3-d]pyrimidin-2,4-diamin,
7-(2-Brom-phenyl)-N4-methyl-pyrido[2,3-d]pyrimidin-2,4-diamin,
7-(2-Benzyloxy-phenyl)-N4-methyl-pyrido[2,3-d]pyrimidin-2,4-diamin,
7-(2-Ethoxy-phenyl)-N4-methyl-pyrido [2,3-d]pyrimidin-2,4-diamin-trifluoressigsäuresalz,
N4-Methyl-7-(2-p-tolyloxy-phenyl)-pyrido[2,3-d]pyrimidin-2,4-diamin-trifluoressigsäuresalz,
N4-Methyl-6-propyl-7-(2-trifluormethyl-phenyl)-pyrido[2,3-d]pyrimidin-2,4-diamin,
N4-Methyl-7-(2,4-dimethyl-phenyl)-pyrido[2,3-d]pyrimidin-2,4-diamin,
7-(2,6-Dichlor-4-trifluormethyl-phenyl)-N4-methyl-pyrido[2,3-d]pyrimidin-2,4-diamin-trifluoressigsäuresalz,
N8-Methyl-5,6-dihydro-benzo[h]pyrimido[4,5-b]chinolin-8,10-diamin,
N4-Methyl-7-naphthalin-1-yl-pyrido[2,3-d]pyrimidin-2,4-diamin,
7-(2-Iod-phenyl)-N4-methyl-pyrido[2,3-d]pyrimidin-2,4-diamin-trifluoressigsäuresalz,
7-(2-Fluor-6-methoxy-phenyl)-N4-methyl-pyrido[2,3-d]pyrimidin-2,4-diamin-trifluoressigsäuresalz,
7-(2-Ethoxy-6-fluor-phenyl)-N4-methyl-pyrido[2,3-d]pyrimidin-2,4-diamin-trifluoressigsäuresalz,
7-(2-Fluor-6-isopropoxy-phenyl)-N4-methyl-pyrido[2,3-d]pyrimidin-2,4-diamin-trifluoressigsäuresalz,
7-(2-Fluor-6-propoxy-phenyl)-N4-methyl-pyrido[2,3-d]pyrimidin-2,4-diamin-trifluoressigsäuresalz,
N4-Methyl-7-(2,3,5,6-tetramethyl-phenyl)-pyrido[2,3-d]pyrimidin-2,4-diamin-trifluoressigsäuresalz,
N4-Methyl-7-phenyl-6-propyl-pyrido[2,3-d]pyrimidin-2,4-diamin-trifluoressigsäuresalz,
6-Ethyl-N4-methyl-7-phenyl-pyrido[2,3-d]pyrimidin-2,4-diamin-trifluoressigsäuresalz,
6-Methansulfonyl-N4-methyl-7-(2-trifluormethyl-phenyl)-pyrido[2,3-d]pyrimidin-2,4-diamin-trifluoressigsäuresalz,
N4-Methyl-7-(2,3,6-trimethyl-phenyl)-pyrido[2,3-d]pyrimidin-2,4-diamin,
7-(2,6-Dichlor-3-fluor-phenyl)-N4-methyl-pyrido[2,3-d]pyrimidin-2,4-diamin,
7-(2,4-Bis-trifluormethyl-phenyl)-N4-methyl-pyrido[2,3-d]pyrimidin-2,4-diamin,
7-(2,6-Bis-trifluormethyl-phenyl)-N4-methyl-pyrido[2,3-d]pyrimidin-2,4-diamin,
7-(2,5-Dimethyl-phenyl)-N4-methyl-pyrido[2,3-d]pyrimidin-2,4-diamin, N4-Methyl-7-(2,3,6-trichlor-phenyl)-pyrido[2,3-d]pyrimidin-2,4-diamin,
2-(2-Amino-4-methylamino-pyrido[2,3-d]pyrimidin-7-yl)-4-methyl-phenol, N9-Methyl-6,7-dihydro-5H-10,12,13-triaza-benzo[3,4]cyclohepta[1,2-b]naphthalin-9,11-diamin-trifluoressigsäuresalz,
6-Isopropyl-N4-methyl-7-phenyl-pyrido[2,3-d]pyrimidin-2,4-diamin-trifluoressigsäuresalz,
2-(2-Amino-4-methylamino-pyrido[2,3-d]pyrimidin-7-yl)-phenol-trifluoressigsäuresalz,
7-(2,5-Dichlor-phenyl)-N4-methyl-pyrido[2,3-d]pyrimidin-2,4-diamin-trifluoressigsäuresalz,
7-(2,4-Dichlor-phenyl)-N4-methyl-pyrido[2,3-d]pyrimidin-2,4-diamin-trifluoressigsäuresalz,
7-(2,3-Dichlor-phenyl)-N4-methyl-pyrido[2,3-d]pyrimidin-2,4-diamin-trifluoressigsäuresalz,
N4-Methyl-6-phenethyl-7-phenyl-pyrido[2,3-d]pyrimidin-2,4-diamin-trifluoressigsäuresalz,
7-(2-Cyclopentyloxy-6-fluor-phenyl)-N4-methyl-pyrido[2,3-d]pyrimidin-2,4-diamin-trifluoressigsäuresalz,
2-[2-(2-Amino-4-methylamino-pyrido[2,3-d]pyrimidin-7-yl)-3-fluor-phenoxy]-ethanol-trifluoressigsäure,
3-[2-(2-Amino-4-methylamino-pyrido[2,3-d]pyrimidin-7-yl)-3-fluor-phenoxy]-propan-1-ol-trifluoressigsäure,
7-(2-Chlor-6-ethoxy-phenyl)-N4-methyl-pyrido[2,3-d]pyrimidin-2,4-diamin-trifluoressigsäuresalz,
2-Amino-4-methylamino-7-(2-trifluormethyl-phenyl)-pyrido[2,3-d]pyrimidin-6-carbonsäuretrifluoressigsäuresalz,
N4-Methyl-7-(1-phenyl-cyclopropyl)-pyrido[2,3-d]pyrimidin-2,4-diamin,
N4-Methyl-7-(1-phenyl-cyclopentyl)-pyrido[2,3-d]pyrimidin-2,4-diamin,
N4-Methyl-7-(1-phenyl-cyclohexyl)-pyrido[2,3-d]pyrimidin-2,4-diamin,
Kalium-2-(2-amino-4-methylamino-pyrido[2,3-d]pyrimidin-7-yl)-benzoat,
7-(2,4-Diethyl-phenyl)-N4-methyl-pyrido[2,3-d]pyrimidin-2,4-diamin,
N4-Ethyl-7-(2-trifluormethyl-phenyl)-pyrido[2,3-d]pyrimidin-2,4-diamin-trifluoressigsäuresalz,
N4-Ethyl-6-methyl-7-(2-trifluormethyl-phenyl)-pyrido[2,3-d]pyrimidin-2,4-diamin,
N4-Ethyl-7-o-tolyl-pyrido[2,3-d]pyrimidin-2,4-diamin-trifluoressigsäuresalz,
7-(2,6-Dichlor-phenyl)-N4-ethyl-pyrido[2,3-d]pyrimidin-2,4-diamin-trifluoressigsäuresalz,
7-(2-Brom-phenyl)-N4-ethyl-pyrido[2,3-d]pyrimidin-2,4-diamin-trifluoressigsäuresalz,
N4-Ethyl-7-(2-fluor-6-trifluormethyl-phenyl)-pyrido[2,3-d]pyrimidin-2,4-diamin-trifluoressigsäuresalz,
7-(2-Chlor-6-fluor-phenyl)-N4-ethyl-pyrido[2,3-d]pyrimidin-2,4-diamin-trifluoressigsäuresalz,
N4-Ethyl-7-(2,3,6-trimethyl-phenyl)-pyrido[2,3-d]pyrimidin-2,4-diamin-trifluoressigsäuresalz,
2-[2-Amino-7-(2,6-dichlor-phenyl)-pyrido[2,3-d]pyrimidin-4-ylamino]-ethanol,
N4-Methyl-7-(2-piperidin-1-yl-6-trifluormethyl-phenyl)-pyrido[2,3-d]pyrimidin-2,4-diamin,
N4-Methyl-7-(2-morpholin-4-yl-6-trifluormethyl-phenyl)-pyrido[2,3-d]pyrimidin-2,4-diamin-trifluoressigsäuresalz,
7-(2,4-Dimethyl-phenyl)-N4-methyl-pyrido[2,3-d]pyrimidin-2,4-diamin,
N4-Methyl-7-[2-(4-methyl-piperazin-1-yl)-6-trifluormethyl-phenyl]-pyrido[2,3-d]pyrimidin-2,4-diamin-trifluoressigsäuresalz,
7-(2-Ethoxy-6-trifluormethyl-phenyl)-N4-methyl-pyrido[2,3-d]pyrimidin-2,4-diamin-trifluoressigsäuresalz,
7-(2-Methoxy-6-trifluormethyl-phenyl)-N4-methyl-pyrido[2,3-d]pyrimidin-2,4-diamin-trifluoressigsäuresalz,
7-(2-Dimethylamino-6-trifluormethyl-phenyl)-N4-methyl-pyrido[2,3-d]pyrimidin-2,4-diamin,
N4-Methyl-7-(2-methylamino-6-trifluormethyl-phenyl)-pyrido[2,3-d]pyrimidin-2,4-diamin-trifluoressigsäuresalz,
7-[2-(2-Dimethylamino-ethoxy)-6-trifluormethyl-phenyl]-N4-methyl-pyrido[2,3-d]pyrimidin-2,4-diamin-trifluoressigsäuresalz,
N4-Methyl-7-(2-phenoxy-6-trifluormethyl-phenyl)-pyrido[2,3-d]pyrimidin-2,4-diamin-trifluoressigsäuresalz,
N4-Methyl-7-(2-methylsulfanyl-6-trifluormethyl-phenyl)-pyrido[2,3-d]pyrimidin-2,4-diamin-trifluoressigsäuresalz,
2-[2-(2-Amino-4-methylamino-pyrido[2,3-d]pyrimidin-7-yl)-3-trifluormethyl-phenoxy]-ethanol-trifluoressigsäuresalz,
7-[2-(2-Methoxy-ethoxy)-6-trifluormethyl-phenyl]-N4-methyl-pyrido[2,3-d]pyrimidin-2,4-diamin-trifluoressigsäuresalz,
N4-Methyl-7-[2-(2-pyrrolidin-1-yl-ethoxy)-6-trifluormethyl-phenyl]-pyrido[2,3-d]pyrimidin-2,4-diamin-trifluoressigsäuresalz,
7-(2-Isopropoxy-6-trifluormethyl-phenyl)-N4-methyl-pyrido[2,3-d]pyrimidin-2,4-diamin-trifluoressigsäuresalz,
N4-Methyl-7-(2-propoxy-6-trifluormethyl-phenyl)-pyrido[2,3-d]pyrimidin-2,4-diamin-trifluoressigsäuresalz,
7-[2-(2-Diethylamino-ethoxy)-6-trifluormethyl-phenyl]-N4-methyl-pyrido[2,3-d]pyrimidin-2,4-diamin-trifluoressigsäuresalz,
N4-Methyl-7-[2-(2-morpholin-4-yl-ethoxy)-6-trifluormethyl-phenyl]-pyrido[2,3-d]pyrimidin-2,4-diamin-trifluoressigsäuresalz,
7-(2-Fluor-6-pyrrolidin-1-yl-phenyl)-N4-methyl-pyrido[2,3-d]pyrimidin-2,4-diamin,
7-(2-Fluor-6-piperidin-1-yl-phenyl)-N4-methyl-pyrido[2,3-d]pyrimidin-2,4-diamin,
2-(2-Amino-4-methylamino-pyrido[2,3-d]pyrimidin-7-yl)-3-fluor-phenol,
7-(2-Fluor-6-morpholino-4-yl-phenyl)-N4-methyl-pyrido[2,3-d]pyrimidin-2,4-diamin,
7-(2-Fluor-6-phenylsulfanyl-phenyl)-N4-methyl-pyrido[2,3-d]pyrimidin-2,4-diamin-trifluoressigsäuresalz,
7-(2-Fluor-6-phenoxy-phenyl)-N4-methyl-pyrido[2,3-d]pyrimidin-2,4-diamin-trifluoressigsäure,
7-(2-Fluor-6-imidazol-1-yl-phenyl)-N4-methyl-pyrido[2,3-d]pyrimidin-2,4-diamin,
7-[2-(4-Benzyl-piperazin-1-yl)-6-fluor-phenyl]-N4-methyl-pyrido[2,3-d]pyrimidin-2,4-diamin-trifluoressigsäuresalz,
7-(2-Fluor-6-methylamino-phenyl)-N4-methyl-pyrido[2,3-d]pyrimidin-2,4-diamin-trifluoressigsäuresalz,
7-(2-Dimethylamino-6-fluor-phenyl)-N4-methyl-pyrido[2,3-d]pyrimidin-2,4-diamin-trifluoressigsäuresalz,
7-[2-Fluor-6-(4-methyl-piperazin-1-yl)-phenyl]-N4-methyl-pyrido[2,3-d]pyrimidin-2,4-diamin-trifluoressigsäuresalz,
1-[2-(2-Amino-4-methylamino-pyrido[2,3-d]pyrimidin-7-yl)-3-fluor-phenyl]-piperidin-2-carbonsäure-ethylester-trifluoressigsäuresalz,
7-(2-Fluor-6-thiomorpholin-4-yl-phenyl)-N4-methyl-pyrido[2,3-d]pyrimidin-2,4-diamin-trifluoressigsäuresalz,
7-[2-(3-Aminomethyl-piperidin-1-yl)-6-fluor-phenyl]-N4-methyl-pyrido[2,3-d]pyrimidin-2,4-diamin-trifluoressigsäuresalz, 7-[2-Fluor-6-(2-methoxymethyl-pyrrolidin-1-yl)-phenyl]-N4-methyl-pyrido[2,3-d]pyrimidin-2,4-diamin-trifluoressigsäuresalz,
7-[2-(4-Fluor-phenoxy)-6-trifluormethyl-phenyl]-N4-methyl-pyrido[2,3-d]pyrimidin-2,4-diamin-trifluoressigsäuresalz,
7-(2-Cyclohexyloxy-6-trifluormethyl-phenyl)-N4-methyl-pyrido[2,3-d]pyrimidin-2,4-diamin-trifluoressigsäuresalz,
N4-Methyl-7-[2-(2-methyl-pyrrolidin-1-yl)-6-trifluormethyl-phenyl]-pyrido[2,3-d]pyrimidin-2,4-diamin,
7-[2-(2,5-Dimethyl-pyrrolidin-1-yl)-6-trifluormethyl-phenyl]-N4-methyl-pyrido[2,3-d]pyrimidin-2,4-diamin-trifluoressigsäuresalz,
1-[2-(2-Amino-4-methylamino-pyrido[2,3-d]pyrimidin-7-yl)-3-trifluormethyl-phenyl]-pyrrolidin-3-ol-trifluoressigsäuresalz,
1-[2-(2-Amino-4-methylamino-pyrido[2,3-d]pyrimidin-7-yl)-3-trifluormethyl-phenyl]-piperidin-4-ol-trifluoressigsäuresalz,
2-[2-Amino-7-(2-phenoxy-6-trifluormethyl-phenyl)-pyrido[2,3-d]pyrimidin-4-ylamino]-ethanol-trifluoressigsäuresalz,
{1-[2-(2-Amino-4-methylamino-pyrido[2,3-d]pyrimidin-7-yl)-3-trifluormethyl-phenyl]-pyrrolidin-2-yl} -methanol-trifluoressigsäuresalz,
7-[2-(2-Methoxymethyl-pyrrolidin-1-yl)-6-trifluormethyl-phenyl]-N4-methyl-pyrido-[2,3-d]pyrimidin-2,4-diamin-trifluoressigsäuresalz,
1-[2-(2-Amino-4-methylamino-pyrido[2,3-d]pyrimidin-7-yl)-3-trifluormethyl-phenyl]-piperidin-3-ol-trifluoressigsäuresalz,
7-[2-(4-Cyclohexyl-piperazin-1-yl)-6-trifluormethyl-phenyl]-N4-methyl-pyrido[2,3-d]pyrimidin-2,4-diamin-trifluoressigsäuresalz,
7-[2-(4-Ethyl-piperazin-1-yl)-6-trifluormethyl-phenyl]-N4-methyl-pyrido[2,3-d]pyrimidin-2,4-diamin-trifluoressigsäuresalz,
{4-[2-(2-Amino-4-methylamino-pyrido[2,3-d]pyrimidin-7-yl)-3-trifluormethyl-phenyl]-piperazin-1-yl}-furan-2-yl-methanon-trifluoressigsäuresalz,
7-(2- {4-[Bis-(4-fluor-phenyl)-methyl]-piperazin-1-yl} -6-trifluormethyl-phenyl)-N4-methyl-pyrido[2,3-d]pyrimidin-2,4-diamin-trifluoressigsäuresalz,
N4-Methyl-7-[2-(4-phenyl-piperazin-1-yl)-6-trifluormethyl-phenyl]-pyrido[2,3-d]pyrimidin-2,4-diamin-trifluoressigsäuresalz,
7-[2-(4-Benzyl-piperazin-1-yl)-6-trifluormethyl-phenyl]-N4-methyl-pyrido[2,3-d]pyrimidin-2,4-diamin-trifluoressigsäuresalz,
1-[2-(2-Amino-4-methylamino-pyrido[2,3-d]pyrimidin-7-yl)-3-trifluormethyl-phenyl]-4-phenyl-piperidin-4-ol-trifluoressigsäuresalz,
7-[2-(4-Benzyl-piperidin-1-yl)-6-trifluormethyl-phenyl]-N4-methyl-pyrido[2,3-d]pyrimidin-2,4-diamin-trifluoressigsäuresalz,
7-[2-(4-Cyclopentyl-piperazin-1-yl)-6-trifluormethyl-phenyl]-N4-methyl-pyrido[2,3-d]pyrimidin-2,4-diamin-trifluoressigsäuresalz,
2-{4-[2-(2-Amino-4-methylamino-pyrido[2,3-d]pyrimidin-7-yl)-3-trifluormethyl-phenyl]-piperazin-1-yl}-N-isopropyl-acetamid-trifluoressigsäuresalz,
7-[2-(4-Isopropyl-piperazin-1-yl)-6-trifluormethyl-phenyl]-N4-methyl-pyrido[2,3-d]pyrimidin-2,4-diamin-trifluoressigsäuresalz,
7-[2-(2-Fluor-phenoxy)-6-trifluormethyl-phenyl]-N4-methyl-pyrido[2,3-d]pyrimidin-2,4-diamin-trifluoressigsäuresalz,
7-[2-(3-Fluor-phenoxy)-6-trifluormethyl-phenyl]-N4-methyl-pyrido[2,3-d]pyrimidin-2,4-diamin-trifluoressigsäuresalz,
7-[2-(3-Chlor-phenoxy)-6-trifluormethyl-phenyl]-N4-methyl-pyrido[2,3-d]pyrimidin,2,4-diamin-trifluoressigsäuresalz,
7-[2-(4-Chlor-phenoxy)-6-trifluormethyl-phenyl]-N4-methyl-pyrido[2,3-d]pyrimidin-2,4-diamin-trifluoressigsäuresalz,
N4-Methyl-7-(2-p-tolyloxy-6-trifluormethyl-phenyl)-pyrido[2,3-d]pyrimidin-2,4-diamin-trifluoressigsäuresalz,
7-[2-(Biphenyl-4-yloxy)-6-trifluormethyl-phenyl]-N4-methyl-pyrido[2,3-d]pyrimidin-2,4-diamin-trifluoressigsäuresalz,
2-[2-Amino-7-(2-pyrrolidin-1-yl-6-trifluormethyl-phenyl)-pyrido[2,3-d]pyrimidin-4-ylamino]-ethanol-trifluoressigsäuresalz,
4-[2-Amino-7-(2-trifluormethyl-phenyl)-pyrido[2,3-d]pyrimidin-4-ylamino]-propan-1-ol-trifluoressigsäuresalz,
2-[2-Amino-7-(2-trifluormethyl-phenyl)-pyrido[2,3-d]pyrimidin-4-ylamino]-ethanol,
N4-Propyl-7-(2-trifluormethyl-phenyl)-pyrido[2,3-d]pyrimidin-2,4-diamin-trifluoressigsäuresalz,
4-[2-Amino-7-(2-trifluormethyl-phenyl)-pyrido[2,3-d]pyrimidin-4-ylamino]-butan-1-ol-trifluoressigsäuresalz,
2-[2-Amino-7-(2-fluor-6-trifluormethyl-phenyl)-pyrido[2,3-d]pyrimidin-4-ylamino]-ethanol,
2-[2-Amino-7-(2-brom-phenyl)-pyrido[2,3-d]pyrimidin-4-ylamino]-ethanol,
5,N-4-Dimethyl-7-phenyl-pyrido[2,3-d]pyrimidin-2,4-diamin-trifluoracetat und
N-4-Methyl-5,7-diphenyl-pyrido[2,3-d]pyrimidin-2,4-diamin-trifluoracetat, und
pharmazeutisch akzeptable Salze und Ester davon.

44. Verbindungen nach einem der Ansprüche 1 bis 43, ausgewählt aus der Gruppe, bestehend aus
N4-Methyl-7-(2-trifluormethyl-phenyl)-pyrido[2,3-d]pyrimidin-2,4-diamin,
7-(2,6-Dichlor-phenyl)-N4-methyl-pyrido[2,3-d]pyrimidin-2,4-diamin,
2-Chlor-6-fluorphenyl-N4-methyl-pyrido[2,3-d]pyrimidin-2,4-diamin,
7-(2-Fluor-6-trifluormethyl-phenyl)-N4-methyl-pyrido[2,3-d]pyrimidin-2,4-diamin,
7-(2-Chlor-6-fluor-phenyl)-N4-methyl-pyrido[2,3-d]pyrimidin-2,4-diamin,
7-(2,6-Dichlor-3-fluor-phenyl)-N4-methyl-pyrido[2,3-d]pyrimidin-2,4-diamin,
7-(2,6-Bis-trifluormethyl-phenyl)-N4-methyl-pyrido[2,3-d]pyrimidin-2,4-diamin,
7-(2-Chlor-6-ethoxy-phenyl)-N4-methyl-pyrido[2,3-d]pyrimidin-2,4-diamin-trifluoressigsäuresalz,
2-Amino-4-methylamino-7-(2-trifluormethyl-phenyl)-pyrido[2,3-d]pyrimidin-6-carbonsäuretrifluoressigsäuresalz,
7-(2,4-Dimethyl-phenyl)-N4-methyl-pyrido[2,3-d]pyrimidin-2,4-diamin,
7-(2-Ethoxy-6-trifluormethyl-phenyl)-N4-methyl-pyrido[2,3-d]pyrimidin-2,4-diamin-trifluoressigsäuresalz,
N4-Methyl-7-(2-phenoxy-6-trifluormethyl-phenyl)-pyrido[2,3-d]pyrimidin-2,4-diamin-trifluoressigsäuresalz,
7-(2-Isopropoxy-6-trifluormethyl-phenyl)-N4-methyl-pyrido[2,3-d]pyrimidin-2,4-diamin-trifluoressigsäuresalz,
7-[2-(4-Fluor-phenoxy)-6-trifluormethyl-phenyl]-N4-methyl-pyrido[2,3-d]pyrimidin-2,4-diamin-trifluoressigsäuresalz,
7-(2-Cyclohexyloxy-6-trifluormethyl-phenyl)-N4-methyl-pyrido[2,3-d]pyrimidin-2,4-diamin-trifluoressigsäuresalz,
7-[2-(2-Fluor-phenoxy)-6-trifluormethyl-phenyl]-N4-methyl-pyrido[2,3-d]pyrimidin-2,4-diamin-trifluoressigsäuresalz, 7-[2-(3-Fluor-phenoxy)-6-trifluormethyl-phenyl]-N4-methyl-pyrido[2,3-d]pyrimidin-2,4-diamin-trifluoressigsäuresalz, 7-[2-(4-Chlor-phenoxy)-6-trifluormethyl-phenyl]-N4-methyl-pyrido[2,3-d]pyrimidin-2,4-diamin-trifluoressigsäuresalz und
N4-Methyl-7-(2-p-tolyloxy-6-trifluormethyl-phenyl)-pyrido[2,3-d]pyrimidin-2,4-diamin-trifluoressigsäuresalz, und
pharmazeutisch akzeptable Salze und Ester davon.

45. Verfahren zur Herstellung von Verbindungen nach einem der Ansprüche 1 bis 44, umfassend das Umsetzen einer Verbindung der Formel (II) 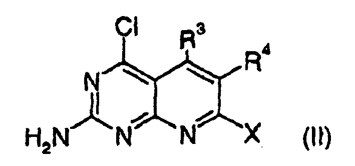
mit einer Verbindung HN(R¹,R²), worin R¹, R², R³, R⁴ und X wie einem der Ansprüche 1 bis 44 definiert sind.

46. Pharmazeutische Zusammensetzungen, umfassend eine Verbindung nach einem der Ansprüche 1 bis 44 und einen pharmazeutisch akzeptablen Träger und/oder ein pharmazeutisch akzeptables Adjuvans.

47. Verbindungen nach einem der Ansprüche 1 bis 44 zur Verwendung als therapeutisch aktive Substanzen.

48. Verbindungen nach einem der Ansprüche 1 bis 44, verwendbar zur therapeutischen und/oder prophylaktischen Behandlung von Diabetes.

49. Verwendung von Verbindungen nach einem der Ansprüche 1 bis 44 zur Herstellung von Medikamenten zur therapeutischen und/oder prophylaktischen Behandlung von Diabetes.

## Revendications

1. Composés de formule (I) : 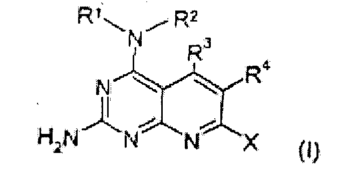
dans laquelle X représente un groupe X-1 de formule 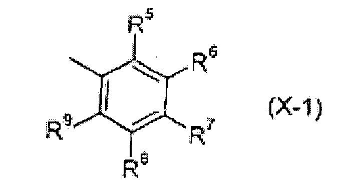
ou X représente un groupe X-2 de formule 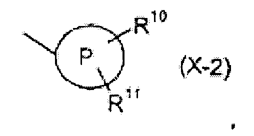
ou bien X représente un groupe X-3 de formule 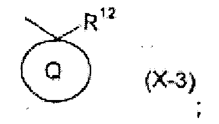
R¹ et R² sont choisis indépendamment dans le groupe consistant en un atome d'hydrogène, des groupes alkyle inférieur, méthoxy-alkyle inférieur et hydroxyalkyle inférieur, sauf que R¹ et R² ne peuvent pas représenter l'un et l'autre un atome d'hydrogène ;
R³ représente un atome d'hydrogène, un groupe alkyle inférieur ou phényle ;
R⁴ représente un atome d'hydrogène, un groupe alkyle inférieur, alkylsulfonyle inférieur, phényle ou carboxy ou bien, conjointement avec R⁵, forme un noyau carbocyclique penta- à heptagonal ;
R⁵ lorsqu'il n'est pas condensé dans un noyau avec R⁴, représente un atome d'hydrogène, un groupe alkyle inférieur, alkyle inférieur substitué, alkoxy inférieur, alkoxy inférieur substitué, hydroxy, carboxy, halogéno, alkylthio inférieur, alkylsulfinyle inférieur, alkylsulfonyle inférieur, aminosulfonyle, cyano, nitro, alcanoyle inférieur, aryle, aroyle, aryloxy, arylthio, perfluoroalkyle inférieur, alkylamino inférieur, alcanoylamino inférieur, sulfonylamino, cycloalkyle, cycloalkoxy, hétérocyclyle, hétérocyclyloxy, hétérocyclylcarbonyle ou hétéroaryle ou bien, conjointement avec R⁶ forme un second noyau aromatique penta- ou hexagonal condensé ;
R⁶ lorsqu'il n'est pas condensé dans un noyau avec R5⁴, représente un atome d'hydrogène, un groupe alkyle inférieur, alkyle inférieur substitué, alkoxy inférieur, alkoxy inférieur substitué, hydroxy, halogéno, alkylthio inférieur, alkylsulfinyle inférieur, alkylsulfonyle inférieur, aminosulfonyle, cyano, nitro, alcanoyle inférieur, aryle, aroyle, aryloxy, alkylamino inférieur, alcanoylamino inférieur, sulfonylamino, cycloalkyle, hétérocyclyle, hétérocyclyloxy ou hétérocyclylcarbonyle ;
R⁷ représente un atome d'hydrogène, un groupe alkyle inférieur, alkoxy inférieur, alkoxy(alkyle inférieur), alkoxy(alkoxy inférieur), hydroxyalkyle inférieur, hydroxy, hydroxyalkoxy, halogéno, alkylthio inférieur, alkylsulfinyle inférieur, alkylsulfonyle inférieur, perfluoroalkyle inférieur, alcanoyle inférieur, aroyle ou alcanoylamino inférieur ;
R⁸ et R⁹ sont choisis chacun indépendamment dans le groupe consistant en un atome d'hydrogène, des groupes alkyle inférieur, alkyle inférieur substitué, alkoxy inférieur, alkoxy inférieur substitué, hydroxy, halogéno, alkylthio inférieur, alkylsulfinyle inférieur, alkylsulfonyle inférieur, aminosulfonyle, cyano, nitro, alcanoyle inférieur, aryle, aroyle, aryloxy, alkylamino inférieur, alcanoylamino inférieur, sulfonylamino, cycloalkyle, hétérocyclyle, hétérocyclyloxy et hétérocyclylcarbonyle ;
P représente un noyau hétéroaromatique penta- ou hexagonal contenant 1 ou 2 hétéroatomes choisis dans le groupe consistant en l'oxygène, le soufre et l'azote ;
R¹⁰ et R¹¹ sont choisis chacun indépendamment dans le groupe consistant en un atome d'hydrogène, des groupes alkyle inférieur, alkoxy inférieur, perfluoroalkyle inférieur, halogéno, aryl(alkyle inférieur), aryle et aryl(alkoxy inférieur) ;
Q représente un noyau cycloalkyle tri- à hexagonal ; et
R¹² représente un atome d'hydrogène ou un groupe aryle ;
ou leurs sels ou esters pharmaceutiquement acceptables,
dans lesquels l'expression "alkyle inférieur", seule ou association, désigne un groupe alkyle à chaîne droite ou à chaîne ramifiée contenant un nombre maximal de six atomes de carbone ;
dans lesquels l'expression "alkoxy inférieur" désigne un groupe alkyle inférieur (répondant à la définition précitée) lié par un atome d'oxygène ;
dans lesquels l'expression "alkylthio inférieur" désigne un groupe alkyle inférieur lié par un atome de soufre divalent ; et
dans lesquels l'expression "alcanoyle inférieur" désigne un groupe alkyle inférieur lié au reste de la molécule par un groupe carbonyle.

2. Composés suivant la revendication 1, de formule (Ia) : 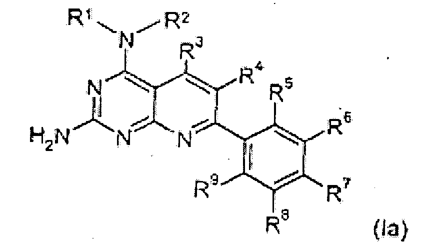
dans lesquels R³ représente un atome d'hydrogène, R⁴ représente un atome d'hydrogène, un groupe alkyle inférieur, alkylsulfonyle inférieur, phényle ou carboxy, et R¹, R², R⁵, R⁶ R⁷, R⁸ et R⁹ sont tels que définis dans la revendication 1.

3. Composés suivant la revendication 2, dans lesquels R⁶ R⁷ et R⁸ représentent chacun indépendamment un atome d'hydrogène, un groupe halogéno, alkyle inférieur, alkoxy inférieur, hydroxy, hydroxyalkyle inférieur, alkylthio inférieur, alkylsulfinyle inférieur, alkylsulfonyle inférieur ou perfluoroalkyle inférieur.

4. Composés suivant la revendication 3, dans lesquels R⁷ représente un atome d'hydrogène ou de fluor.

5. Composés suivant la revendication 4, dans lesquels un de R⁶ et R⁸ représente un atome d'hydrogène ou de fluor.

6. Composés suivant la revendication 4, dans lesquels un de R⁶ et R⁸ représente un atome d'hydrogène ou de fluor et l'autre représente un groupe halogéno, alkyle inférieur, alkoxy inférieur, hydroxy, hydroxyalkyle inférieur, alkylthio inférieur, alkylsulfinyle inférieur, alkylsulfonyle inférieur ou perfluoroalkyle inférieur.

7. Composés suivant la revendication 5, dans lesquels R⁶ R⁷ et R⁸ représentent des atomes d'hydrogène.

8. Composés suivant l'une quelconque des revendications 2 à 7, dans lesquels R⁵ et R⁹ sont choisis chacun indépendamment dans le groupe consistant en un atome d'hydrogène, des groupes halogéno, alkyle inférieur, alkoxy inférieur, alkoxy(alkoxy inférieur), nitro, hydroxy, hydroxyalkoxy inférieur, hydroxyalkyle inférieur, alkylthio inférieur, alkylamino inférieur, alkylsulfonyle inférieur, alkylsulfinyle inférieur, perfluoroalkyle inférieur, cycloalkyle, cycloalkoxy, aryle, hétéroaryle, aryloxy, arylthio et hétérocyclyle.

9. Composés suivant l'une quelconque des revendications 5 à 7, dans lesquels R⁵ et R⁹ sont choisis chacun indépendamment dans le groupe consistant en des atomes de chlore ou de fluor, des groupes trifluorométhyle, alkyle en C₁ à C₄, alkylthio en C₁ à C₃, alkylsulfonyle en C₁ à C₃, alkoxy en C₁ à C₃, phénoxy, phénoxy monosubstitué avec un substituant fluoro, chloro ou oxygène, et alkoxy en C₁ à C₃ substitué avec un substituant hydroxy, méthoxy ou éthoxy.

10. Composés suivant la revendication 2, dans lesquels R¹ ou R² représente un atome d'hydrogène.

11. Composé suivant la revendication 10, dans lequel le groupe R¹ ou R² qui est substitué est substitué avec un substituant alkyle en C₁ à C₄ ou hydroxyalkyle en C₁ à C₃.

12. Composés suivant la revendication 10, dans lesquels R⁶, R⁷ et R⁸ représentent chacun indépendamment un atome d'hydrogène, un groupe halogéno, alkyle inférieur, alkoxy inférieur, hydroxy, hydroxyalkyle inférieur, alkylthio inférieur, alkylsulfinyle inférieur, alkylsulfonyle inférieur ou perfluoroalkyle inférieur.

13. Composés suivant la revendication 10, dans lesquels R⁷ représente un atome d'hydrogène ou de fluor.

14. Composés suivant la revendication 13, dans lesquels un de R⁶ et R⁸ représente un atome d'hydrogène ou de fluor.

15. Composés suivant la revendication 13, dans lesquels un de R⁶ et R⁸ représente un atome d'hydrogène ou de fluor et l'autre représente un groupe halogéno, alkyle inférieur, alkoxy inférieur, hydroxy, hydroxyalkyle inférieur, alkylthio inférieur, alkylsulfinyle inférieur, alkylsulfonyle inférieur ou perfluoroalkyle inférieur.

16. Composés suivant la revendication 14, dans lesquels R⁶ R⁷ et R⁸ représentent des atomes d'hydrogène.

17. Composés suivant la revendication 16, dans lesquels le groupe R¹ ou R² qui est substitué est substitué avec un substituant alkyle en C₁ à C₄ ou hydroxyalkyle en C₁ à C₃.

18. Composés suivant l'une quelconque des revendications 10 à 15, dans lesquels R⁵ et R⁹ sont choisis chacun indépendamment dans le groupe consistant en un atome d'hydrogène, des groupes halogéno, alkyle inférieur, alkoxy inférieur, alkoxy(alkoxy inférieur), nitro, hydroxy, hydroxyalkoxy inférieur, hydroxyalkyle inférieur, alkylthio inférieur, alkylamino inférieur, alkylsulfonyle inférieur, alkylsulfinyle inférieur, perfluoroalkyle inférieur, cycloalkyle, cycloalkoxy, aryle, hétéroaryle, aryloxy, arylthio et hétérocyclyle.

19. Composés suivant la revendication 18, dans lesquels le groupe R¹ ou R² qui est substitué est substitué avec un substituant alkyle en C₁ à C₄ ou hydroxyalkyle en C₁ à C₃.

20. Composés suivant la revendication 16, dans lesquels R⁵ et R⁹ sont choisis chacun indépendamment dans le groupe consistant en des atomes de chlore ou de fluor, des groupes trifluorométhyle, alkyle en C₁ à C₄, alkylthio en C₁ à C₃, alkylsulfonyle en C₁ à C₃, alkoxy en C₁ à C₃, phénoxy, phénoxy monosubstitué avec un substituant fluoro, chloro ou oxygène, et alkoxy en C₁ à C₃ substitué avec un substituant hydroxy, méthoxy ou éthoxy.

21. Composés suivant la revendication 20, dans lesquels le groupe R¹ ou R² qui est substitué est substitué avec un substituant alkyle en C₁ à C₄ ou hydroxyalkyle en C₁ à C₃.

22. Composés suivant la revendication 1, dans lesquels R⁴ et R⁵ forment un noyau carbocyclique penta- à heptagonal.

23. Composés suivant la revendication 22, dans lesquels R¹ ou R² représente un atome d'hydrogène.

24. Composés suivant la revendication 23, dans lesquels R⁷ représente un atome d'hydrogène ou de fluor.

25. Composés suivant la revendication 24, dans lesquels un de R⁶ et R⁸ représente un atome d'hydrogène.

26. Composés suivant la revendication 24, dans lesquels un de R⁶ et R⁸ représente un atome d'hydrogène ou de fluor et l'autre représente un groupe halogéno, alkyle inférieur, alkoxy inférieur, hydroxy, hydroxyalkyle inférieur, alkylthio inférieur, alkylsulfinyle inférieur, alkylsulfonyle inférieur ou perfluoroalkyle inférieur.

27. Composés suivant la revendication 25, dans lesquels R⁶ R⁷ et R⁸ représentent des atomes d'hydrogène.

28. Composés suivant la revendication 27, dans lesquels le groupe R¹ ou R² qui est substitué est substitué avec un substituant alkyle en C₁ à C₄ ou hydroxyalkyle en C₁ à C₃.

29. Composés suivant la revendication 23, dans lesquels R⁵ et R⁹ représentent chacun indépendamment un atome d'hydrogène, un groupe halogéno, alkyle inférieur, alkoxy inférieur, alkoxy(alkoxy inférieur), nitro, hydroxy, hydroxyalkoxy inférieur, hydroxyalkyle inférieur, alkylthio inférieur, alkylsulfinyle inférieur, alkylsulfonyle inférieur ou perfluoroalkyle inférieur.

30. Composés suivant la revendication 23 ou 25, dans lesquels le groupe R¹ ou R² qui est substitué est substitué avec un substituant alkyle en C₁ à C₄ ou hydroxyalkyle en C₁ à C₃.

31. Composés suivant la revendication 1, de formule (Ib) : 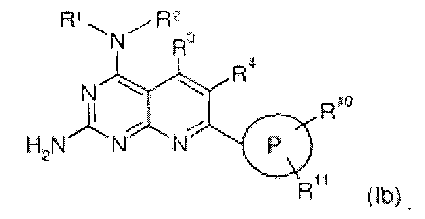
dans laquelle R¹, R², R³, R⁴, P, R¹⁰ et R¹¹ sont tels que définis dans la revendication 1.

32. Composés suivant la revendication 31, dans lesquels R¹ ou R² représente un atome d'hydrogène.

33. Composés suivant la revendication 32, dans lesquels R³ représente un atome d'hydrogène et R⁴ représente un atome d'hydrogène, un groupe alkyle inférieur, alkylsulfonyle inférieur, phényle ou carboxy.

34. Composés suivant la revendication 32 ou 33, dans lesquels R¹⁰ et R¹¹ représentent chacun indépendamment un groupe alkyle inférieur, alkoxy inférieur, perfluoroalkyle inférieur ou halogéno.

35. Composés suivant la revendication 34, dans lesquels le groupe R¹ ou R² qui est substitué est substitué avec un substituant alkyle en C₁ à C₄ ou hydroxyalkyle en C₁ à C₃.

36. Composés suivant la revendication 34, dans lesquels le groupe R¹ ou R² qui est substitué est substitué avec un substituant alkyle en C₁ à C₄ ou hydroxyalkyle en C₁ à C₃.

37. Composés suivant la revendication 1, de formule (Ic) : 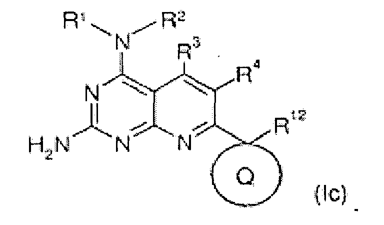
dans laquelle R¹, R², R³, R⁴ Q et R¹² sont tels que définis dans la revendication 1.

38. Composés suivant la revendication 37, dans lesquels R¹ ou R² représente un atome d'hydrogène.

39. Composés suivant la revendication 38, dans lesquels R³ représente un atome d'hydrogène et R⁴ représente un atome d'hydrogène, un groupe alkyle inférieur, alkylsulfonyle inférieur, phényle ou carboxy.

40. Composés suivant la revendication 38, dans lesquels R¹² représente un groupe phényle non substitué ou substitué.

41. Composés suivant la revendication 38, dans lesquels R¹² représente un groupe phényle monosubstitué.

42. Composés suivant la revendication 38 ou 40, dans lesquels le groupe R¹ ou R² qui est substitué est substitué avec un substituant alkyle en C₁ à C₄ ou hydroxyalkyle en C₁ à C₃.

43. Composés suivant l'une quelconque des revendications 1 à 42, choisis dans le groupe consistant en :
la N4-méthyl-7-o-tolyl-pyrido[2,3-d]pyrimidine-2,4-diamine,
la N4-méthyl-7-(2-trifluorométhylphényl)-pyrido[2,3-d]-pyrimidine-2,4-diamine,
la 7-(2,6-dichlorophényl)-N4-méthyl-pyrido[2,3-d]pyrimidine-2,4-diamine,
la 7-(2-chlorophényl)-N4-méthyl-pyrido[2,3-d]pyrimidine-2,4-diamine,
la 7-(2,6-difluorophényl)-N4-méthyl-pyrido[2,3-d]pyrimidine-2,4-diamine,
la 7-tertiobutyl-N4-méthyl-pyrido[2,3-d]pyrimidine-2,4-diamine,
la 2-chloro-6-fluorophényl-N4-méthyl-pyrido[2,3-d]-pyrimidine-2,4-diamine,
la 7-(2-fluoro-6-trifluorométhylphényl)-N4-méthyl-pyrido-[2,3-d]pyrimidine-2,4-diamine,
la 7-cyclohexyl-N4-méthyl-pyrido[2,3-d]pyrimidine-2,4-diamine,
la 7-(2-méthoxyphényl)-N4-méthyl-pyrido[2,3-d]pyrimidine-2,4-diamine,
la N4-méthyl-7-(2-nitrophényl)-pyrido[2,3-d]pyrimidine-2,4-diamine,
la 6,N4-diméthyl-7-(2-trifluorométhylphényl)-pyrido-[2,3-d]pyrimidine-2,4-diamine,
la N4-méthyl-7-thiophène-2-yl-pyrido[2,3-d]pyrimidine-2,4-diamine,
la N4-méthyl-6,7-diphényl-pyrido[2,3-d]pyrimidine-2,4-diamine,
la 7-(2,6-diméthylphényl)-N4-méthyl-pyrido[2,3-d]pyrimidine-2,4-diamine,
la 7-(2,6-diméthylphényl)-N4-éthyl-pyrido[2,3-d]pyrimidine-2,4-diamine,
la 7-(2-fluoro-6-trifluorométhylphényl)-N4-méthyl-pyrido-[2,3-d]pyrimidine-2,4-diamine,
la 7-(2-chloro-6-fluorophényl)-N4-méthyl-pyrido[2,3-d]-pyrimidine-2,4-diamine,
la N4-méthyl-7-(2,4,6-triméthylphényl)-pyrido[2,3-d]-pyrimidine-2,4-diamine,
la 7-(2-bromophényl)-N4-méthyl-pyrido[2,3-d]pyrimidine-2,4-diamine,
la 7-(2-benzyloxyphényl)-N4-méthyl-pyrido[2,3-d]pyrimidine-2,4-diamine, le sel d'acide trifluoro-acétique de 7-(2-éthoxy-phényl)-N4-méthyl-pyrido[2,3-d]pyrimidine-2,4-diamine,
le sel d'acide trifluoro-acétique de N4-méthyl-7-(2p-tolyloxyphényl)-pyrido[2,3-d]pyrimidine-2,4-diamine,
le sel d'acide trifluoro-acétique de N4-méthyl-6-propyl-7-(2-trifluorométhylphényl)-pyrido[2,3-d]pyrimidine-2,4-diamine,
la N4-méthyl-7-(2,4-diméthylphényl)-pyrido[2,3-d]pyrimidine-2,4-diamine,
le sel d'acide trifluoro-acétique de 7-(2,6-dichloro-4-trifluorométhylphényl)-N4-méthyl-pyrido[2,3-d]pyrimidine-2,4-diamine,
la N8-méthyl-5,6-dihydro-benzo[H]pyrimido[4,5-b]quinoléine-8,10-diamine,
la N4-méthyl-7-naphtalène-1-yl--pyrido[2,3-d]pyrimidine-2,4-diamine,
le sel d'acide trifluoro-acétique de 7-(2-iodophényl)-N4-méthyl-pyrido[2,3-d]pyrimidine-2,4-diamine,
le sel d'acide trifluoro-acétique de 7-(2-fluoro-6-méthoxyphényl)-N4-méthyl-pyrido[2,3-d]pyrimidine-2,4-diamine,
le sel d'acide trifluoro-acétique de 7-(2-éthoxy-6-fluorophényl)-N4-méthyl-pyrido[2,3-d]pyrimidine-2,4-diamine,
le sel d'acide trifluoro-acétique de 7-(2-fluoro-6-isopropoxyphényl)-N4-méthyl-pyrido[2,3-d]pyrimidine-2,4-diamine,
le sel d'acide trifluoro-acétique de 7-(2-fluoro-6-propoxyphényl)-N4-méthyl-pyrido[2,3-d]pyrimidine-2,4-diamine,
le sel d'acide trifluoro-acétique de N4-méthyl-7-(2,3,5,6-tétraméthylphényl)-pyrido[2,3-d]pyrimidine-2,4-diamine,
le sel d'acide trifluoro-acétique de N4-méthyl-7-phényl-6-propyl-pyrido[2,3-d]pyrimidine-2,4-diamine,
le sel d'acide trifluoro-acétique de 6-éthyl-N4-méthyl-7-phényl-pyrido[2,3-d]pyrimidine-2,4-diamine,
le sel d'acide trifluoro-acétique de 6-méthanesulfonyl-N4-méthyl-7-(2-trifluorométhylphényl)-pyrido[2,3-d]pyrimidine-2,4-diamine,
la N4-méthyl-7-(2,3,6-triméthylphényl)-pyrido[2,3-d]-pyrimidine-2,4-diamine,
la 7-(2,6-dichloro-3-fluorophényl)-N4-méthyl-pyrido-[2,3-d]pyrimidine-2,4-diamine,
la 7-(2,4-bis-trifluorométhylphényl)-N4-méthyl-pyrido-[2,3-d]pyrimidine-2,4-diamine,
la 7-(2,6-bis-trifluorométhylphényl)-N4-méthyl-pyrido-[2,3-d]pyrimidine-2,4-diamine,
la 7-(2,5-diméthylphényl)-N4-méthyl-pyrido[2,3-d]pyrimidine-2,4-diamine,
la N4-méthyl-7-(2,3,6-trichlorophényl)-pyrido[2,3-d]-pyrimidine-2,4-diamine,
le 2-(2-amino-4-méthylamino-pyrido[2,3-d]pyrimidine-7-yl)-4-méthylphénol,
le sel d'acide trifluoro-acétique de N9-méthyl-6,7-dihydro-5H-10,12,13-triaza-benzo[3,4]cyclohepta[1,2-b]naphtalène-9,11-diamine,
le sel d'acide trifluoro-acétique de 6-isopropyl-N4-méthyl-7-phényl-pyrido[2,3-d]pyrimidine-2,4-diamine,
le sel d'acide trifluoro-acétique de 2-(2-amino-4-méthylamino-pyrido[2,3-d]pyrimidine-7-yl)-phénol,
le sel d'acide trifluoro-acétique de 7-(2,5-dichlorophényl)-N4-méthyl-pyrido[2,3-d]pyrimidine-2,4-diamine,
le sel d'acide trifluoro-acétique de 7-(2,4-dichlorophényl)-N4-méthyl-pyrido[2,3-d]pyrimidine-2,4-diamine,
le sel d'acide trifluoro-acétique de 7-(2,3-dichlorophényl)-N4-méthyl-pyrido[2,3-d]pyrimidine-2,4-diamine,
le sel d'acide trifluoro-acétique de N4-méthyl-6-phénéthyl-7-phényl-pyrido[2,3-d]pyrimidine-2,4-diamine,
le sel d'acide trifluoro-acétique de 7-(2-cyclopentyloxy-6-fluorophényl)-N4-méthyl-pyrido[2,3-d]pyrimidine-2,4-diamine,
l'acide trifluoro-acétique de 2-[2-(2-amino-4-méthylamino-pyrido[2,3-d]pyrimidine-7-yl)-3-fluorophénoxy]-éthanol,
l'acide trifluoro-acétique de 3-[2-(2-amino-4-méthylamino-pyrido[2,3-d]pyrimidine-7-yl)-3-fluorophénoxy]-propane-1-ol,
le sel d'acide trifluoro-acétique de 7-(2-chloro-6-éthoxyphényl)-N4-méthyl-pyrido[2,3-d]pyrimidine-2,4-diamine,
le sel d'acide trifluoro-acétique d'acide 2-amino-4-méthylamino-7-(2-trifluorométhylphényl)-pyrido[2,3-d]pyrimidine-6-carbocyclique,
la N4-méthyl-7-(1-phényl-cyclopropyl)-pyrido[2,3-d]-pyrimidine-2,4-diamine,
la N4-méthyl-7-(1-phényl-cyclopentyl)-pyrido[2,3-d]-pyrimidine-2,4-diamine,
la N4-méthyl-7-(1-phényl-cyclohexyl)-pyrido[2,3-d]-pyrimidine-2,4-diamine,
le 2-(2-amino-4-méthylamino-pyrido[2,3-d]pyrimidine-7-yl)-benzoate de potassium,
la 7-(2,4-diéthylphényl)-N4-méthyl-pyrido[2,3-d]pyrimidine-2,4-diamine,
le sel d'acide trifluoro-acétique de N4-éthyl-7-(2-trifluorométhylphényl)-pyrido[2,3-d]pyrimidine-2,4-diamine,
la N4-éthyl-6-méthyl-7-(2-trifluorométhylphényl)-pyrido-[2,3-d]pyrimidine-2,4-diamine,
le sel d'acide trifluoro-acétique de N4-éthyl-7-o-tolyl-pyrido[2,3-d]pyrimidine-2,4-diamine,
le sel d'acide trifluoro-acétique de 7-(2,6-dichloro-phényl)-N4-éthyl-pyrido[2,3-d]pyrimidine-2,4-diamine,
le sel d'acide trifluoro-acétique de 7-(2-bromophényl)-N4-éthyl-pyrido[2,3-d]pyrimidine-2,4-diamine,
le sel d'acide trifluoro-acétique de N4-éthyl-7-(2-fluoro-6-trifluorométhylphényl)-pyrido[2,3-d]pyrimidine-2,4-diamine,
le sel d_{'}acide trifluoro-acétique de 7-(2-chloro-6-fluorophényl)-N4-éthyl-pyrido[2,3-d]pyrimidine-2,4-diamine,
le sel d'acide trifluoro-acétique de N4-éthyl-7-(2,3,6-triméthylphényl)-pyrido[2,3-d]pyrimidine-2,4-diamine,
le 2-[2-amino-7-(2,6-dichlorophényl)-pyrido[2,3-d]pyrimidine-4-ylamino]-éthanol,
la N4-méthyl-7-(2-pipéridine-1-yl-6-trifluorométhylphényl)-pyrido[2,3-d]pyrimidine-2,4-diamine,
le sel d'acide trifluoro-acétique de N4-méthyl-7-(2-morpholine-4-yl-6-trifluorométhylphényl)-pyrido[2,3-d]pyrimidine-2,4-diamine,
la 7-(2,4-diméthylphényl)-N4-méthyl-pyrido[2,3-d]pyrimidine-2,4-diamine,
le sel d'acide trifluoro-acétique de N4-méthyl-7-[2-(4-méthyl-pipérazine-1-yl)-6-trifluorométhylphényl]-pyrido-[2,3-d]pyrimidine-2,4-diamine,
le sel d'acide trifluoro-acétique de 7-(2-éthoxy-6-trifluorométhylphényl)-N4-méthyl-pyrido[2,3-d]pyrimidine-2,4-diamine,
le sel d'acide trifluoro-acétique de 7-(2-méthoxy-6-trifluorométhylphényl)-N4-méthyl-pyrido[2,3-d]pyrimidine-2,4-diamine,
la 7-(2-diméthylamino-6-trifluorométhylphényl)-N4-méthyl-pyrido[2,3-d]pyrimidine-2,4-diamine,
le sel d'acide trifluoro-acétique de N4-méthyl-7-(2-méthylamino-6-trifluorométhylphényl)-pyrido[2,3-d]pyrimidine-2,4-diamine,
le sel d'acide trifluoro-acétique de 7-[2-(2-diméthylamino-éthoxy)-6-trifluorométhylphényl]-N4-méthyl-pyrido[2,3-d]-pyrimidine-2,4-diamine,
le sel d'acide trifluoro-acétique de N4-méthyl-7-(2-phénoxy-6-trifluorométhylphényl)-pyrido[2,3-d]pyrimidine-2,4-diamine,
le sel d'acide trifluoro-acétique de N4-méthyl-7-(2-méthylsulfanyl-6-trifluorométhylphényl)-pyrido[2,3-d]pyrimidine-2,4-diamine,
le sel d'acide trifluoro-acétique de 2-[2-(2-amino-4-méthylamino-pyrido[2,3-d]pyrimidine-7-yl)-3-trifluorométhylphénoxy]-éthanol,
le sel d'acide trifluoro-acétique de 7-[2-(2-méthoxy-éthoxy)-6-trifluorométhylphényl]-N4-méthyl-pyrido[2,3-d]-pyrimidine-2,4-diamine,
le sel d'acide trifluoro-acétique de N4-méthyl-7-[2-(2-pyrrolidine-1-yl-éthoxy)-6-trifluorométhylphényl]-pyrido-[2,3-d]pyrimidine-2,4-diamine,
le sel d'acide trifluoro-acétique de 7-(2-isopropoxy-6-trifluorométhylphényl)-N4-méthyl-pyrido[2,3-d]pyrimidine-2,4-diamine,
le sel d'acide trifluoro-acétique de N4-méthyl-7-(2-propoxy-6-trifluorométhylphényl)-pyrido[2,3-d]pyrimidine-2,4-diamine,
le sel d'acide trifluoro-acétique de 7-[2-(2-diéthylamino-éthoxy)-6-triflorométhylphényl]-N4-méthyl-pyrido[2,3-d]-pyrimidine-2,4-diamine,
le sel d'acide trifluoro-acétique de N4-méthyl-7-[2-(2-morpholine-4-yl-éthoxy)-6-trifluorométhylphényl]-pyrido-[2,3-d]pyrimidine-2,4-diamine,
la 7-(2-fluoro-6-pyrrolidine-1-ylphényl)-N4-méthyl-pyrido-[2,3-d]pyrimidine-2,4-diamine,
la 7-(2-fluoro-6-pipéridine-1-ylphényl)-N4-méthyl-pyrido-[2,3-d]pyrimidine-2,4-diamine,
le 2-(2-amino-4-méthylamino-pyrido[2,3-d]pyrimidine-7-yl)-3-fluorophénol,
la 7-(2-fluoro-6-morpholino-4-ylphényl)-N4-méthyl-pyrido-[2,3-d]pyrimidine-2,4-diamine,
le sel d'acide trifluoro-acétique de 7-(2-fluoro-6-phénylsulfanylphényl)-N4-méthyl-pyrido[2,3-d]pyrimidine-2,4-diamine,
l'acide trifluoro-acétique 7-(2-fluoro-6-phénoxyphényl)-N4-méthyl-pyrido[2,3-d]pyrimidine-2,4-diamine,
la 7-(2-fluoro-6-imidazole-1-ylphényl)-N4-méthyl-pyrido-[2,3-d]pyrimidine-2,4-diamine,
le sel d'acide trifluoro-acétique de 7-[2-(4-benzyl-pipérazine-1-yl)-6-fluorophényl]-N4-méthyl-pyrido[2,3-d]-pyrimidine-2,4-diamine,
le sel d'acide trifluoro-acétique de 7-(2-fluoro-6-méthylaminophényl)-N4-méthyl-pyrido[2,3-d]pyrimidine-2,4-diamine,
le sel d'acide trifluoro-acétique de 7-(2-diméthylamino-6-fluorophényl)-N4-méthyl-pyrido[2,3-d]pyrimidine-2,4-diamine,
le sel d'acide trifluoro-acétique de 7-[2-fluoro-6-(4-méthyl-pipérazine-1-yl)-phényl]-N4-méthyl-pyrido[2,3-d]-pyrimidine-2,4-diamine,
le sel d'acide trifluoro-acétique d'ester éthylique d'acide 1-[2-(2-amino-4-méthylamino-pyrido[2,3-d]pyrimidine-7-yl)-3-fluorophényl]-pipéridine-2-carboxylique,
le sel d'acide trifluoro-acétique de 7-(2-fluoro-6-thiomorpholine-4-ylphényl)-N4-méthyl-pyrido[2,3-d]pyrimidine-2,4-diamine,
le sel d'acide trifluoro-acétique de 7-[2-(3-aminométhyl-pipéridine-1-yl)-6-fluorophényl]-N4-méthyl-pyrido[2,3-d]-pyrimidine-2,4-diamine,
le sel d'acide trifluoro-acétique de 7-[2-fluoro-6-(2-méthoxyméthyl-pyrrolidine-1-yl)-phényl-N4-méthyl-pyrido[2,3-d]-pyrimidine-2,4-diamine,
le sel d'acide trifluoro-acétique de 7-[2-(4-fluorophénoxy)-6-trifluorométhylphényl]-N4-méthyl-pyrido[2,3-d]-pyrimidine-2,4-diamine,
le sel d'acide trifluoro-acétique de 7-(2-cyclohexyloxy-6-trifluorométhylphényl)-N4-méthyl-pyrido[2,3-d]pyrimidine-2,4-diamine,
la N4-méthyl-7-[2-(2-méthylpyrrolidine-1-yl)-6-trifluorométhylphényl]-pyrido[2,3-d]pyrimidine-2,4-diamine,
le sel d'acide trifluoro-acétique de 7-[2-(2,5-diméthyl-pyrrolidine-1-yl)-6-trifluorométhylphényl]-N4-méthyl-pyrido[2,3-d]pyrimidine-2,4-diamine,
le sel d'acide trifluoro-acétique de 1-[2-(2-amino-4-méthylamino-pyrido[2,3-d]pyrimidine-7-yl)-3-trifluorométhylphényl]-pyrrolidine-3-ol,
le sel d'acide trifluoro-acétique de 1-[2-(2-amino-4-méthylamino-pyrido[2,3-d]pyrimidine-7-yl)-3-trifluorométhylphényl]-pipéridine-4-ol,
le sel d'acide trifluoro-acétique de 2-[2-amino-7-(2-phénoxy-6-trifluorométhylphényl)-pyrido[2,3-d]pyrimidine-4-ylamino-éthanol,
le sel d'acide trifluoro-acétique de {1-[2-(2-amino-4-méthylamino-pyrido[2,3-d]pyrimidine-7-yl)-3-trifluorométhylphényl]-pyrrolidine-2-yl}-méthanol,
le sel d'acide trifluoro-acétique de 7-[2-(2-méthoxy-méthyl-pyrrolidine-1-yl)-6-trifluorométhyl-phényl]-N4-méthyl-pyrido[2,3-d]pyrimidine-2,4-diamine,
le sel d'acide trifluoro-acétique de 1-[2-(2-amino-4-méthylamino-pyrido[2,3-d]pyrimidine-7-yl)-3-trifluorométhylphényl]-pipéridine-3-ol,
le sel d'acide trifluoro-acétique de 7-[2-(4-cyclohexyl-pipérazine-1-yl)-6-trifluorométhylphényl]-N4-méthyl-pyrido-[2,3-d]pyrimidine-2,4-diamine,
le sel d'acide trifluoro-acétique de 7-[2-(4-éthyl-pipérazine-1-yl)-6-trifluorométhylphényl]-N4-méthyl-pyrido-[2,3-d]pyrimidine-2,4-diamine,
le sel d'acide trifluoro-acétique de{4-[2-(2-amino-4-méthylamino-pyrido[2,3-d]pyrimidine-7-yl)-3-trifluorométhylphényl]-pipérazine-1-yl}-furane-2-yl-méthanone,
le sel d'acide trifluoro-acétique de 7-(2-{4-[bis-(4-fluorophényl)-méthyl]-pipérazine-1-yl}-6-trifluorométhylphényl)-N4-méthyl-pyrido[2,3-d]pyrimidine-2,4-diamine,
le sel d'acide trifluoro-acétique de N4-méthyl-7-[2-(4-phényl-pipérazine-1-yl)-6-trifluorométhylphényl]-pyrido-[2,3-d]pyrimidine-2,4-diamine,
le sel d'acide trifluoro-acétique de 7-[2-(4-benzyl-pipérazine-1-yl)-6-trifluorométhylphényl]-N4-méthyl-pyrido-[2,3-d]pyrimidine-2,4-diamine,
le sel d'acide trifluoro-acétique de 1-[2-(2-amino-4-méthylamino-pyrido[2,3-d]pyrimidine-7-yl)-3-trifluorométhylphényl]-4-phényl-pipéridine-4-ol,
le sel d'acide trifluoro-acétique de 7-[2-(4-benzyl-pipéridine-1-yl)-6-trifluorométhylphényl]-N4-méthyl-pyrido-[2,3-d]pyrimidine-2,4-diamine,
le sel d'acide trifluoro-acétique de 7-[2-(4-cyclobenzyl-pipérazine-1-yl)-6-trifluorométhylphényl]-N4-méthyl-pyrido-[2,3-d]pyrimidine-2,4-diamine,
le sel d'acide trifluoro-acétique de 2-{4-[2-(2-amino-4-méthylamino-pyrido[2,3-d]pyrimidine-7-yl)-3-trifluorométhylphényl]-pipérazine-1-yl}-N-isopropyle-acétamide,
le sel d'acide trifluoro-acétique de 7-[2-(4-isopropyl-pipérazine-1-yl)-6-trifluorométhylphényl]-N4-méthyl-pyrido-[2,3-d]pyrimidine-2,4-diamine,
le sel d'acide trifluoro-acétique de 7-[2-(2-fluorophénoxy)-6-trifluorométhylphényl]-N4-méthyl-pyrido[2,3-d]-pyrimidine-2,4-diamine,
le sel d'acide trifluoro-acétique de 7-[2-(3-fluorophénoxy)-6-trifluorométhylphényl]-N4-méthyl-pyrido[2,3-d]-pyrimidine-2,4-diamine,
le sel d'acide trifluoro-acétique de 7-[2-(3-chlorophénoxy)-6-trifluorométhylphényl]-N4-méthyl-pyrido[2,3-d]-pyrimidine-2,4-diamine,
le sel d'acide trifluoro-acétique de 7-[2-(4-chlorophénoxy)-6-trifluorométhylphényl]-N4-méthyl-pyrido[2,3-d]-pyrimidine-2,4-diamine,
le sel d'acide trifluoro-acétique de N4-méthyl-7-(2-p-tolyloxy-6-trifluorométhylphényl)-pyrido[2,3-d]pyrimidine-2,4-diamine,
le sel d'acide trifluoro-acétique de 7-[2-biphényl-4-yloxy)-6-trifluorométhylphényl]-N4-méthyl-pyrido[2,3-d]-pyrimidine-2,4-diamine,
le sel d'acide trifluoro-acétique de 2-[2-amino-7-(2-pyrrolidine-1-yl-6-trifluorométhylphényl)-pyrido[2,3-d]-pyrimidine-4-ylamino]-éthanol,
le sel d'acide trifluoro-acétique de 4-[2-amino-7-(2-trifluorométhylphényl)-pyrido[2,3-d]pyrimidine-4-ylamino]-propane-1-ol,
le 2-[2-amino-7-(2-trifluorométhylphényl)-pyrido[2,3-d]-pyrimidine-4-ylamino]-éthanol,
le sel d'acide trifluoro-acétique de N4-propyl-7-(2-trifluorométhylphényl)-pyrido[2,3-d]pyrimidine-2,4-diamine,
le sel d'acide trifluoro-acétique de 4-[2-amino-7-(2-trifluorométhylphényl)-pyrido[2,3-d]pyrimidine-4-ylamino]-butane-1-ol,
le 2-[2-amino-7-(2-fluoro-6-trifluorométhylphényl)-pyrido-[2,3-d]pyrimidine-4-ylamino]-éthanol,
le 2-[2-amino-7-(2-bromophényl)-pyrido[2,3-d]pyrimidine-4-ylamino]-éthanol,
le trifluoro-acétate de 5,N-4-diméthyl-7-phényl-pyrido-[2,3-d]pyrimidine-2,4-diamine, et
le trifluoro-acétate de N-4-méthyl-5,7-diphényl-pyrido-[2,3-d]pyrimidine-2,4-diamine
et leurs sels et esters pharmaceutiquement acceptables.

44. Composés suivant l'une quelconque des revendications 1 à 43, choisis dans le groupe consistant en :
la N4-méthyl-7-(2-trifluorométhylphényl)-pyrido[2,3-d]-pyrimidine-2,4-diamine,
la 7-(2,6-dichlorophényl)-N4-méthyl-pyrido[2,3-d]pyrimidine-2,4-diamine,
la 2-chloro-6-fluorophényl-N4-méthyl-pyrido[2,3-d]-pyrimidine-2,4-diamine,
la 7-(2-fluoro-6-trifluorométhylphényl)-N4-méthyl-pyrido-[2,3-d]pyrimidine-2,4-diamine,
la 7-(2-chloro-6-fluorophényl)-N4-méthyl-pyrido[2,3-d]-pyrimidine-2,4-diamine,
la 7-(2,6-dichloro-3-fluorophényl)-N4-méthyl-pyrido-[2,3-d]pyrimidine-2,4-diamine,
la 7-(2,6-bis-trifluorométhylphényl)-N4-méthyl-pyrido-[2,3-d]pyrimidine-2,4-diamine,
le sel d'acide trifluoro-acétique de 7-(2-chloro-6-éthoxyphényl)-N4-méthyl-pyrido[2,3-d]pyrimidine-2,4-diamine,
le sel d'acide trifluoro-acétique d'acide 2-amino-4-méthylamino-7-(2-trifluorométhylphényl)-pyrido[2,3-d]pyrimidine-6-carbocyclique,
la 7-(2,4-diméthylphényl)-N4-méthyl-pyrido[2,3-d]pyrimidine-2,4-diamine,
le sel d'acide trifluoro-acétique de 7-(2-éthoxy-6-trifluorométhylphényl)-N4-méthyl-pyrido[2,3-d]pyrimidine-2,4-diamine,
le sel d'acide trifluoro-acétique de N4-méthyl-7-(2-phénoxy-6-trifluorométhylphényl)-pyrido[2,3-d]pyrimidine-2,4-diamine,
le sel d'acide trifluoro-acétique de 7-(2-isopropoxy-6-trifluorométhylphényl)-N4-méthyl-pyrido[2,3-d]pyrimidine-2,4-diamine,
le sel d'acide trifluoro-acétique de 7-[2-(4-fluorophénoxy)-6-trifluorométhylphényl]-N4-méthyl-pyrido[2,3-d]-pyrimidine-2,4-diamine,
le sel d'acide trifluoro-acétique de 7-(2-cyclohexyloxy-6-trifluorométhylphényl)-N4-méthyl-pyrido[2,3-d]pyrimidine-2,4-diamine,
le sel d'acide trifluoro-acétique de 7-[2-(2-fluorophénoxy)-6-trifluorométhylphényl]-N4-méthyl-pyrido[2,3-d]-pyrimidine-2,4-diamine,
le sel d'acide trifluoro-acétique de 7-[2-(3-fluorophénoxy)-6-trifluorométhylphényl]-N4-méthyl-pyrido[2,3-d]-pyrimidine-2,4-diamine,
le sel d'acide trifluoro-acétique de 7-[2-(4-chlorophénoxy)-6-trifluorométhylphényl]-N4-méthyl-pyrido[2,3-d]-pyrimidine-2,4-diamine,
le sel d'acide trifluoro-acétique de N4-méthyl-7-(2-p-tolyloxy-6-trifluorométhylphényl)-pyrido[2,3-d]pyrimidine-2,4-diamine,
et leurs sels et esters pharmaceutiquement acceptables.

45. Procédé pour la préparation de composés suivant l'une quelconque des revendications 1 à 44, comprenant la réaction d'un composé de formule (II) 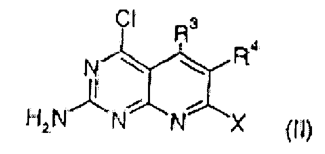
avec un composé de formule HN(R¹,R²), formules dans lesquelles R¹, R², R³, R⁴ et X sont tels que définis dans l'une quelconque des revendications 1 à 44.

46. Compositions pharmaceutiques comprenant un composé suivant l'une quelconque des revendications 1 à 44 et un support et/ou adjuvant pharmaceutiquement acceptables.

47. Composés suivant l'une quelconque des revendications 1 à 44, destinés à être utilisés comme substances thérapeutiques actives.

48. Composés suivant l'une quelconque des revendications 1 à 44, utiles pour le traitement thérapeutique et/ou prophylactique du diabète.

49. Utilisation de composés suivant l'une quelconque des revendications 1 à 44 pour la préparation de médicaments destinés au traitement thérapeutique et/ou prophylactique du diabète.
